(19) Europäisches Patentamt
European Patent Office
Office européen des brevets

(11) **EP 4 251 216 B1**

(12) **EUROPEAN PATENT SPECIFICATION**

(45) Date of publication and mention
of the grant of the patent:
**27.05.2026 Bulletin 2026/22**

(21) Application number: **21898823.6**

(22) Date of filing: **30.11.2021**

(51) International Patent Classification (IPC):
*A61L 24/04* $^{(2006.01)}$      *C09J 201/02* $^{(2006.01)}$
*C08G 83/00* $^{(2006.01)}$      *A61K 47/38* $^{(2006.01)}$
*A61K 47/56* $^{(2017.01)}$      *A61L 26/00* $^{(2006.01)}$

(52) Cooperative Patent Classification (CPC):
(C-Sets available)
**A61K 9/006; A61K 31/167; A61K 31/522;**
**A61K 31/573; A61K 31/717; A61K 35/74;**
**A61K 36/889; A61K 41/00; A61K 47/34;**
**A61K 47/38; A61L 26/0023; A61L 26/0052;**
**A61L 26/0066; A61L 26/008; A61L 26/0095;**
(Cont.)

(86) International application number:
**PCT/SG2021/050741**

(87) International publication number:
**WO 2022/115046 (02.06.2022 Gazette 2022/22)**

(54) **SELF-REGULATING BIOADHESIVES FOR WET SUBSTRATES**

SELBSTREGULIERENDE BIOKLEBSTOFFE FÜR FEUCHTE SUBSTRATE

BIOADHÉSIFS AUTO-RÉGULATEURS POUR SUBSTRATS HUMIDES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **30.11.2020 SG 10202011922U**

(43) Date of publication of application:
**04.10.2023 Bulletin 2023/40**

(73) Proprietor: **Nanyang Technological University**
**Singapore 639798 (SG)**

(72) Inventors:
• **LIM, Sierin**
**Singapore 639798 (SG)**
• **STEELE, Terry William Joseph**
**Singapore 639798 (SG)**
• **SINGH, Juhi**
**Singapore 639798 (SG)**

(74) Representative: **Viering, Jentschura & Partner**
**mbB**
**Patent- und Rechtsanwälte**
**Am Brauhaus 8**
**01099 Dresden (DE)**

(56) References cited:
**WO-A1-2014/081391      US-A1- 2020 171 200**

• **WICAKSONO GAUTAMA ET AL: "Photorheology**
**of bioadhesive dendrimer polycaprolactone**
**composites", POLYMER TESTING, ELSEVIER,**
**AMSTERDAM, NL, vol. 80, 3 September 2019**
**(2019-09-03), XP085935838, ISSN: 0142-9418,**
**[retrieved on 20190903], DOI: 10.1016/**
**J.POLYMERTESTING.2019.106099**

- SINGH JUHI; TAN NIGEL C.S.; MAHADEVASWAMY USHA RANI; CHANCHAREONSOOK NATTHAREE; STEELE TERRY W.J.; LIM SIERIN: "Bacterial cellulose adhesive composites for oral cavity applications", CARBOHYDRATE POLYMERS, APPLIED SCIENCE PUBLISHERS , LTD BARKING, GB, vol. 274, 7 July 2021 (2021-07-07), GB
, XP086841625, ISSN: 0144-8617, DOI: 10.1016/j.carbpol.2021.118403
- SINGH JUHI, STEELE TERRY W. J., LIM SIERIN: "Fibrillated bacterial cellulose liquid carbene bioadhesives for mimicking and bonding oral cavity surfaces", JOURNAL OF MATERIALS CHEMISTRY. B, ROYAL SOCIETY OF CHEMISTRY, GB, vol. 10, no. 14, 6 April 2022 (2022-04-06), GB
, pages 2570 - 2583, XP055940331, ISSN: 2050-750X, DOI: 10.1039/D1TB02044G
- GAO FENG: "On-Demand Bioadhesive Hydrogel based On Diazirine", PHD THESIS, NANYANG TECHNOLOGICAL UNIVERSITY, SINGAPORE, 28 February 2017 (2017-02-28), XP055940335, Retrieved from the Internet <URL:https://dr.ntu.edu.sg/handle/10356/69596>
- DJORDJEVIC IVAN; POKHOLENKO OLEKSANDR; SHAH ANKUR HARISH; WICAKSONO GAUTAMA; BLANCAFORT LLUIS; HANNA JOHN V.; PAGE SAMUEL J.; NAND: "CaproGlu: Multifunctional tissue adhesive platform", BIOMATERIALS, ELSEVIER, AMSTERDAM, NL, vol. 260, 11 July 2020 (2020-07-11), AMSTERDAM, NL
, XP086262132, ISSN: 0142-9612, DOI: 10.1016/j.biomaterials.2020.120215
- PINKAS ODED, HANEMAN OR, CHEMKE OMER, ZILBERMAN MEITAL: "Fiber-reinforced composite hydrogels for bioadhesive and sealant applications : Fiber-reinforced Composite Bioadhesives", POLYMERS FOR ADVANCED TECHNOLOGIES, WILEY & SONS , BOGNOR REGIS, GB, vol. 28, no. 9, 1 September 2017 (2017-09-01), GB
, pages 1162 - 1169, XP055940336, ISSN: 1042-7147, DOI: 10.1002/pat.4009
- AHMAD NAVEED, AMIN MOHD CAIRUL IQBAL MOHD, MAHALI SHALELA MOHD, ISMAIL ISMANIZAN, CHUANG VICTOR TUAN GIAM: "Biocompatible and Mucoadhesive Bacterial Cellulose -g- Poly(acrylic acid) Hydrogels for Oral Protein Delivery", MOLECULAR PHARMACEUTICS, AMERICAN CHEMICAL SOCIETY, US, vol. 11, no. 11, 3 November 2014 (2014-11-03), US
, pages 4130 - 4142, XP055940339, ISSN: 1543-8384, DOI: 10.1021/mp5003015

(52) Cooperative Patent Classification (CPC): (Cont.)
A61P 1/02; A61P 41/00; C08B 15/005; C08G 83/004; C08L 89/00; C09D 201/005; C09J 101/02; C09J 201/005; A61L 2430/22

C-Sets
A61K 31/167, A61K 2300/00;
A61K 31/522, A61K 2300/00;
A61K 31/573, A61K 2300/00;
A61K 31/717, A61K 2300/00;
A61K 35/74, A61K 2300/00;
A61K 36/889, A61K 2300/00;
A61L 26/0052, C08L 1/00;
A61L 26/0052, C08L 67/04;
A61L 26/0095, C08L 1/00;
A61L 26/0095, C08L 67/04;
C09J 101/02, C08L 89/00, C08L 101/005;
C09J 201/005, C08L 1/02

## Description

### Cross-Reference to Related Application

This application claims the benefit of priority of Singapore Patent Application No. 10202011922U, filed 30 November 2020.

### Technical Field

The present disclosure relates to a mucoadhesive. The present disclosure also relates to a method of forming the mucoadhesive.

### Background

The Global Burden of Disease Study 2017 estimated that about 45% of the world population is affected by non-communicable oral diseases, which include a series of chronic diseases affecting the oral cavity and may manifest in the form of wounds and ulcers. The oral cavity presents challenges like high bacterial and microorganisms load, presence of saliva and salivary enzymes, wet environment due to constant salivary flow, soft mucosal tissues, and dynamic shear forces that prevent implant fixation and subsequent treatment. Therefore, materials and adhesives have been generated specifically for soft tissue fixation within the buccal and gastrointestinal regions, which may primarily consist of mucous membranes on epithelial surfaces. The materials and adhesive may be referred to as mucoadhesives, and may consist of topical formulations of semi-solid, liquid, or film-based formulations.

Existing therapies based on plasters or mucoadhesives are limited in effectiveness due to adhesive failure (in less than 24 hrs), loss of structural integrity, or a combination thereof. Alternatives may include frequent applications (3-5/day) of liquid or gel, leading to poor patient compliance. Commercial mucoadhesive designs are based on water swelling polymers that stick to soft tissues via polymer entanglement at the tissue surface. The design is relatively safe, but the uncontrolled water absorption leads to worsening adhesion strength over time (0.5-5 kPa, assuming area of 2 $cm^2$). Hence, the mucoadhesive's structures have a limited fixation of 6-8 hours. Mucoadhesive designs may be improved if swelling plateaued, thus preventing cohesive and adhesive failure.

According to one example, current mechanisms of buccal mucoadhesion may rely on hydrophilic polymers that absorb water on the buccal tissues. Hydrophilic attractive forces result from mucoadhesive polymers entangling macromolecules on the tissue surface (e.g. glycoproteins, mucin, extracellular matrix proteins). The mucoadhesive forces may withstand the physiological shear forces for only a limited length of time. The primary reason for adhesion failure may be due to uncontrolled absorption of water, which decreases the number of entanglements, weakens the cohesive forces within the mucoadhesive, decreases the hydrophilic attractive forces, or a combination thereof. After a critical amount of water absorption, adhesion forces may be overcome by physiological shear forces and delamination, disintegration, fracture, or a combination thereof. Currently available adhesives may not adequately address uncontrolled water absorption, do not provide covalent crosslinking to tissue surfaces, or allow predetermined periods of fixation.

Current mucoadhesives based on water swelling, hydrophilic polymers are activated when applied to wetted tissue surfaces. They are reported to adhere non-specifically to wet surfaces with weak adhesion stresses ranging from 1-5 kPa (assuming an area of 2 $cm^2$). The main limitation is the limited adhesion time of 6-8 hours. Overhydration tends to be responsible for the short retention periods. Treatments based on mucoadhesives with extended fixation times of 2-7 days may target unmet clinical needs, however, mucoadhesives which typically rely on reversible attractive forces such as hydrogen bonds, electrostatic interactions, and van der Waals bonds, may not allow fixation of medical implants for 2-7 days. Biocompatible and Mucoadhesive Bacterial Cellulose-g-Poly(acrylic acid) Hydrogels for Oral Protein Delivery (Ahmad Naveed, Amin Mohd Cairul Iqbal Mohd, Mahali Shalela Mohd, Ismail Ismanizan, Chuang Victor Tuan Giam; Molecular Pharmaceutics, Americal Chemical Society Vol. 11, Nr. 11, pages 4130-4142) discloses the investigation of stimuli-responsive bacterial cellulose-g-poly(acrylic acid) hydrogels for their potential use as an oral delivery system for proteins.

There is thus a need to provide for a solution that addresses one or more of the limitations mentioned above. The solution should at least provide for a mucoadhesive that resolves one or more of the limitations mentioned above and a method of forming such a mucoadhesive.

### Summary

In a first aspect, there is provided a mucoadhesive including:

    a bacterial cellulose; and

a bioadhesive incorporated to the bacterial cellulose,

wherein the bioadhesive comprises a polymer or a dendrimer, wherein the polymer and the dendrimer are grafted with a moiety which forms covalent interactions with a tissue surface in the presence of a stimulus, and wherein the moiety comprises a diazirine.

In another aspect, there is provided a method of forming the mucoadhesive described in various embodiments of the first aspect, the method includes:

providing a bacterial cellulose;

providing a bioadhesive comprising a polymer or a dendrimer, wherein the polymer and the dendrimer are grafted with a moiety which forms covalent interactions with a tissue surface in the presence of a stimulus, and wherein the moiety comprises a diazirine; and

incorporating the bioadhesive to the bacterial cellulose.

## Brief Description of the Drawings

The drawings are not necessarily to scale, emphasis instead generally being placed upon illustrating the principles of the present disclosure. In the following description, various embodiments of the present disclosure are described with reference to the following drawings, in which:

FIG. 1 is a table comparing the present mucoadhesive (last row) with existing formulations with respect to the ingredients/components used and frequency of applications.

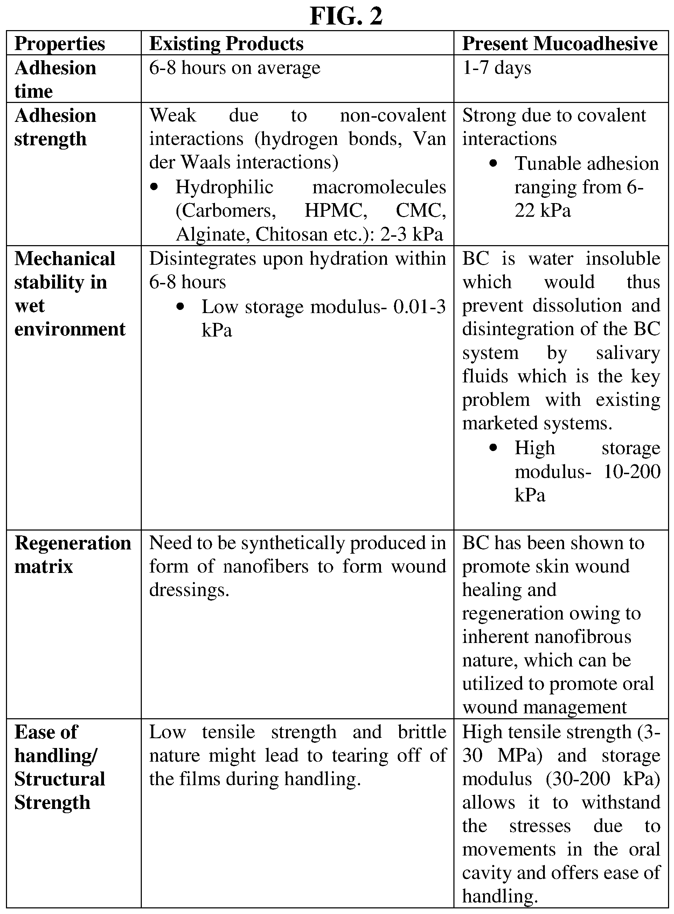

FIG. 2 is a table comparing the various properties between existing products and the present mucoadhesive.

FIG. 3 shows various bacterial cellulose with PAMAM-g-diazirine (BC_PDz) adhesive systems. BC denotes for bacterial cellulose and PDz denotes for PAMAM-g-diazirine.

FIG. 4A shows the sandwich setup and working apparatus for lap shear test consisting of BC film, PDz and porcine tissue.

FIG. 4B is a plot of the maximum adhesion strength at failure at varying UV dose and hydration state of BC_FD (bacterial cellulose freeze-dried) films. Data presented as mean $\pm$ SD, n = 5, p-values are calculated using two-way ANOVA with Bonferroni post hoc test, *$p < 0.05$, **$p < 0.01$, *** $p< 0.001$.

FIG. 4C is a plot of the maximum adhesion strength at failure at varying UV dose and hydration state of BC_PD (bacterial cellulose press-dried) films. Data presented as mean $\pm$ SD, n = 5, p-values are calculated using two-way ANOVA with Bonferroni post hoc test, *$p < 0.05$, **$p < 0.01$, *** $p< 0.001$.

FIG. 4D is a comparison of adhesion strength of BC_PDz hydrogel systems with that of BC film-based systems. BC_FD represents freeze-dried BC films and BC_PD represents press-dried BC films. Hydrogel represents BC_PDz aqueous adhesive hydrogels as presented in last row of FIG. 3. Data presented as mean $\pm$ SD, n = 5.

FIG. 5A shows the experimental setup under constant stirring. Scale bar denotes 1.5 cm.

FIG. 5B is a plot of ex vivo mucoadhesion time for tests performed in solutions of viscosities 1, 10 and 30 cps against porcine tissue (test stopped at 2 days) and at 1 cps against rabbit tongue for a 7-day period.

FIG. 5C shows photographs of ex vivo mucoadhesion time test with rabbit tongue and BC_PDz film for 7 days performed at 1cps viscosity. Scale bars denote 1.5 cm.

FIG. 5D shows photographs of cohesive fracture upon failure observed upon visual inspection for mucoadhesion time test performed with porcine tissue with 1 cps viscosity solution. Scale bars denote 1.5 cm.

FIG. 6 shows various results for mechanical characterizations of dry and rehydrated films. The top left plot is a comparison of tensile stress at break between different samples; inset shows low tensile stress. The top right plot shows tensile stress at break. The bottom left plot shows modulus of elasticity calculated up to 90% of the total strain up to break. The bottom right plot shows percentage elongation at break for films. Data presented as mean $\pm$ SD, n=3.

FIG. 7 shows a setup for determining light transmission properties of BC.

FIG. 8 is a schematic representation of BC production and drying processes. BC pellicles are typically grown for 5 days and form at the air-liquid interface. Washing with distilled water and treatment with 0.25 N NaOH at 60°C for 30 minutes remove the bacterial cells from BC further confirmed by protein determination. Two drying processes that are freeze-drying and press-drying result in similar water content removal. %Rehydration represents water absorbed by the dry films upon swelling as a percentage of initial weight of native BC (860 mg). Data presented as mean $\pm$ SD, n=3.

FIG. 9 demonstrates for surface and light transmission characterizations of BC_FD and BC_PD. The top left shows a SEM image of BC_FD, the top right shows a SEM image of BC_PD. Scale bars denote for 1 $\mu$m. The insets show the actual film and scale bars in both insets denote for 1.5 cm. The bottom left image is a plot of the light transmission properties of the BC_PD and BC_FD films as a function of varying hydration percentages and wavelengths, and the bottom right table tabulates the absorption coefficient for films with varying hydration percentage and wavelengths.

Data presented as mean ± SD, n=3. FD represents freeze-dried BC films, PD represents press-dried BC films.

FIG. 10A is a plot of adhesion strength versus strain curves at varying UV dose for BC_FD films.

FIG. 10B is a plot of adhesion strength versus strain curves at varying UV dose for BC_FD Rh films. BC_FD Rh represents rehydrated freeze-dried BC films.

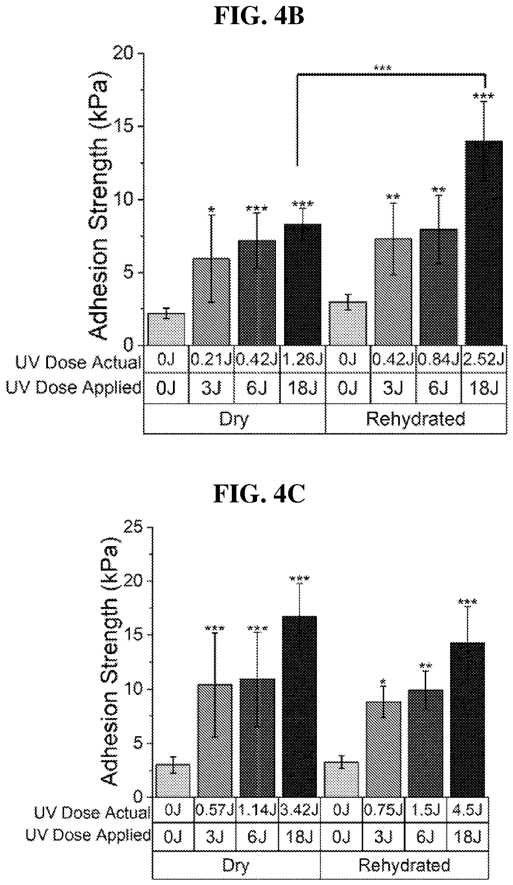

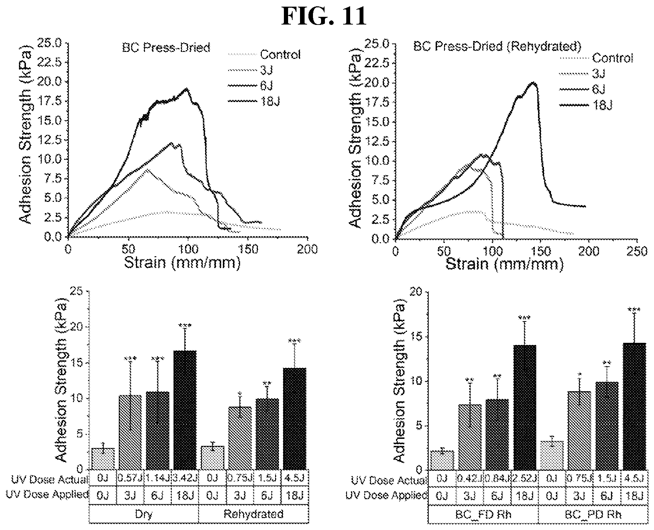

FIG. 11 demonstrates for adhesion strength versus strain curves at varying UV dose for (top left plot) BC_PD films and (top right plot) BC_PD Rh films. The bottom left plot is a plot of maximum adhesion strength at failure for varying UV dose and hydration state of BC_PD films. No significant difference in adhesion strength is observed between dry and rehydrated BC_PD films. The bottom right plot compares maximum adhesion strength of BC_FD and BC_PD in wet state. BC_PD represents press-dried BC films and BC_PD Rh represents rehydrated press-dried BC films. Data presented as mean ± SD, n = 5, p-values are calculated using two-way ANOVA with Bonferroni post hoc test, *p < 0.05, **p < 0.01, *** p< 0.001. The bottom left plot is reiterated from FIG. 4C.

FIG. 12A is a SEM image of BC_Pdz surface, wherein the BC was FD and the Pdz uncured. Scale bar denotes for 1 μm.

FIG. 12B is a SEM image of BC_Pdz surface, wherein the BC was FD and the PDz cured. The image shows FD state (dry state) of the BC. Scale bar denotes for 100 μm.

FIG. 12C is a SEM image showing cohesive fracture of a surface of a BC_PDz film, wherein the BC was FD and Rh and the PDz UV cured. Scale bar denotes for 100 μm.

FIG. 12D is a SEM image showing cohesive fracture of a surface of a BC_PDz film, wherein the BC was PD and the PDz uncured. Scale bar denotes for 1 μm.

FIG. 12E is a SEM image showing cohesive fracture of a surface of a BC_PDz film, wherein the BC was PD and the PDz UV cured. Scale bar denotes for 100 μm.

FIG. 12F is a SEM image showing cohesive fracture of a surface of a BC_PDz film, wherein the BC was PD and Rh and the PDz UV cured. Scale bar denotes for 100 μm.

FIG. 12G is a SEM image showing cohesive fracture of a surface of pure 50% PDz. Scale bar denotes for 100 μm.

FIG. 12H is a plot showing the pore size distribution of the cured BC_PDz films with varying film type and hydration state.

FIG. 12I is a plot of variation of the pore diameter and pore count as a function of varying BC film type.

FIG. 13 top image shows the FTIR spectra of pristine individual components (BC film and PDz) and representative BC_PDz film before and after UVA based photocuring at 18 J. PDz represents PAMAM-g-diazirine bioadhesive. BC_PDz represents bacterial cellulose film adhered with help of PDz. The table in bottom image shows the percentage change in the absorbance of functional groups before and after photocuring. Amide I is the carbonyl stretching vibrations. FTIR peak at 1630 cm$^{-1}$ is set as control for peak ratio comparison. % change = [cured ratio - uncured ratio]/uncured ratio * 100%.

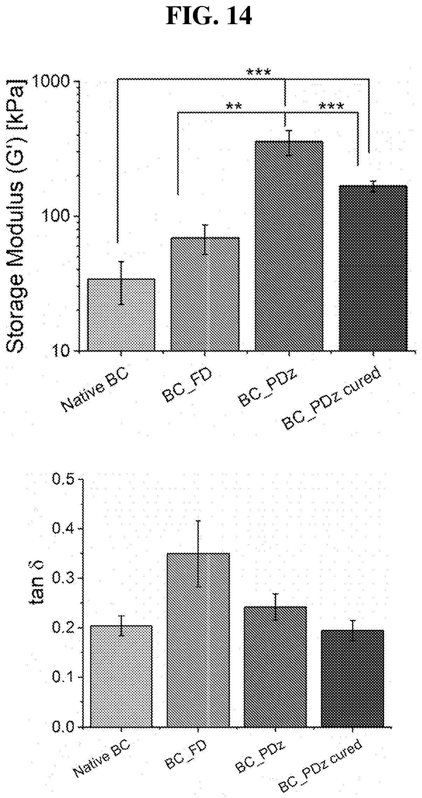

FIG. 14 demonstrates the rheological characterizations of rehydrated films. Top plot shows storage modulus G' of BC films with and without PDz and photocuring at 1.75 Hz frequency. Bottom plot shows loss tangent (tan δ) at 1.75 Hz. Native BC represents the wet BC pellicle obtained after washing, BC_FD represents the freeze-dried films, BC_PDz represents BC_FD with PDz, and BC_PDz cured represents BC_PDz photocured. Data presented as mean ± SD, n=3, p-values are calculated using two-way ANOVA with Bonferroni post hoc test, *p < 0.05, **p < 0.01, *** p< 0.001. Rheological measurements are conducted using Anton Paar Physica MCR 501 Rheometer. Parallel plate geometry with plate diameter of 10 mm is used with measuring gap of 3.0 mm and strain percentage set to 1%. Steady shear tests are conducted over a shear rate range of 0.065-65 1/s and conducted between 0.01 and 10 Hz in frequency sweeps. Three replicate scans are observed, wherein storage modulus (G'), loss modulus (G") and tan δ is recorded.

FIG. 15 is a table tabulating results of the FTIR spectra of BC_PDz before and after photocuring as shown in FIG. 13 bottom image, but additionally showing their absorbance.

FIG. 16A shows an ex vivo mucoadhesion study setup.

FIG. 16B is a table showing variation of absorbance of BC films with varying wavelengths (365-405 nm).

FIG. 17 is a schematic representation of BC_PDz hydrogel production. BC pellicles typically grown for 5 days are sonicated to obtain hydrogels. 1.3% w/v hydrogel obtained is then mixed PDz to obtain BC_PDz composite hydrogel, followed by covalent insertion with cellulose macromolecules.

FIG. 18 is a table showing composition of BC_PDz hydrogel formulations and % BC remaining after curing (varying total solute %).

FIG. 19 is a table showing composition of BC_PDz hydrogel formulations and % BC remaining after curing (varying BC moles).

FIG. 20 top left image is a schematic representation of photorheology setup with glass slide as sample holder and probe, equipped with UV source. The top right shows a plot of dynamic viscosity of pre-crosslinked formulations. The bottom left is a plot of comparative results of G' maximum measured at UV energy of 10J. The bottom right shows representative photorheometry diagrams of dynamic change of G' and G" during UV exposure in the range of 0-10 J. Data presented as mean ± SD, n=3.

FIG. 21A shows a photorheological evaluation of composite samples in 3 phases (I) measurement of dynamic viscosity before photocuring (II) measurement of Loss modulus (G") and Storage modulus (G') as a function of applied UV energy followed by (III) amplitude sweep of cured composites.

FIG. 21B is a plot of amplitude sweep of photocured BC_PDz composites with increasing total solute percentage in the composites.

FIG. 21C is a photorheogram of 1.3% BC hydrogel depicting changes in Loss modulus (G") and Storage modulus (G') as a function of applied UV energy up to 10J.

FIG. 21D is a plot of amplitude sweep of 1.3% BC hydrogel.

FIG. 22A is a plot of dynamic viscosity of pre-crosslinked formulations. Data presented as mean $\pm$ SD, n=3, p-values are calculated using two-way ANOVA with Bonferroni post hoc test, *$p$ < 0.05, **p < 0.01, *** p< 0.001.

FIG. 22B plots the comparative results of G' maximum measured at UV energy of 10J. Data presented as mean $\pm$ SD, n=3, p-values are calculated using two-way ANOVA with Bonferroni post hoc test, *$p$ < 0.05, **p < 0.01, *** p< 0.001.

FIG. 22C is a photorheometry diagrams of dynamic change of G' and G" during UV exposure in the range of 0-10 J at 20% total solute.

FIG. 22D is a photorheometry diagrams of dynamic change of G' and G" during UV exposure in the range of 0-10 J at 33.3% total solute.

FIG. 22E is a BC_PDz vector plots (gelation line) within the UV curing energy range of 0-10 J at 20% total solute.

FIG. 22F is a BC_PDz vector plots (gelation line) within the UV curing energy range of 0-10 J at 33.3% total solute.

FIG. 23 shows amplitude sweep of crosslinked BC_PDz composites after photocuring for composites of varying BC moles 20% total solute (see top left plot) and 33.3% total solute (top right plot). The yield stress and yield strain plotted with Pearson's r values for composites of varying total solute percentage (bottom left plot) and for composites of 20 and 33.3% total solute percentage (bottom right plot).

FIG. 24A is a SEM image of a cohesively fractured photocured BC_PDz composite surface of 20% total solute with BC mole at 0 mM. Scale bar denotes for 100 $\mu$m.

FIG. 24B is a SEM image of a cohesively fractured photocured BC_PDz composite surface of 20% total solute with BC mole at 20 mM. Scale bar denotes for 100 $\mu$m.

FIG. 24C is a SEM image of a cohesively fractured photocured BC_PDz composite surface of 20% total solute with BC mole at 40 mM. Scale bar denotes for 100 $\mu$m.

FIG. 24D is a SEM image of a cohesively fractured photocured BC_PDz composite surface of 20% total solute with BC mole at 80 mM. Scale bar denotes for 100 $\mu$m.

FIG. 24E is a SEM image of a cohesively fractured photocured BC_PDz composite surface of 33.3% total solute with BC mole at 0 mM. Scale bar denotes for 100 $\mu$m.

FIG. 24F is a SEM image of a cohesively fractured photocured BC_PDz composite surface of 33.3% total solute with BC mole at 20 mM. Scale bar denotes for 100 $\mu$m.

FIG. 24G is a SEM image of a cohesively fractured photocured BC_PDz composite surface of 33.3% total solute with BC mole at 40 mM. Scale bar denotes for 100 $\mu$m.

FIG. 24H is a SEM image of a cohesively fractured photocured BC_PDz composite surface of 33.3% total solute with BC mole at 80 mM. Scale bar denotes for 100 $\mu$m.

FIG. 24I is a plot of the pore size distribution for the cured BC_PDz hydrogels with varying BC moles at 20% total solute.

FIG. 24J is a plot of the average pore size and count for the cured BC_PDz films with varying BC moles at 20% total solute.

FIG. 24K is a plot of the pore size distribution for the cured BC_PDz hydrogels with varying BC moles at 33.3% total solute.

FIG. 24L is a plot of the average pore size and count for the cured BC_PDz films with varying BC moles at 33.3% total solute.

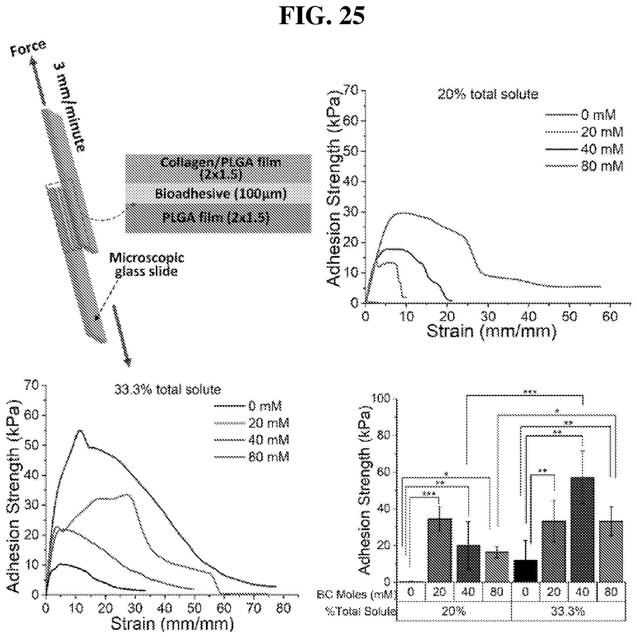

FIG. 25 demonstrates for the lap shear adhesion dependence on total solute percentage and BC moles. The top left image shows the sandwich setup for lap shear test consisting of PLGA film, BC_PDz bioadhesive and wet tissue mimic/PLGA film. A plot of adhesion strength versus strain curves performed against PLGA for varying BC moles for composites with 20% total solute and 33.3% total solute are shown in top right and bottom left plots, respectively. The bottom right plot shows maximum adhesion strength at failure for composites with varying total solute percentage and BC moles. Data presented as mean $\pm$ SD, n=5, p-values are calculated using two-way ANOVA with Bonferroni post hoc test, *$p$ < 0.05, **p < 0.01, *** p< 0.001.

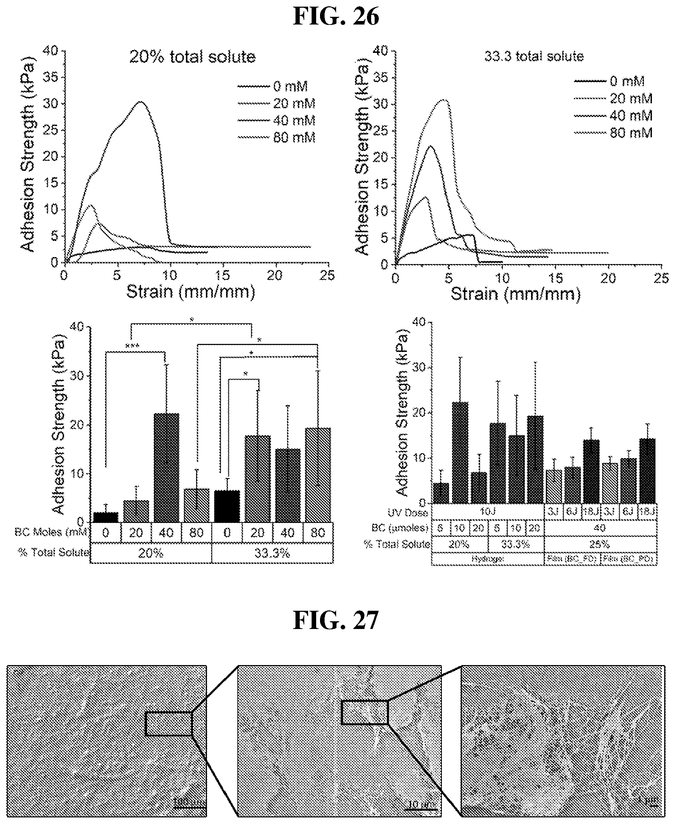

FIG. 26 shows plots of adhesion strength versus strain curves performed against wet tissue mimic for varying BC moles for composites with 20% total solute and 33.3% total solute (top left and top right, respectively). The bottom left plot shows maximum adhesion strength at failure for composites with varying total solute percentage and BC moles. The bottom right plot is a comparison of adhesion strength of BC_PDz hydrogel systems with that of BC film-based systems. Data presented as mean $\pm$ SD, n=5, *p*-values are calculated using two-way ANOVA with Bonferroni post hoc test, *p* < 0.05, **p < 0.01, *** p< 0.001.

FIG. 27 shows SEM images of 1.3% hydrogel. Scale bars denote for 100 μm, 10 μm, 1 μm, in left, center and right images, respectively.

FIG. 28 shows images of the photocured BC_PDz composites after completion of **III** phase of photorheology, i.e. amplitude sweep with varying total solute percentage (left to right) and varying BC moles (top to bottom). Scale bars denote for 5 mm.

FIG. 29 shows the FTIR spectra of pristine individual components (BC film and PDz) and representative BC_PDz hydrogel film before and after UVA based photocuring at 10 J. The peak at 1626 cm$^{-1}$ serves as reference for qualitative peak evaluation. FTIR measurements are (PerkinElmer Frontier) performed with Universal ATR fixture of the diamond crystal. Cured and uncured BC_PDz composites are placed directly onto the crystal. Each measurement is an accumulation of 32 scans with a resolution of 4 cm$^{-1}$ and a scan range of 4000 to 600 cm$^{-1}$.

FIG. 30 is a table tabulating the FTIR spectra results of 33.3% total solute and 80 mM BC_PDz hydrogel before and after photocuring.

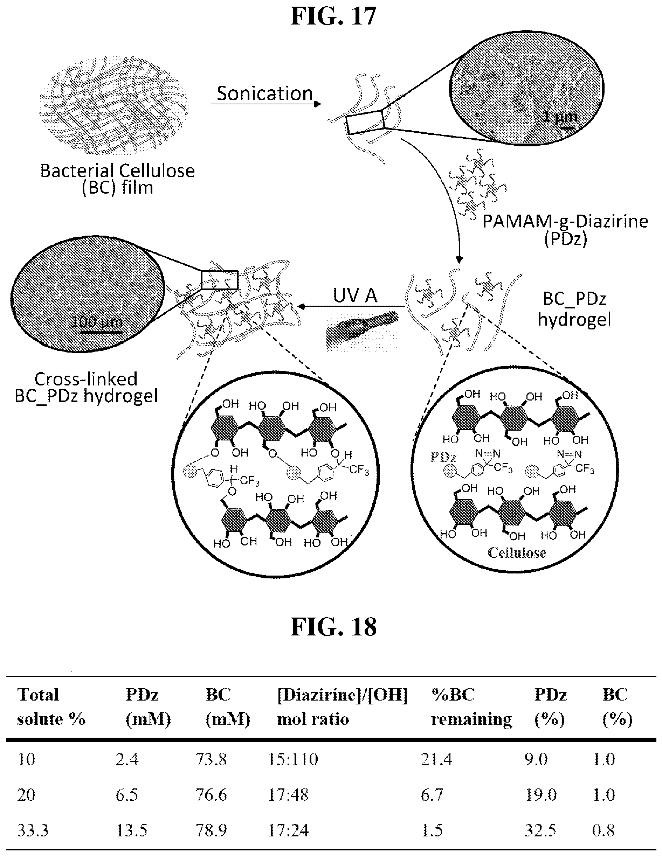

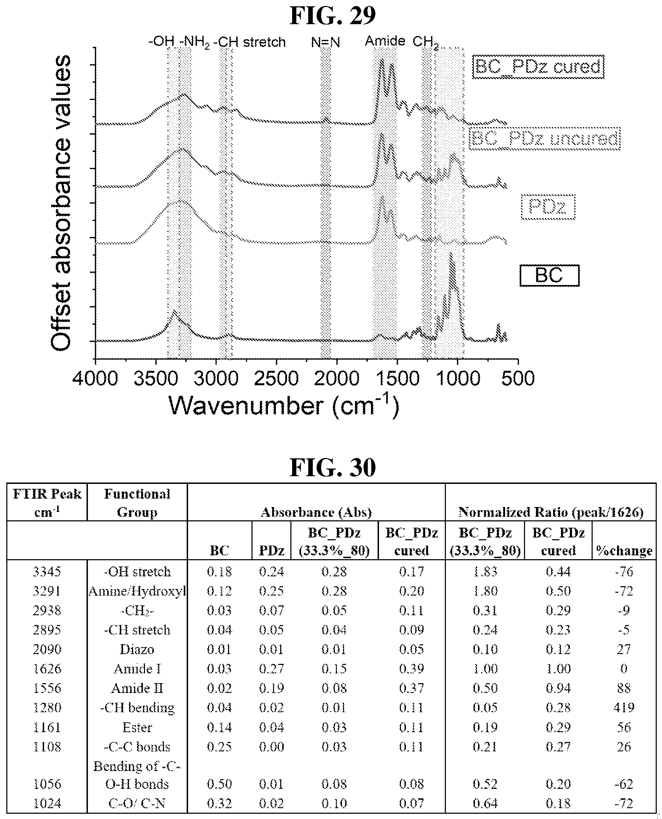

The primary adhesion mechanism provided by PDz is due the ability to form carbenes upon UV exposure. In case of BC_PDz composites, it is hypothesized that these carbenes would react majorly with the -OH groups and minorly with -CH- groups present in the bacterial cellulose structure as depicted in FIG. 17. The FTIR spectra for BC, PDz, uncured BC_PDz composite, and UV cured BC_PDz composites are displayed in FIG. 29 with associated peak assignments enumerated in FIG. 30. A new peak at 2090 cm$^{-1}$ in the spectra of photocured BC_PDz composite is observed, which corresponds to diazo formation, an intermediate which further lead to formation of carbene radical. Moreover, it is observed that there is a reduction in the absorbance of -CH$_2$- peak (2938 cm$^{-1}$), -CH stretch at (2895 cm$^{-1}$), -OH peak (3345 cm$^{-1}$), -NH$_2$ peak (3291 cm$^{-1}$) and -C-O-H bonds at (1056 cm$^{-1}$) which supports the carbene insertion mechanism. An increase in the absorbance of ester groups (-C-O-C-) peak (1161 cm$^{-1}$) and -C-C- bonds peak (1161 cm$^{-1}$) is observed. An increase in ester group absorbance might be due to the reaction of carbene with -OH groups forming ester and increase in -C-C- bonds due to reaction of carbene with -CH$_2$ groups as hypothesized in FIG. 17.

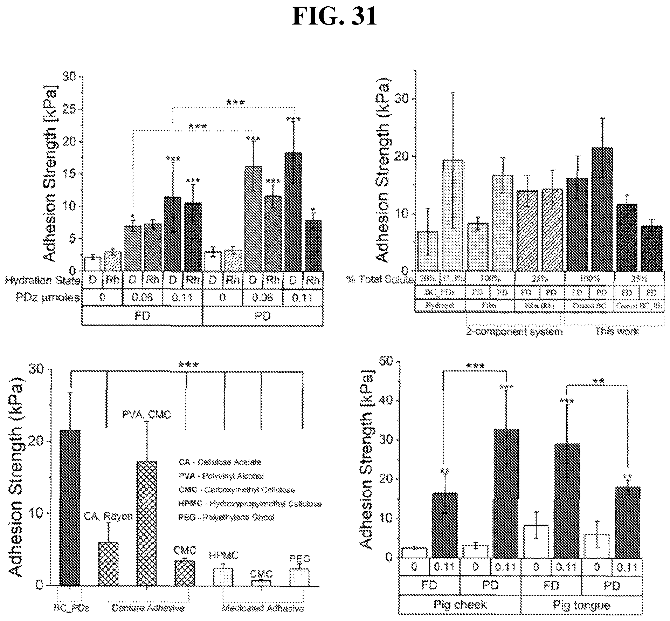

FIG. 31 demonstrates for Lap shear adhesion dependence on PDz moles and hydration. The top left plot shows maximum adhesion strength at failure at varying PDz moles and hydration state for BC_PDz composites (FD & PD films) against porcine tissue. The top right plot shows comparison of adhesion strength of different BC_PDz composites (Hydrogels, 2-component film system, integrated BC_PDz patch). The bottom left plot shows comparison of adhesion strength of BC_PDz composites with that of marketed denture and medicated adhesives. The bottom right plot shows maximum adhesion strength at failure for BC_PDz adhesive patches against mucosal tissues (porcine cheek, porcine tongue). BC_FD represents freeze-dried BC films and BC_PD represents press-dried BC films. Hydrogel represents BC_PDz aqueous adhesive hydrogels. D represents dry composites (100% solute), whereas Rh represents rehydrated composites (25% solute). Data presented as mean ± SD, n = 5, p-values are calculated using two-way ANOVA with Bonferroni post hoc test, *p < 0.05, **p < 0.01, *** p< 0.001.

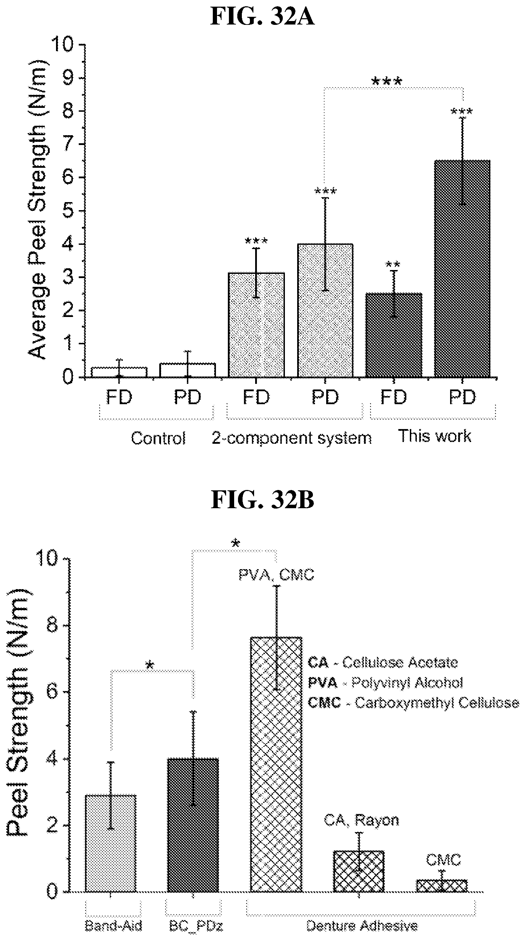

FIG. 32A is a plot of comparison of average peel strength of different BC_PDz composites (2-component film system, integrated BC_PDz patch) as determined by 90-degree peel test. Data presented as mean ± SD, n = 5, p-values are calculated using two-way ANOVA with Bonferroni post hoc test, *p < 0.05, **p < 0.01, *** p< 0.001.

FIG. 32B is a plot of comparison of adhesion strength of BC_PDz composites with that of marketed denture adhesives and wound plaster (Band-aid). Data presented as mean ± SD, n = 5, p-values are calculated using two-way ANOVA with Bonferroni post hoc test, *p < 0.05, **p < 0.01, *** p< 0.001.

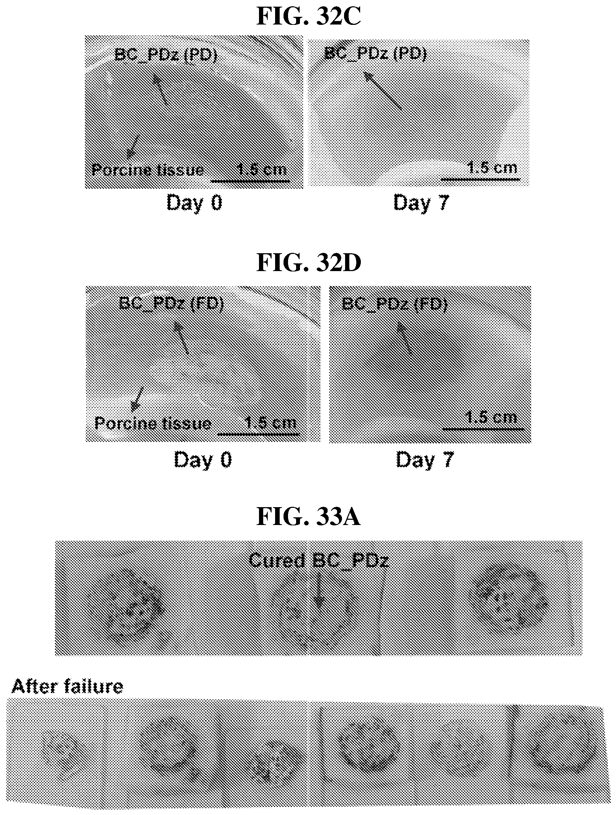

FIG. 32C demonstrates ex vivo mucoadhesion time test with porcine tissue and BC_PDz composites for 7 days performed at 1 cps viscosity for PD samples. BC_PD represents press-dried BC films.

FIG. 32D demonstrates ex vivo mucoadhesion time test with porcine tissue and BC_PDz composites for 7 days performed at 1 cps viscosity for FD samples. BC_FD represents freeze-dried BC films.

FIG. 33A shows BC_PDz activated through electrosurgery before and after lap shear.

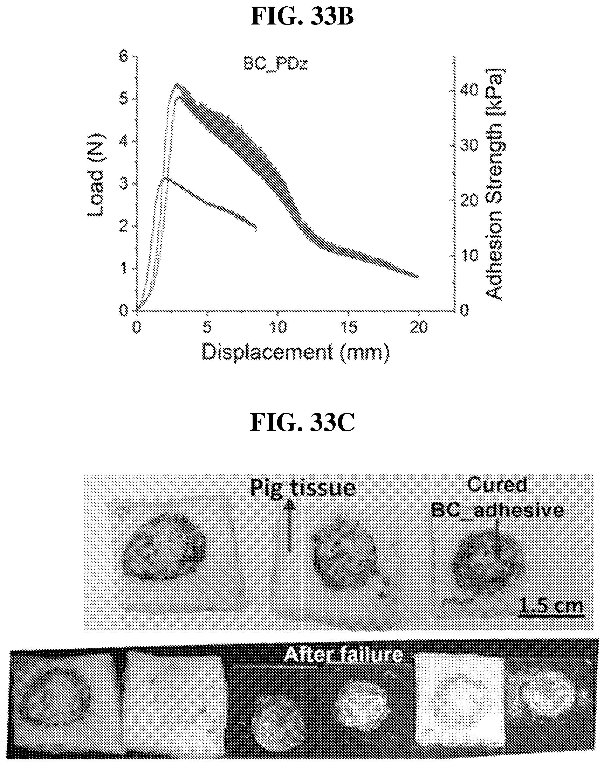

FIG. 33B is a plot of load versus displacement curves for BC_PDz patches against porcine skin.

FIG. 33C shows BC_caproglue activated through electrosurgery before and after lap shear.

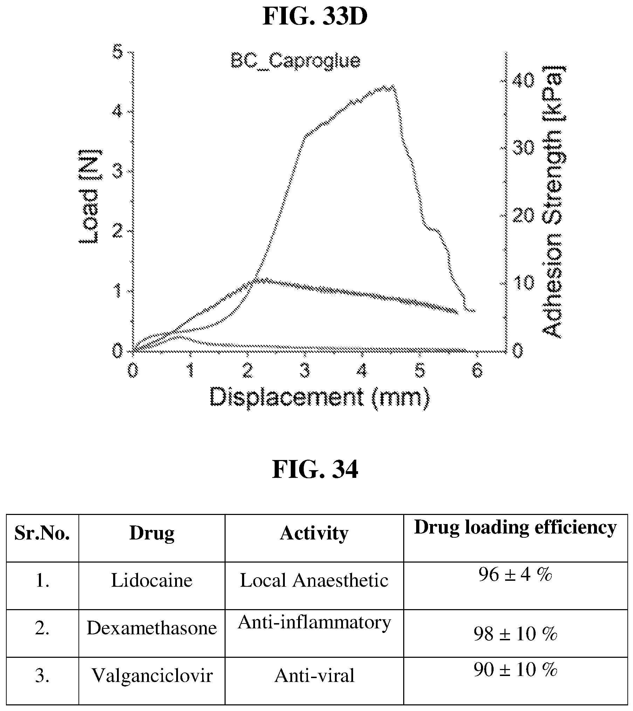

FIG. 33D is a plot of Load versus displacement curves for BC_PDz patches against porcine skin.

FIG. 34 is a table indicating a list of drugs loaded onto BC_PDz patches and the respective drug loading efficiencies.

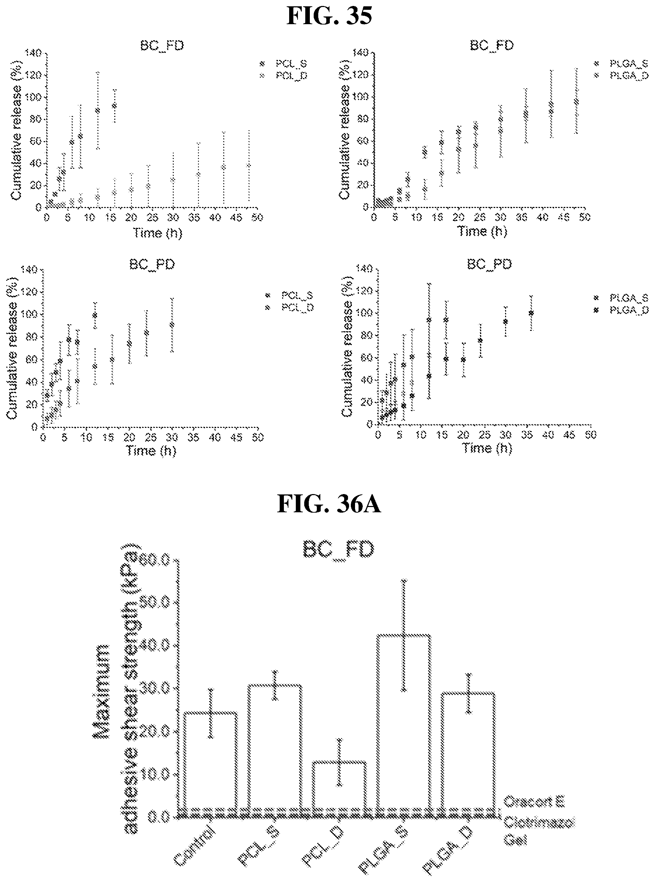

FIG. 35 shows release profile of valganciclovir from BC_PDz composites coated with polymers (PCL, PLGA). S represents single coating, D represents double coating. Top left plot shows for BC_FD coated with PCL, top right plot shows for BC_FD coated with PLGA, bottom left plot shows for BC_PD coated with PCL, and bottom right plot shows for BC_PD coated with PLGA. BC_FD represents freeze-dried BC films and BC_PD represents press-dried BC films. Data presented as mean ± SD, n = 3.

FIG. 36A is a plot of maximum adhesion strength at failure for drug loading BC_PDz composites with varying coating characteristics for BC_FD coated with PCL and PLGA. Data presented as mean ± SD, n = 5. BC_FD represents freeze-dried BC films. S represents single coating, D represents double coating.

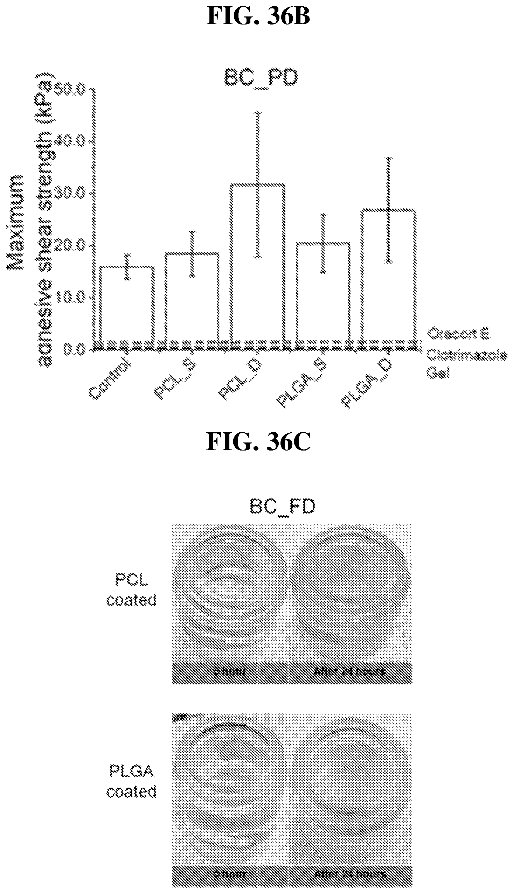

FIG. 36B is a plot of maximum adhesion strength at failure for drug loading BC_PDz composites with varying coating characteristics for BC_PD coated with PCL and PLGA. Data presented as mean ± SD, n = 5. BC_PD represents press-dried BC films. S represents single coating, D represents double coating.

FIG. 36C shows the ex vivo mucoadhesion time test with porcine tissue and drug loaded BC_PDz composites (single coating) for 24 hrs performed at 1 cps viscosity for BC_FD coated with PCL and PLGA.

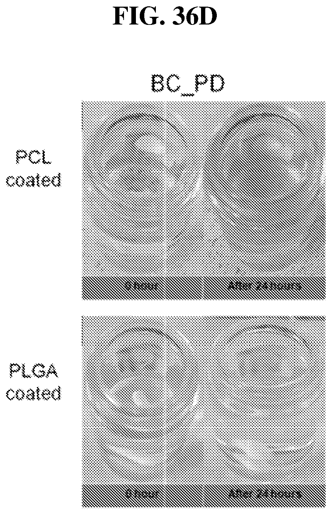

FIG. 36D shows the ex vivo mucoadhesion time test with porcine tissue and drug loaded BC_PDz composites (single coating) for 24 hrs performed at 1 cps viscosity for BC_PD coated with PCL and PLGA.

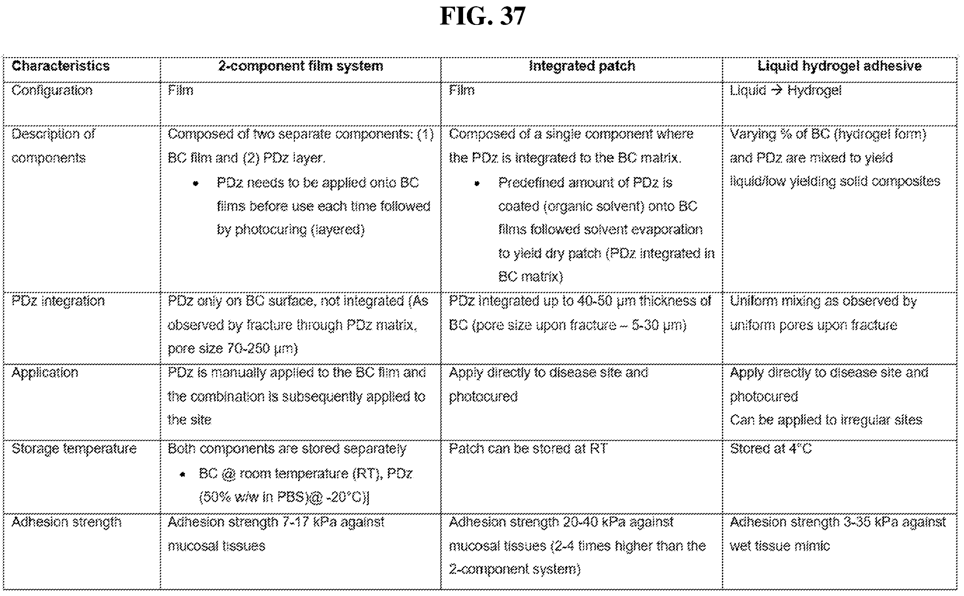

FIG. 37 is a table comparison of the various configurations of the mucoadhesive of the present disclosure.

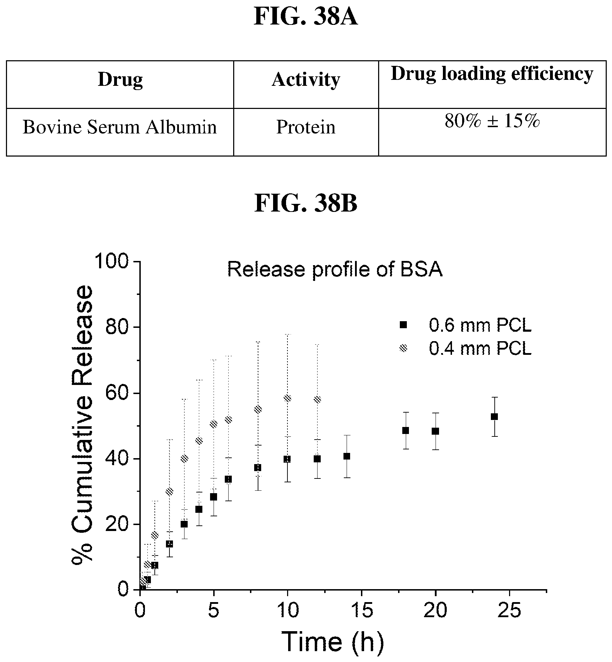

FIG. 38A is a table indicating bovine serine albumin (BSA), a non-limiting example of a pharmaceutical composition that was loaded in BC_PDz composite patches, and its loading efficiency.

FIG. 38B shows the release profile of BSA from BC_PDz composites coated with varying thickness of PCL. Data presented as mean ± SD, n =3.

## Detailed Description

The following detailed description refers to the accompanying drawings that show, by way of illustration, specific details and embodiments in which the present disclosure may be practised.

Features that are described in the context of an embodiment may correspondingly be applicable to the same or similar features in the other embodiments. Features that are described in the context of an embodiment may correspondingly be applicable to the other embodiments, even if not explicitly described in these other embodiments. Furthermore, additions and/or combinations and/or alternatives as described for a feature in the context of an embodiment may correspondingly be applicable to the same or similar feature in the other embodiments.

The present disclosure relates to a mucoadhesive and method of forming the mucoadhesive. The present mucoadhesive and method relates to an approach through covalent bonding that may provide enhanced adhesion strength and retention on a tissue surface. Film release then becomes dependent on natural epithelial sloughing, where complete renewal of the oral epithelium surface may occur over 5 days. This is advantageous over current mucoadhesives, which traditionally may be hydrogels that rely on weak forces for soft tissue fixation, for example, hydrogen bonds and van der Waals forces in order of decreasing bond strength.

The present mucoadhesive and method involves bacterial cellulose and a bioadhesive. As the present mucoadhesive is not composed of a single component but formed from multiple components, the present mucoadhesive may be herein interchangeably referred to as a "bioadhesive composite", "bioadhesive platform", "composite" and "system".

Bacterial cellulose (BC) is a naturally derived hygroscopic polymer which may be obtained from *Acetobacteraceae* family members, which absorbs 80-100 times water of its own dry weight. However, unlike other water swelling macromolecules, hydration reaches a plateau with retention of material properties. This inherent self-regulating water absorption ability of BC can be advantageous for moist environments like the oral cavity. The nanoporous structure provides a barrier with translucent properties for light transmission. Other industrial-friendly attributes include its production in the metric tons and edible nature attesting to low biotoxicity risks. It is sold commercially as a food product, *nata de coco,* justifying its safe use. BC-based wound dressings are also approved by FDA specifically for burns owing to its hydration and barrier properties.

The present mucoadhesive and method involve a bioadhesive. In various embodiments and examples herein, poly(amido amine) (PAMAM) dendrimer grafted with aryl-diazirine was utilized as one non-limiting example to demonstrate for the present bioadhesive platform for investigating covalent bonding of surface proteins upon wet surfaces. Diazirine generates reactive carbene crosslinking group upon activation with UVA (365 nm) or voltage. Various PAMAM-g-diazirine (PDz) formulations incorporating natural and synthetic polymers have resulted in tunable shear moduli of 1-250 kPa and maximum shear adhesion strength of 5-200 kPa when attached to collagen matrix or *ex vivo* tissue surfaces. However, PDz does not control for water swelling, but when PDz is integrated with BC (BC_PDz), the resultant bioadhesive platform has improved properties to handle water swelling. Hence, the present mucoadhesive may also be termed herein a "BC_PDz composite" or "BC_PDz adhesive". Apart from PDz, other bioadhesives that can form covalent bonding with a wet tissue surface can be used, e.g., those containing a moiety which generate a carbene upon activation.

The present mucoadhesive is assessed for enhanced wet soft mucosal tissue adhesion. The combination of self-regulating water absorption of BC, a bioadhesive that can form covalent bonds with a wet tissue surface (e.g. carbene-based crosslinking), and cohesive strength retention, yields a composite for adhesion to a variety of buccal and gastrointestinal ailments. Performance assessment prioritizes the structure activity relationship between ultraviolet A (UVA) energy and hydration state on lap shear adhesion strength. In the present disclosure, the hydration state may be controlled via two processes, i.e., freeze-drying and press-drying. Both processes advantageously retain the properties of BC without shrinkage, which allows for manageable film thicknesses between 30-250 μm, suitable for oral cavity applications, to be produced.

**EP 4 251 216 B1**

Details of various embodiments of the present mucoadhesive and advantages associated with the various embodiments are now described below. Where the advantages are already demonstrated in the examples further hereinbelow, they shall not be iterated for brevity.

In the present disclosure, there is provided a mucoadhesive. The mucoadhesive includes a bacterial cellulose and a bioadhesive incorporated to the bacterial cellulose. The bioadhesive includes a polymer or a dendrimer. The polymer and the dendrimer are grafted with a moiety which forms covalent interactions with a tissue surface in the presence of a stimulus, and wherein the moiety comprises a diazirine. The tissue surface may be a wet tissue surface, e.g. containing water or moisture. The tissue surface herein refers to any surface of a biological tissue.

In various embodiments, the polymer may include polycaprolactone, polyethylenimine, polyester, chitosan, etc. Other polymers which do not hinder covalent interactions with the tissue surface may be suitable.

In various embodiments, the dendrimer may include a poly(amidoamine) or polyethylenimine. A "dendrimer" is distinct from other classes of polymers (including the polymer mentioned hereinabove) in that the dendrimer has a plurality of dendritic chains emanating radially from a central core unit as compared to a polymer merely having entities extending from a polymer backbone wherein the moieties are not radially emanating therefrom. The term "poly(amidoamine)" may be herein abbreviated as "PAMAM". The poly(amidoamine) may be a G5-PAMAM dendrimer (5th generation, molecular weight (Mw) = 28.8 kDa)

The moiety includes a diazirine. The moiety is a moiety capable of forming covalent interactions with a tissue surface via carbene-based crosslinking. The moiety includes a diazirine, and the polymer or the dendrimer may include polycaprolactone or a poly(amidoamine) dendrimer, respectively.

In certain non-limiting embodiments, the moiety may include an electron donating moiety capable of oxidizing into a ketone (e.g. a monoketone or diketone) when subject to a stimulus. Such electron donating moiety may include, and is not limited to, 1,2-aryl-diol (e.g. a catechol or a catechol variant (e.g. a triol)), 1,4-aryl-diol, a vanillin, a derivative thereof, etc. In this instance, the stimulus may include water (e.g. moisture, water containing oxygen, saliva, mucus), an oxidant, an electrical voltage, or a combination thereof. In instances where the mucoadhesive contains such a moiety and/or is activated by water, such a mucoadhesive may be termed herein "water-activated mucoadhesive". In certain non-limiting embodiments, the electron donating moiety may be attached directly on an electron accepting moiety that is in turn directly on the polymer and/or the dendrimer. The electron accepting moiety advantageously gets reduced as the electron donating moiety gets oxidized for cross-linking and renders the present adhesive composition operable as an adhesive. The electron accepting moiety may include, and is not limited to, an imine, an alkene, an oxadiazole, a tetrazinealkyne, a fluoren-9-one, a thioazole, or a malononitrile.

In various embodiments, the bacterial cellulose may be a film including a network of cellulose nanofibers. In various embodiments, the bacterial cellulose may be dried or rehydrated.

In various embodiments, the bioadhesive may be deposited on the bacterial cellulose. In certain non-limiting embodiments, the bioadhesive may be deposited solely on the bacterial cellulose. Said differently, having the bioadhesive deposited on the bacterial cellulose means that the bioadhesive is present only on the surface of the bacterial cellulose, i.e. not present within the bacterial cellulose (the bacterial cellulose may be internally absent of the bioadhesive).

In certain non-limiting embodiments, the bacterial cellulose may be a hydrogel. In embodiments where the bacterial cellulose is a hydrogel, the bioadhesive may be present in an amount of 10 wt% to 35 wt%, 10 wt% to 33.3 wt%, 20 wt% to 33.3 wt%, etc. of the mucoadhesive.

In certain non-limiting embodiments, the bioadhesive may be included in the bacterial cellulose. In certain non-limiting embodiments, the bioadhesive may be included in the bacterial cellulose and may be present on the surface of the bacterial cellulose. In other words, the bioadhesive may be present within the bacterial cellulose and externally on the bacterial cellulose. In certain non-limiting embodiments, the bioadhesive may be solely within the bacterial cellulose, i.e. absent on the surface of the bacterical cellulose or externally absent on the bacterial cellulose. In certain non-limiting embodiments, the mucoadhesive may be an integrated patch wherein the bioadhesive is included within the bacterial cellulose. In embodiments where the bioadhesive is present within the bacterial cellulose, e.g. the integrated patch, the bioadhesive may be present in an amount of 15 wt% to 60 wt%, 20 wt% to 40 wt%, etc. of the mucoadhesive.

In various embodiments, the bacterial cellulose is of a species derived from a family of *Acetobacteraceae* (*Acetobacteraceae* family), which include and is not limited to, *Acetobacter, Gluconacetobacter, and/or Komagataeibacter* species.

In various embodiments, the bacterial cellulose may be optically pervious to light having a wavelength ranging from 280 nm to 1100 nm.

In various embodiments, the stimulus may be moisture, a chemical (e.g. water with or without oxygen), a biological fluid (e.g. saliva, mucus), an electrical and/or an optical stimulus. In various embodiments. the stimulus may include light having a wavelength ranging from 280 nm to 500 nm, and/or a voltage ranging from -10 kV to 10 kV. Advantageously, the present optical stimulus of using light having such wavelengths avoid cancer-causing wavelengths of 100 nm to 250 nm.

The present mucoadhesive may further include a pharmaceutical drug incorporated to the mucoadhesive. The pharmaceutical drug may include peptide or protein drugs. In certain non-limiting embodiments, the pharmaceutical drug may be incorporated to the bacterial cellulose. Non-limiting examples of the pharmaceutical drug include lidocaine, dexamethasone and valganciclovir. The present mucoadhesive may further include a polymeric coating, for example, on the bacterial cellulose. Non-limiting examples of the polymeric coating include polycaprolactone (PCL) and poly(lactic-co-glycolic acid) (PLGA). The polymeric coating may reside on any surface of the bacterial cellulose. Said differently, the polymeric coating may be configured between the bacterial cellulose and the bioadhesive, such that the bioadhesive is deposited on the polymeric coating. In certain instances, the polymeric coating may reside on the bioadhesive, for example, the polymeric coating may reside on one side of the bioadhesive and the bacterial cellulose resides on the opposing side of the bioadhesive away from the polymeric coating. This configuration may be denoted as, for example, BC//bioadhesive//polymeric coating.

The present disclosure also relates to a method of forming the mucoadhesive described in various embodiments of the first aspect. The method may include providing a bacterial cellulose, providing a bioadhesive including a polymer or a dendrimer, wherein the polymer and the dendrimer are grafted with a moiety which forms covalent interactions with a tissue surface in the presence of a stimulus, and wherein the moiety comprises a diazirine, and incorporating the bioadhesive to the bacterial cellulose. Embodiments and advantages described for the present mucoadhesive of the first aspect can be analogously valid for the present method subsequently described herein, and vice versa. As the various embodiments and advantages have already been described above and demonstrated in the examples further hereinbelow, they shall not be iterated for brevity.

In various embodiments, providing the bacterial cellulose may include inoculating bacteria in one or more culture media, extracting raw bacterial cellulose from the one or more culture media, and/or treating the raw bacterial cellulose with an alkali to remove the bacteria from the bacterial cellulose.

In various embodiments, providing the bacterial cellulose may include freeze-drying or press-drying the bacterial cellulose.

The present method may further include rehydrating the bacterial cellulose which is freeze-dried or press-dried prior to incorporating the bioadhesive.

In various embodiments, providing the bioadhesive may include grafting a diazirine on a polycaprolactone or a poly(amidoamine) dendrimer, and mixing the polycaprolactone or the poly(amidoamine) dendrimer grafted with the diazirine in a buffer. Other suitable polymers or dendrimers and moieties as described in various embodiments of the mucoadhesive of the first aspect may be used.

In certain non-limiting embodiments, incorporating the bioadhesive to the bacterial cellulose may include depositing the bioadhesive on the bacterial cellulose. In certain non-limiting embodiments, the bioadhesive may be deposited solely external of the bacterial cellulose. Said differently, the bioadhesive may be absent within the bacterial cellulose and deposited only on the surface of the bacterial cellulose.

In certain non-limiting embodiments, incorporating the bioadhesive to the bacterial cellulose may include contacting the bacterial cellulose with an organic solvent containing the bioadhesive, and removing the organic solvent to have the bioadhesive included in the bacterial cellulose. This may help in forming the integrated patch mentioned above in certain non-limiting embodiments of the mucoadhesive of the first aspect.

In certain non-limiting embodiments, incorporating the bioadhesive to the bacterial cellulose may include mixing the bacterial cellulose and the bioadhesive to form a hydrogel.

In various embodiments, providing the bacterial cellulose may include mechanically or chemically treating the bacterial cellulose to render cellulose nanofibers. For example, providing the bacterial cellulose may include sonicating the bacterial cellulose, which helps in the forming of the hydrogel.

The present method may further include a polymeric coating on the bacterial cellulose.

In summary, the present BC-based mucoadhesive is biocompatible and edible which makes it a good candidate for oral applications. BC has FDA GRAS status. Hence, the present BC-based mucoadhesive may allow for localized and extended delivery of therapeutics to the target sites in the oral cavity, i.e. an approach that addresses (prevents) uncontrolled absorption of moisture, provides attractive forces, covalent bonding, or combinations thereof. which would lead to mucoadhesive fixation for predetermined periods. Advantageously, due to the better adhesion from covalent bonding and control over water absorption, prolonged retention can be achieved on a wet tissue surface which in turn confers increased contact time at the oral lesion, extending therapeutic doses and providing a wound barrier.

The word "substantially" does not exclude "completely" e.g. a composition which is "substantially free" from Y may be completely free from Y. Where necessary, the word "substantially" may be omitted from the definition of the present disclosure.

In the context of various embodiments, the articles "a", "an" and "the" as used with regard to a feature or element include a reference to one or more of the features or elements.

In the context of various embodiments, the punctuation "~", term "about" and "approximately" as applied to a numeric value encompasses the exact value and a reasonable variance.

As used herein, the term "and/or" includes any and all combinations of one or more of the associated listed items.

Unless specified otherwise, the terms "comprising" and "comprise", and grammatical variants thereof, are intended to represent "open" or "inclusive" language such that they include recited elements but also permit inclusion of additional, unrecited elements.

**Examples**

The present disclosure relates to a self-regulating mucoadhesive and a method of forming thereof. The present mucoadhesive provides numerous advantages over existing mucoadhesive formulations.

Traditionally, topical approaches to oral diseases may require frequent dosing due to limited wound retention times. The present mucoadhesive serves as a drug delivery platform applicable to oral wound sites with soft tissue adhesion capability of up to 7 days. The present mucoadhesive and method involve, as non-limiting examples, bacterial cellulose (BC) and photoactivated carbene-based bioadhesives combined to yield a flexible film platform for interfacing soft tissues in dynamic, wet environments. Structure-activity relationships are evaluated via UV dose and hydration state with respect to adhesive strength on soft tissue mimics. The present mucoadhesive, which may be exchangably termed herein "bioadhesive composite", can have an adhesion strength ranging from 7-17 kPa and duration exceeding 48 hours in wet conditions under sustained shear forces. This is advanageous over mucoadhesives based on hydrophilic macromolecules which unfortunately exhibits adhesion strength of 0.5-5 kPa and last only a few hours. The examples hereinbelow describe evaluations of the mucoadhesive treatments in the buccal cavity.

The present mucoadhesive and method are described in further details, by way of non-limiting examples, as set forth below.

**Example 1A: General Discussion on Method of Present Bioadhesive Production Including Bacterial Cellulose Production and Characterization**

Examples 1A to 1C are generally intended to demonstrate non-limiting examples of the composition and utility of bacterial cellulose (BC) and the present photoactivated bioadhesive for adhesion to wet and buccal tissue surfaces. BC is used herein as a self-regulating, water-absorbing polymer. PAMAM-g-Diazirine (PDz) bioadhesive provides covalent bonding to wet tissue substrates but has limited adhesion strength to buccal tissues due to fouling by the immobilized water layer. Water competes with macromolecule covalent bonding. BC removes and absorbs water from the tissue surface, increasing the probability of covalent interactions between bioadhesive and tissue grafted macromolecules (e.g., glycoproteins, proteins within the extracellular matrix).

BC films are produced using 5% inoculum of *Gluconacetobacter hansenii 53582* sp. in 2 ml HS (Hestrin-Schramm) media inoculated in 24 well plates. The films produced after 5 days of culture were used for further studies. The produced wet films weighed around 400-600 mg and the diameter of the films was found to be around 1.5 $\pm$ 0.1 cm. In this study, freeze-drying (BC_FD films, 160 $\mu$m thickness) and press-drying (BC_PD films, 30 $\mu$m thickness) methods are employed to obtain dry BC films as they do not require any chemicals, help retain the properties of BC without shrinkage of nanofibers, and produce films with thickness 30-250 $\mu$m suitable for oral cavity applications.

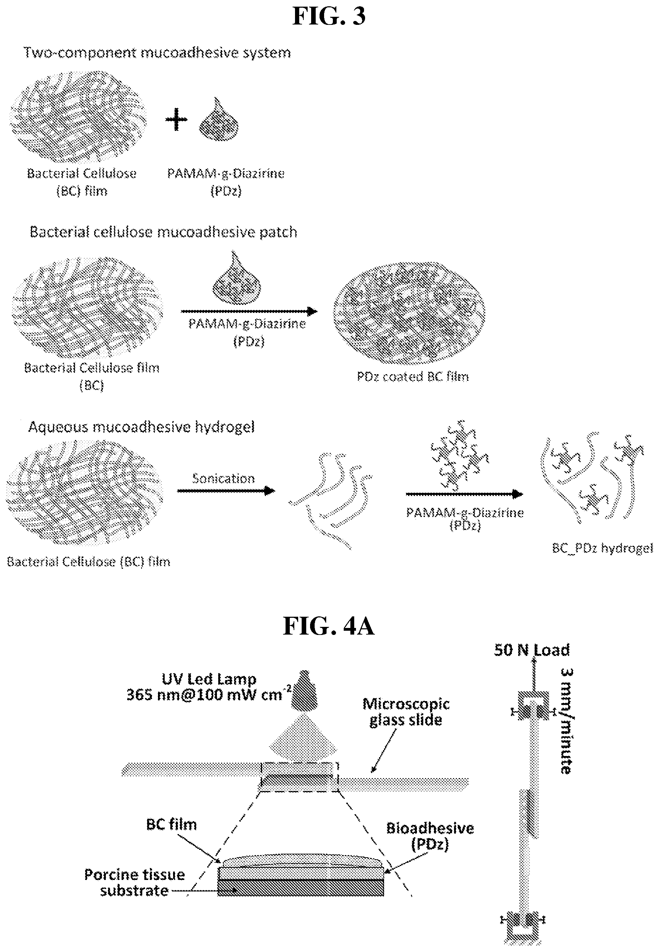

Grafting of diazirine to PAMAM backbone is performed. In brief, a PAMAM solution in methyl alcohol (0.5% w/v) was prepared and diazirine was added directly into 5 mL of the PAMAM solution (according to theoretical calculations). The conjugation reaction was set for 36 hours with stirring under room temperature. Methyl alcohol was removed by vacuum and PAMAM-g-diazirine was collected as a pale yellow viscous liquid. PAMAM-g-diazirine with 20% conjugation and 50% concentration in PBS is employed for further experiments. BC_PDz composites are formed by adding varying moles of PDz to BC, either in film or hydrogel form as depicted in FIG. 3. The adhesive system can be in the form of adhesive film, adhesive hydrogel or a combination of drug loaded film adhered with help of bioadhesive component. The wavelength 365 nm has been employed for photoactivation of PDz bioadhesives in this study. An increase in the light transmission ability of both BC_FD and BC_PD is observed with the increase in the hydration percentage. The films exhibit maximum light transmittance at 200% hydration, thus it is used in further studies. In case of BC_PD percentage light transmitted varies from 22-40 % while for the BC_FD film it varies from 7-22% for different wavelengths and different hydration percentages. The BC_PD films transmit about 2-3 folds light when compared to that of BC_FD films. BC films exhibit high storage modulus ranging from 40-200 kPa in native as well as rehydrated state, with or without presence of PDz.

Example 1B: Utility on Wet Tissue Surfaces

This example demonstrates the BC films exhibit UV dose based tunable adhesion with strength of 6-22 kPa against wet porcine tissue.

The adhesion strength of photocured BC films with PDz bioadhesive is determined using lap shear test against porcine cheek skin as illustrated in FIG. 4A. Three UV doses 3J, 6J and 18J are applied by exposing the films with 365 nm UVA of 100 mW cm$^{-2}$ intensity for varied time periods (30, 60, 180 seconds, respectively). The time period 30-180 seconds is selected as the general curing time in dentistry varies from 5-100 seconds with light intensity ranging from 300-2000 mW cm$^{-2}$, dose of 8-50 J/cm$^2$ for lights with 350-500 nm wavelengths. FIG. 4B and 4C compare the maximum adhesion strength at failure for dry and rehydrated BC films, respectively, with varying UV doses. The wet environment in the oral cavity due to constant salivary flow will lead to hydration of films upon application. Thus, the films are evaluated in dry and rehydrated state to evaluate the effect of hydration on adhesion performance.

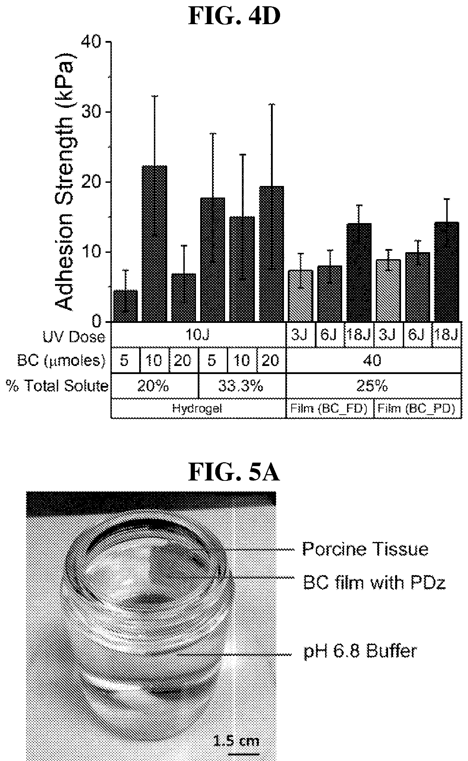

It is found that BC films exhibit tunable adhesion based on the type of BC film (BC_PD, BC_FD), hydration state (dry, rehydrated) and UV dose. The average adhesion strength for BC_FD for varying applied UV dose ranges from 5.9-8.3 kPa for dry films and 7.3-14 kPa for rehydrated films (FIG. 4B). On the other hand, mean adhesion strength for BC_PD films for varying UV doses ranges from 10.4-16.7 kPa (FIG. 4C) which is almost double the adhesion strength exhibited by BC_FD films in dry state. Mean adhesion strength in case of BC_PD Rh ranges from 8.8-14.2 kPa which is similar to that exhibited that by BC_FD Rh (rehydrated freeze-dried BC films) as depicted in FIG. 4B. It is observed that increased UV dose leads to enhanced adhesion strength for BC_FD and BC_PD films in both dry and rehydrated state which leads to the inference that BC films do not lose adhesion properties in wet conditions. Similarly, BC_PDz hydrogel systems with varying moles of BC and total solute percentage exhibit adhesion strength of 6-22kPa in wet environments as presented in FIG. 4D.

Diazirine functionalized PLGA films have also been evaluated for adhesion to tissues in wet environment with average lap shear adhesion strength of 4.5 kPa. Injectable mucoadhesive system based on chitosan grafted dihydrocaffeic acid and oxidized pullulan have been reported to exhibit average adhesion strength of 5 kPa. Maximum adhesion strengths of previously reported film based mucoadhesive systems have been reported to be 0.65 kPa for polyacrylates based system, 0.2-0.55 kPa (assuming 2 cm$^2$ film area) for chitosan and hydroxyethylcellulose blend, 1-2.7 kPa for Eudragit®, carboxymethylcellulose (CMC) and hydroxymethylcellulose blends, 4-10 kPa for polyvinylpyrrolidone (PVP) and CMC blend and 0.5-2 kPa (assuming 2 cm$^2$ film area) for various natural gum based films. Herein, it is demonstrated higher adhesion strength ranging from 6-22 kPa exhibited by BC films adhered with the aid of PDz bioadhesive compared to existing film based adhesive systems.

## Example 1C: Extended Duration of Fixation within Wet Environments under Physiological Stresses

The present example involves an ex vivo mucoadhesion study that reveals adhesion time of 2-7 days in viscous aqueous environment.

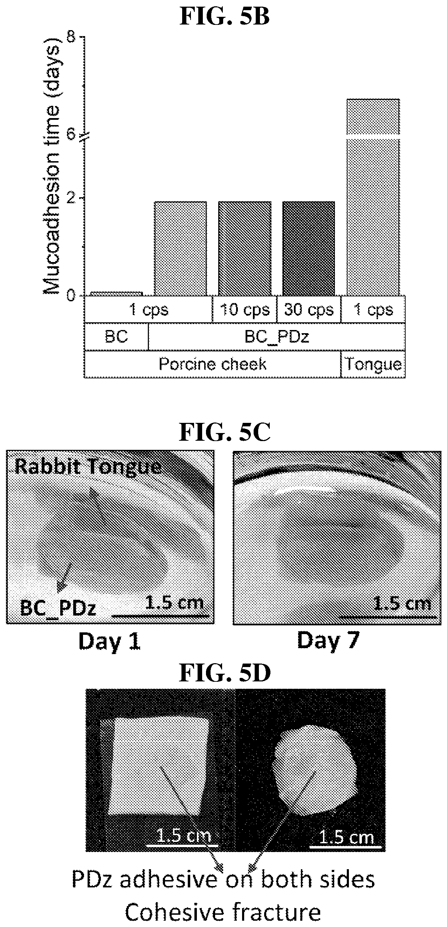

The present example aims to investigate the adhesive behaviour of BC films with PDz in aqueous environment for long durations. FIG. 5A depicts the experimental setup wherein BC film with PDz is adhered to porcine tissue and applied with constant shear forces of around 0.5-1 kPa. It is observed that BC films without PDz (control) remained adhered to the tissue for about 2 hours. Contrary to that, BC films adhered with the help of PDz are found to be adhering for 48 hours, which is the point where film is peeled away and observed for fracture. The same study is performed in solutions of three different viscosities, 1, 10 and 30 cps considering the variations in saliva viscosity of patients and it is found that BC films with PDz could adhere up to 48 hours, maximum study time (FIG. 5B). The study is then performed with rabbit tongue in 1 cps viscosity solution and extended for 7 days. It is found that films kept adhering to the tissue until day 7 as observed in FIG. 5C. Moreover, the films maintained their structural integrity even after being exposed to aqueous environment for 7 days as can be seen in the images. No antimicrobial agent was added to the media during the study, thus suggesting that microbial load does not hamper the adhesion of films to the tissues, important in case of infectious wounds. Moreover, upon removal the films exhibit cohesive failure within the bioadhesive matrix as visible in FIG. 5D, wherein bioadhesive is present on the film as well as tissue. The cohesive failure implies that the tissues are not damaged/teared off upon removal of mucoadhesive system.

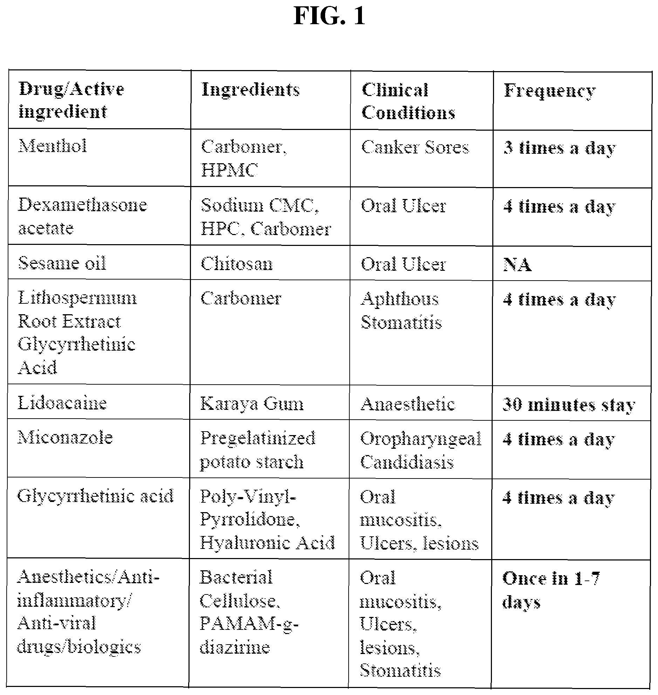

Similar synthetic electrospun nanofiber membranes formed of dextran and poly(ethylene oxide) as adhesive agents have been reportedly evaluated for their potential as mucoadhesive membranes for targeting oral cavity. The membranes exhibited *in vitro* and *ex vivo* mucoadhesion time of 200-250 minutes (4 hours approximately). Another study reported synthesis of mucoadhesive hydrogels wherein catechol was covalently bonded to chitosan further crosslinked with genipin exhibiting *in vitro* mucoadhesion time of 6 hours. Another reported study described the synthesis of sulfhydryl anchored adhesive alginate for management of oral aphthous stomatitis which exhibited mucoadhesion time of 100 minutes. The frequency of currently marketed mucoadhesives as mentioned in FIG. 1 is 3-4 times a day, suggesting adhesion time of 6-8 hours. The reported BC_PDz patch exhibits *ex vivo* mucoadhesion time of 2-7 days which is about 8-20 times higher than that of currently researched systems.

## Example 2A: Materials and Methods - Demonstration for Oral Cavity Applications

Examples 2A to 2T are generally intended to demonstrate non-limiting examples of the present mucoadhesive and method for oral cavity applications.

Polyamidoamine (PAMAM) dendrimer, Generation 5, Mw = 28.8 kDa, was purchased from Dendritech, Inc., USA and 3-[4-(bromomethyl) phenyl]-3-(trifluoromethyl)-diazirine(bromodiazirine) from TCI, Japan. All other chemicals were purchased from Sigma-Aldrich unless specified otherwise.

Strains and culture medium: *G.hansenii* 53582 ATCC was utilized for the fermentation of BC in *Hestrin Schramm* (HS) medium consisting of glucose (20 g L$^{-1}$, 0.11 M), citric acid monohydrate (1.15 g L$^{-1}$, 0.005 M), Na$_2$HPO$_4$ (2.7 g L$^{-1}$, 0.02 M) enriched with yeast extract (5 g L$^{-1}$) and peptone (5 g L$^{-1}$) with final pH 4.5. HS media is prepared and autoclaved at 120 °C for 15 min.

### Example 2B: Microbial Fermentation

For the production of inoculum, bacterial strain *Gluconacetobacter hansenii* ATCC 53582 is inoculated in the culture media (HS Media) for 24 hours in shaking condition at 26°C. *G.hansenii* is later inoculated in 5% (v/v) HS basic medium and cultured in 24-well plates at 26 °C for 5 days. The obtained BC pellicles are treated with sodium hydroxide solution (0.25 N) for 30 minutes at 60 °C, and then washed thoroughly with deionized water for removal of bacterial cells (confirmed with protein determination - see example 2C). The films are then dried using lyophilizer (Alpha 2-4 LSC basic freeze dryer) which are later denoted as Freeze-dried BC (BC_FD). The other set of films are dried under pressure using bloating paper and designated as Press-dried BC (BC_PD). Both kinds of films are evaluated for mechanical properties (Mechanical Tester Instron 5567 - see example 2D), light transmission properties (see example 2E) and structural morphology (Scanning electron microscopy, JEOL JSM-6700 FESEM) (see example 2F).

### Example 2C: Determination of Protein Content in BC Films

300 mg of BC native pellicle corresponding to 3.9 mg of dry BC is extracted in 20 ml of PBS buffer by sonication. The PBS extract is assayed for protein content using Thermo Scientific™ Micro BCA™ Protein Assay Kit. The microplate reader procedure with working range of 2-40 $\mu$g/ml is used for the assay. The protein concentration in extract was found to be 9.3 $\pm$ 0.2 $\mu$g/ml. Thus, the total protein extracted is (9.3x20) 186 $\mu$g, which is approximately 48 $\mu$g/mg of dry cellulose.

### Example 2D: Tensile Test of BC Films

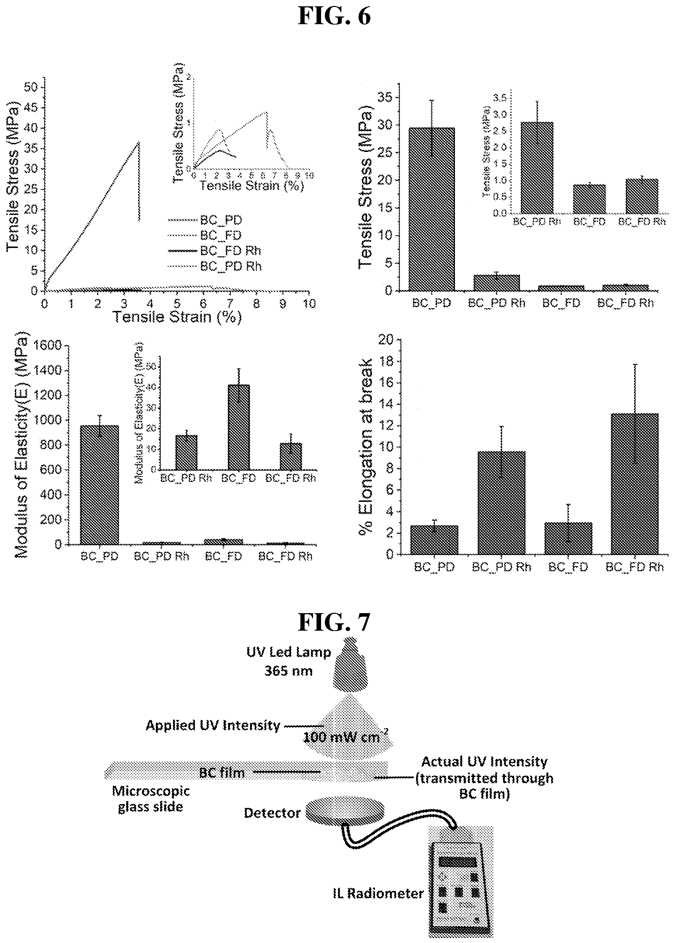

The tensile strength of the FD, FD Rh, PD and PD Rh BC films is determined using Instron 5567 Electromechanical Tester. The films of rectangular shape with average gauze length of 7 cm and width of 1.5 cm according to ASTM standards are prepared. The thickness of freeze-dried and press-dried films was determined before the experiment. Tension Test Method was used for determining the tensile strength with load cell of 500 N and Tensile Extension of 3mm/min. Rate of Load (50% Sensitivity) was set as the end of test criteria. The results were expressed as average of 3 test specimens and shown in FIG. 6.

### Example 2E: Light Transmission Properties of BC Films

The light transmission properties of different kinds of BC films is determined using the setup shown in FIG. 7. LED light sources with different wavelength (365 nm, 385 nm and 405 nm) are set to emit light with intensity of 100 mW/cm$^2$ detected through the radiometer. The sample is sandwich between two slides and placed near the light source. Then the LED is switched on and the light transmitted through the sandwich setup is detected using International Light Radiometer/Photometer Model 1400A. The light transmitted through single slide and two slides without the film sample is also measured as a control. The percentage hydration is defined as amount of water added as a fraction of the total weight of the dry film as shown in equation (S1) below. Said differently, equation (S1) below refers to controlled hydration by adding defined amount of PBS to achieve a certain hydration % with respect to BC dry weight.

$$\% \text{hydration} = \frac{Total\ weight\ of\ hydrated\ film - weight\ of\ dry\ film}{weight\ of\ dry\ film} * 100 \qquad (S1)$$

### Example 2F: Morphological Analysis of BC films using SEM

The structural morphology of dried BC_FD and BC_PD films is observed using field emission scanning electron microscope (JEOL, JSM-6700 F, Japan) operating at an accelerating voltage of 5kV and 8 mm working distance. The samples are staged on stubs with the help of double-sided carbon tape, and sputter coated with platinum for 70 s, 20 mA current in vacuum (8-9 Pa) before observation.

### Example 2G: Synthesis of PAMAM-g-diazirine

Grafting of diazirine to PAMAM backbone is performed as described in example 1A. PAMAM-g-diazirine with 20% conjugation and 50% w/w concentration in phosphate buffered saline (PBS) with pH 7.2, is employed for further experiments.

### Example 2H: SEM Imaging

BC_PDz films adhered to PET sheets are cured at UV dose of 18 J cm$^{-2}$ (365 nm, 100 mW.cm$^{-2}$). After curing, the PET sheet is peeled away to expose the cohesively fractured surface of PDz. The structural morphology of these fractured surfaces is observed using field emission scanning electron microscope (JEOL, JSM-6700 FESEM) operating at an accelerating voltage of 5kV and 8 mm working distance. The samples are staged on stubs with the help of double-sided carbon tape, and sputter coated (JEOL JFC-1600 Auto Fine Coater) with platinum for 70 s, 20 mA current in vacuum (8-9 Pa) before observation. Image J software is employed to determine the pore length and pore number.

### Example 2I: Lap Shear Adhesion Assessment Against Porcine Tissues

Porcine jowl skin (2x1.5 cm$^2$, 0.2 cm thickness) obtained from local suppliers and BC films (2 cm$^2$) are subsequently mounted onto glass slides using cyanoacrylate glue. PDz (~4 mg, 30 $\mu$m thickness) sandwiched between BC films and porcine tissue is photocured (365 nm, 100 mW.cm$^{-2}$) for 30 s, 60 s, or 180 s. Adhesion strength test of BC_PDz films is determined by a modified tensile tester (Chatillon force measurement, Largo, FL, USA) with constant linear speed of 3 mm/min with 50N loading cell (n = 5).

### Example 2J: FTIR Analysis of Cured and Uncured BC PDz Films

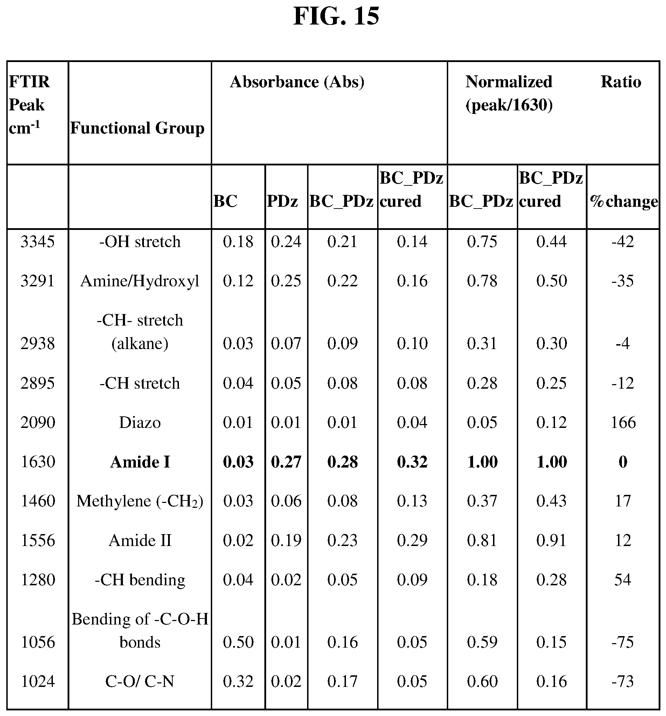

FTIR (PerkinElmer Frontier) measurements are performed with Universal ATR fixture of diamond crystal. Photocured and uncured BC films with PDz are placed directly onto the crystal. The samples are scanned over a range of 4000 to 600 cm$^{-1}$ with a resolution of 4 cm$^{-1}$ and presented as an accretion of 32 scans. FIG. 15 shows the FTIR spectra of BC_PDz before and after photocuring, and additionally the absorbance. Amide I is the carbonyl stretching vibrations. FTIR peak at 1630 cm$^{-1}$ is set as control for peak ratio comparison. % change= [cured ratio - uncured ratio]/uncured ratio * 100%.

### Example 2K: Ex-vivo Mucoadhesion Time and Measurement of Shear Stress and Normal Force on the Tissue Substrate for Ex Vivo Mucoadhesion Study

Ex vivo mucoadhesion time is assessed for BC films with aid of PAMAM-g-diazirine (PDz). The porcine tissue (2x2 cm$^2$) is attached to the internal side of the beaker using cyanoacrylate glue, and the BC films are adhered to tissue with the aid of PDz. The beaker is filled with 50 ml of the 50 mM phosphate buffer (pH 6.8) with magnetic stirring rate of 200-300 rpm accounting for constant shear stress of approximately 0.072-1.08 Pa per minute. The duration of film adhesion is observed during a period of 2 days under solutions with apparent viscosities of 1, 10, and 30 mPa.s. The viscous liquids are composed of 0, 2 and 3% w/v with 20-40 kDa sodium alginate respectively. An extended analysis is performed with rabbit tongue (fresh tongue tissue of rabbit carcass donated from other PI project from SingHealth Experimental Medicine Centre, Singapore) and 1 mPa.s solution for 7 days period.

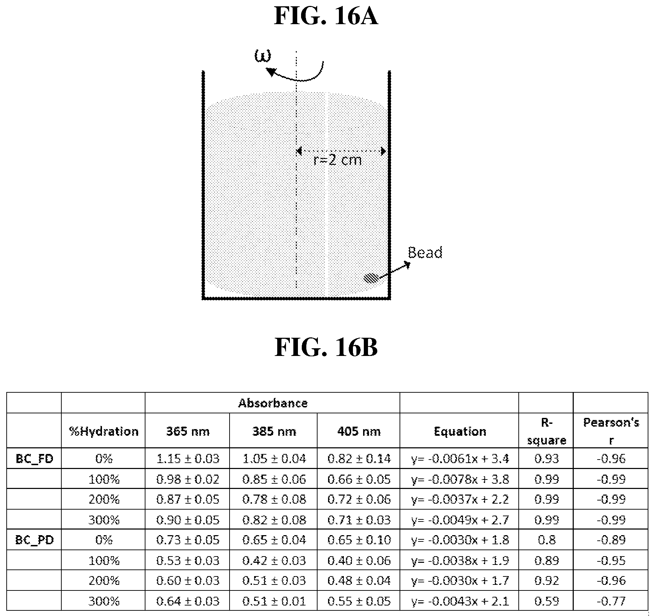

Measurement of shear stress and normal force on the tissue substrate were performed using a beaker of radius 2 cm as shown in FIG. 16A. The system was magnetically stirred and the angular velocity $\omega$ of the spherical bead revolving along the beaker wall was calculated by recording the revolutions per minute (rpm) of the bead using a digital camera. The shear rate is then calculated using the formula (v/h) where, v is the linear velocity of bead ($\omega$r) and h is the height (h) of bead equal to the diameter (2r). The shear stress at any point at the wall of beaker can be calculated by knowing the shear rate and viscosity of the fluid (1, 10, 30 cps). Thus, the shear stress is calculated using the following formula:

$$\text{Shear rate} = \text{velocity/height;}$$

$$\text{Shear stress} = \text{viscosity*shear rate;}$$

$\omega$ is the angular velocity calculated from the recorded rpm. The bead rpm is 24 for 1 cps fluid, 36 for 10 cps and 6 for 30 cps fluid.

Placing in the values here, the shear stress at the wall was found to be 0.072 Pa for 1 cps, 1.08 Pa for 10 cps and 0.54 Pa for 30 cps approximately every minute.

The friction coefficient of tongue against the soft tissues in the oral cavity is found to be 0.1-0.3.

$$\text{Friction coefficient} = \text{Shear force/Normal force}$$

Thus, placing the values of friction coefficient and shear force in the above equation, the normal force on the BC_PDz film adhered to the tissue at the wall of beaker would be around 0.01-0.2 N, equivalent to gram force of 1-22. (wherein in stress = Force/Area).

**Example 2L: Statistical Analysis**

All the data are presented in the form of mean $\pm$ SD (n=3, unless otherwise stated). OriginPro 2018 64-bit software is employed to determine significance using Two-way ANOVA with Bonferroni as post-hoc test. A $p < 0.05$ (*) is considered statistically significant.

**Example 2M: Discussion of Results for Oral Cavity Applications**

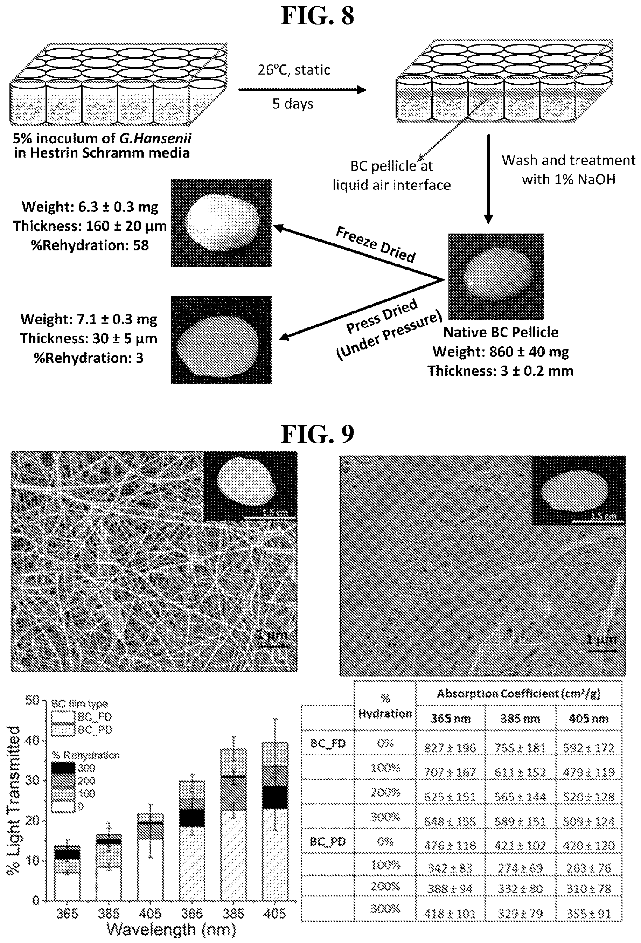

BC pellicles are obtained after 5 days of inoculation of G. *hansenii* under static conditions, and further washed (0.25 N NaOH, deionized water) to obtain native BC pellicle (FIG. 8). Before any composites are formulated, BC is first compared under its native pellicle state and then with respect to two methods of dehydration processing: freeze-drying and press-drying resulting in BC_FD and BC_PD, respectively (FIG. 8). Both drying methods lead to varying structural and mechanical characteristics of film, the effect of which on optical properties and adhesion strength is evaluated further. As dried state is expected to be a preferred storage under ambient condition, the UV/VIS optical characteristics as a function of wavelength and rehydration state are analyzed. PDz (50% w/w in PBS, ~4 mg) is topically applied to the dried BC films to form composite films. The films are evaluated for lap-shear adhesion dependence on rehydration and overall light dose (intensity x time = $W.cm^{-2}$ * seconds = $joules.cm^{-2}$). The pore size and density are determined by scanning electron microscopy (SEM) after photocuring. Surface chemistry is qualitatively evaluated with infrared spectroscopy to correlate functional groups with material properties. A final *ex vivo* mucoadhesion test in submerged saline is performed to evaluate the holding strength under constant shear stress for up to 7 days.

**Example 2N: Discussion of Results for Oral Cavity Applications - BC Transparency Increases Upon Hydration**

As photocuring is dependent on the UV light transmission through the BC films, the optical properties require moderate light transmission. The mass of a 16-mm diameter native BC pellicle is $860 \pm 40$ mg (FIG. 8). Upon drying, the BC films retain about 1% of their hydrated pellicle mass of $6.3 \pm 0.3$ mg and thickness of $160 \pm 20$ $\mu$m for freeze-dried preparation (BC_FD) and $7.1 \pm 0.3$ mg and thickness of $30 \pm 5$ $\mu$m for press-dried preparation (BC_PD). Rehydration of BC_FD and BC_PD films showed that dried films swell to about 59% and 4% of the native BC pellicles, respectively (see equations S2-S4 below). Equation S4 below refers to %hydration with respect to swelling upon incubation with excess PBS for 3 hours.

For the determination of swelling ratio of BC films, the dried films (BC_FD, BC_PD) are weighed and noted as $H_D$. The weighed films are then immersed in 5 ml of phosphate buffer saline (pH 7.4) at room temperature for 3 hours. The swollen films are then re-weighed and noted as $H_S$. The swelling ability of the films is calculated using the formula:

$$\text{Swelling Ratio} = (H_S - H_D)/ H_D \qquad\qquad (S2)$$

$$\text{Swelling Percentage} = (H_S - H_D)*100/ H_D \qquad\qquad (S3)$$

Table 1: Swelling ratio of BC films

|  | BC_FD | BC_PD |
|---|---|---|
| **Swelling Ratio (%)** | $8000 \pm 400$ % | $400 \pm 150$% |
| **Swelling Ratio** | $80 \pm 4$ | $4 \pm 1.5$ |

$$\%\text{Rehydration} = \frac{Hs*100}{Weight\ of\ native\ BC} \qquad\qquad (S4)$$

Electron micrographs of BC_FD and BC_PD surfaces reveal that freeze-dried films retain their nanofiber networks and nanoporous structure, which collapsed in press-dried films (FIG. 9).

Photocuring adhesives are typically activated by UVA with wavelengths ranging from 350 to 405 nm and thus require sufficiently transparent film for its activation. The experiment is conducted using representative UV LEDs of 365 nm, 385 nm, and 405 nm that spanned the range of photocuring wavelengths required of both diazirine and free-radical based bioadhesives. An increase in light transmission is observed with hydration, but no clear correlation is observed with hydration percentage. Not surprisingly, transparency also increases with respect to longer wavelengths due to reduced scattering. The BC_FD films exhibit maximum light transmittance at 200% hydration at 365 nm (required for PDz activation), while that for BC_PD films is at 100% hydration (not significantly different from 200%, p> 0.05). Thus, 200% hydration is chosen for the remaining studies. The detailed method for measurement of light transmittance through BC films is already described in example 2E above.

**Example 2O: Discussion of Results for Oral Cavity Applications - BC/PDz Composite Adhesives Exhibit 7-17 kPa Lap Shear Adhesion Strength Against ex vivo Porcine Tissue**

Constant salivary flow is expected to lead to instant hydration of films upon application to the diseased oral sites. The hydration of films affects the optical properties and subsequently might affect the adhesion properties. To examine the effect of hydration on adhesion performance, the films are evaluated in dry and rehydrated states. Hydration of the films led to variations in the light transmission properties of films (see FIG. 9 bottom left plot), thereby affecting the actual UV dose reaching the bioadhesive surface (FIG. 8). Thus, both kinds of films, BC_FD and BC_PD in dry and rehydrated (Rh) form, are assessed for their adhesion properties in combination with PDz with varying UV dose.

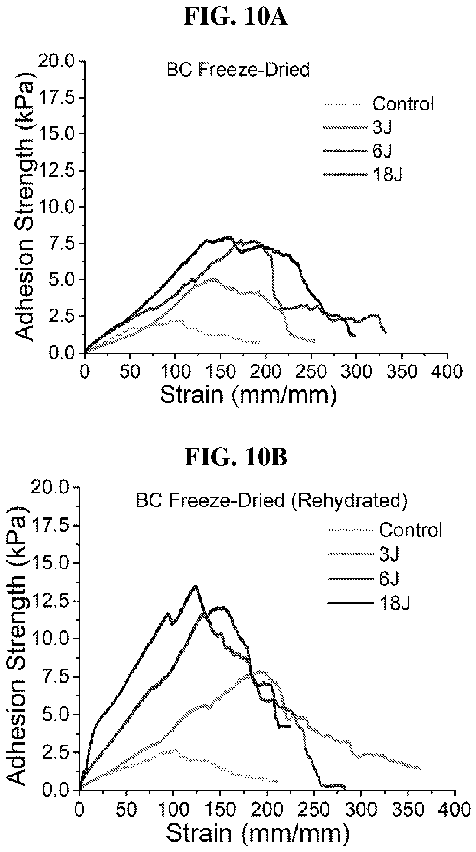

The adhesion strength of photocured BC films is determined using lap shear test against porcine cheek skin as illustrated in FIG. 4A. Three parameters are evaluated for their effects on adhesion strength: UV dose, method of dehydration, and rehydration state. UV dose applied for photocuring is correlated against the maximum adhesion strength at failure for BC_FD and BC_FD Rh films with PDz in FIG. 10A and 10B, respectively. Three UV doses of 3 J, 6 J, and 18 J (per cm$^{-2}$) are applied by exposing the films with 365 nm UVA of 100 mW.cm$^{-2}$ intensity. The applied dose is on par with that used in dentistry; 8-50 J/cm$^{-2}$ with 350-500 nm wavelengths. FIG. 4B compares the maximum adhesion strength at failure for dry and rehydrated FD films. The actual UV dose transmitted through BC_FD films is found to be 0.21, 0.42, and 1.26 J for applied doses of 3 J, 6 J, and 18 J, respectively. All three UV doses exhibit significantly higher adhesion strength as compared to the 0J control, with a maximum observed at 14 ± 3 kPa. Moreover, the adhesion strength for BC_FD exhibits a strong positive correlation with actual UV dose (Pearson's r = 0.81). The higher transparency of rehydrated films allows double joules exposure than dry films. Consequently, adhesion strength is twice as high at the 18 J UV exposure. However, similar increase is not observed for 3J and 6J UV exposure.

The top left and top right plots in FIG. 11 exhibit the lap shear stress versus strain curve examples for for the press-dried films (BC_PD). The bottom left plot of FIG. 11 (also see FIG. 4C) represents the mean adhesion strength for BC_PD and BC_PD Rh with varying applied and actual UV doses (19% - 25% light transmission). The rehydrated press-dried films displayed similar trends as described for BC_FD formulations. Both BC_PD and BC_PD Rh exhibit strong positive correlation with actual UV dose (Pearson's r = 0.91 and 0.88, respectively). A decrease in the mean adhesion strength is observed for BC_PD Rh, however it is not significantly different (p > 0.05) as compared to BC_PD films, thereby suggesting similar adhesion in dry and rehydrated states.

Mean adhesion strength for BC_PD films for varying UV doses ranges from 10.4-16.7 kPa which is almost double the adhesion strength exhibited by BC_FD (5.9-8.3 kPa) films in dry state, however not siginifically different. The bottom right plot of FIG. 11 displays both formulations had similar adhesion strengths despite the difference in the UV dose actual in rehydrated state. No significant difference is observed. Both BC_FD and BC_PD films exhibit linear elastic region followed by plastic region ultimately leading to delamination. However, no clear yielding point is observed. All the groups exhibit high strain values ranging from 100-350 which is characteristic of the test setup comprising of BC film, PDz adhesive, porcine tissue and glass slide, and cannot be attributed solely to PDz thickness sandwiched between tissue and BC film. The adhesion strength exhibited by previously studied mucoadhesives against wet tissue range from 0.5-5 kPa, which is about 2-3 times lower than BC_PDz film systems. BC_PDz films exhibit adhesion strength similar to photosensitive/electrosensitive PDz based tissue adhesives ranging from 5-40 kPa. The adhesion strength is also similar to biopolymer film/gel based surgical tissue adhesives like gelatin/glutaraldehyde films (24.5 kPa), gelatin/alginate adhesives (5-15 kPa), collagen/citric acid derivative (~19 kPa), gelatin/polysaccharides (dextran, chitosan, starch) (12-22kPa), gelatin/poly(ethylene glycol) diacrylate (12kPa), dopamine modified PEG/Laponite (8 kPa), and catechol-functionalized poly(ester urea) (9-10 kPa, similar to fibrin glue).

**Example 2P: Discussion of Results for Oral Cavity Applications** **- Films undergo Cohesive Fracture as Observed by Visual Inspection and SEM Micrographs**

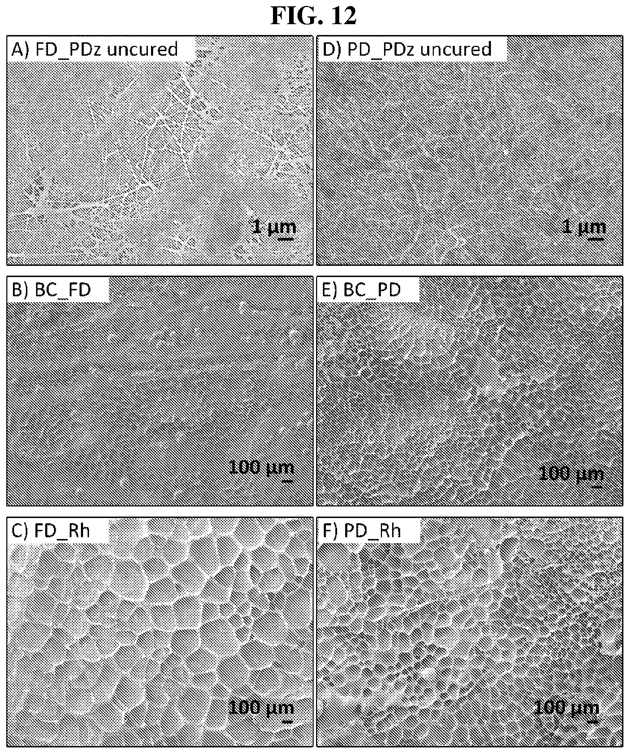

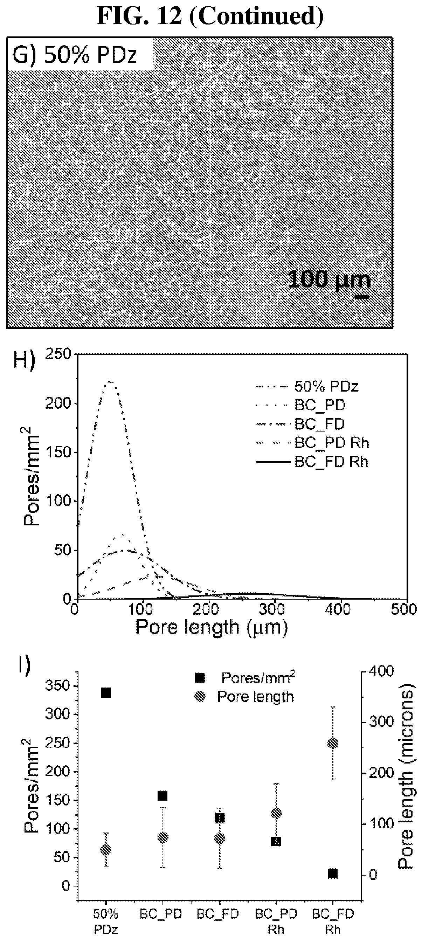

Surface morphology of the uncured and cured films is observed using SEM. Variations in terms of pore length and density are analyzed. FIG. 12A and 12D represent the SEM image of uncured BC_FD and BC_PD with PDz. It is observed that the nanofibers in both the cases are covered with a layer of PDz bioadhesive thus inferring that PDz penetrates to the top nanofiber layers of BC. FIG. 12B and 12C display SEM images of the cohesively fractured BC_FD and BC_FD Rh films after photocuring. BC_FD exhibits non-uniform distribution of pores, with an average pore length of 70 $\mu$m as opposed to BC_FD Rh which exhibit uniformly distributed pores with pore length ranging from 120-250 $\mu$m when compared to BC_FD. Similar trend is observed in FIG. 12E and 12F representing SEM images of BC_PD and BC_PD Rh wherein average pore length of 75 $\mu$m is observed for BC_PD and 120 $\mu$m for rehydrated BC_PD films. The adhesion failure occurs cohesively through the PDz bioadhesive matrix, where brittle fracture likely occurs through the pores. FIG. 12H and 12I display the pore size distribution vs. pore length and pore count per formulation. The rehydrated films exhibit the highest pore length, and lowest pore count. Overall, pore length increases with hydration. However, pore formation upon photocuring does not affect the adhesion strength of the BC_PDz systems. Cohesive fracture upon failure is observed upon visual inspection for mucoadhesion time test performed with porcine tissue with 1 cps viscosity solution (see FIG. 5D).

**Example 2Q: Discussion of Results for Oral Cavity Applications** **- FTIR Spectra of Photocured BC PDz Film Display Reactive Intermediates and Reduction of Nucleophiles -OH and -NH$_2$**

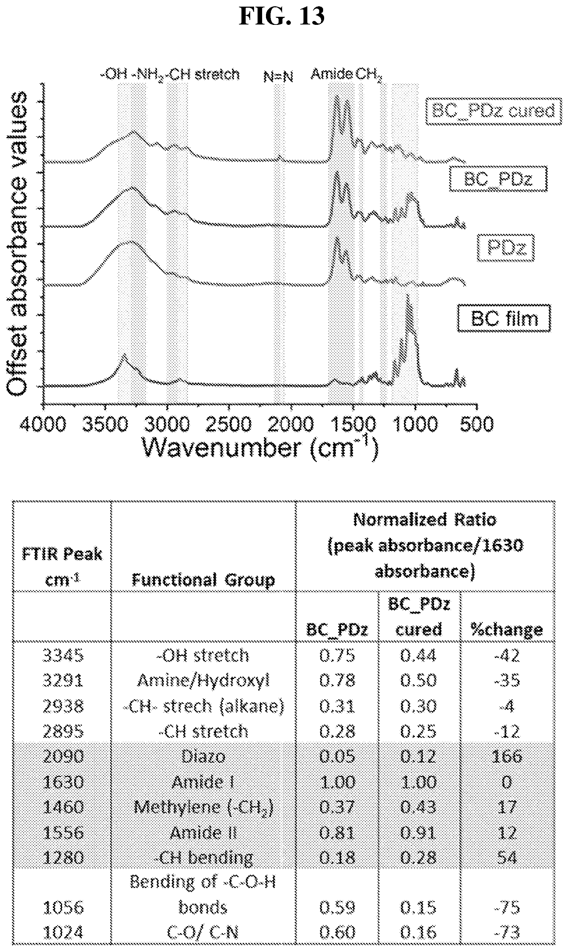

FTIR analysis of BC films with PDz before and after curing aids in the qualitative analysis of changes in the functional groups. Reactions with the -OH and - CH- groups present in the BC structure are monitored before and after UVA exposure. The FTIR spectra for BC, PDz, uncured BC_PDz film, and photocured BC_PDz film are displayed in FIG. 13 (top image) with peak assignments listed in FIG. 13 bottom table. Absorbance values are utilized to qualitatively compare functional groups intensity before and after photocuring. The amide carbonyl (1630 cm$^{-1}$) present on G5-PAMAM is utilized as a non-reactive control for peak normalization. Diazoalkane formation at 2090 cm$^{-1}$ is an intermediate which further leads to formation of carbenes. A reduction in the absorbance of -CH$_2$- peak (2938 cm$^{-1}$), -CH stretch at (2895 cm$^{-1}$), -OH peak (3345 cm$^{-1}$), and -C-O-H bonds at (1056 cm$^{-1}$) supports the working hypothesis of carbene insertion activated by photocuring. An increase in the absorbance of methylene groups (-CH$_2$-) peak (1460 cm$^{-1}$) and -CH bending peak (1280 cm$^{-1}$) is attributed to -CH- insertion in cellulose. The in situ generated carbene upon UV irradiation would thus crosslink with the mucosal tissue proteins as well as the cellulose matrix, thereby causing adhesion.

**Example 2R: Discussion of Results for Oral Cavity Applications** **-** *Ex vivo* **Mucoadhesion Displays 2-7 Days Fixation in Constant Shear, Wet Environments**

In order to challenge the BC_PDz films, the photocured adhesives are exposed to constant shear aqueous environments for 2 days on porcine tissues. To further stress the films, hypotonic 50 mM phosphate buffer pH 6.8 is employed. The dried BC_PD films are applied with PDz on the porcine tissue fixed on the beaker wall and activated using UVA (365 nm, 100 mW.cm$^{-2}$, 180 s) directly from the BC film side placed on the tissue. Later, the beaker is filled with fluids of varying viscosities, 1, 10, and 30 cps, to simulate the range of human saliva viscosity. The process is similar to clinicians' procedure of applying medication to oral tissue, which is later hydrated due to salivary release. BC_PD films are chosen in this experiment owing to their low swelling ability, thus, to study the matrix stability of thin films upon prolonged aqueous exposure. The beaker in FIG. 5A depicts the experimental setup wherein constant shear stress of 0.072 Pa (1 cps), 1.08 Pa (1 cps), and 0.54 Pa (30 cps) is applied through the magnetic stirrer. Neat BC films adhered to the tissue for 2 hours before failure. Composites adhered for 48 hours (FIG. 5B) after which the study is stopped, and the film is manually peeled off to observe the fracture. The fracture is cohesive, wherein PDz is present on both the film and the tissue surfaces (FIG. 5D). It is found that BC films with PDz adhere up to 48 hours, maximum study time; however, no difference is observed for varying viscosity. Thus, one longer trial is then performed with rabbit tongue in 1 cps viscosity solution for 7 days. It is found that the films remain adhered to the tissue until day 7, as observed in FIG. 5C. Moreover, the films maintained their structural integrity despite being exposed to aqueous environment for 7 days in the absence of antibiotics which allows growth of bacteria and possibly biofilm formation.

**Example 2S: Overall Discussion of Results for Oral Cavity Applications**

Overcoming weak adhesion strength (0.5-5 kPa) and short retention durations (6-8 hours) in wet oral environment is a current unmet clinical need in the treatment of oral wounds. Frequent applications lead to reduced patient compliance. Thus, systems that offer adhesion strength sufficient for prolonged retention durations and maintain structural integrity in the wet environment may improve therapeutic outcomes. Towards addressing this limitation, bacterial cellulose (BC) films were paired with carbene-based bioadhesives with the aim of long-term retention on hydrated soft mucosal tissue surfaces. To the best of our knowledge, this is the first demonstration of BC with covalent bond formation as the mechanism of adhesion. The system consists of two independent components to yield a synergistic composite: (1) BC film (freeze-dried, FD, and press-dried, PD) and (2) PDz (G5-PAMAM grafted with diazirine). BC films undergoing different drying processes exhibit different mechanical, morphological, and light transmission properties (FIG. 9 and FIG. 6). The BC_PDz systems exhibit adhesion strength ranging from 7-17 kPa in both dry and rehydrated states, 2-5 times higher than conventional mucoadhesives. The adhesion strength is also similar to biopolymer film/gel-based surgical tissue adhesives like gelatin/glutaraldehyde films (24.5 kPa), gelatin/alginate adhesives (5-15 kPa), collagen/citric acid derivative (~19 kPa), gelatin/polysaccharides (dextran, chitosan, starch) (12-22 kPa), gelatin/poly(ethylene glycol) diacrylate (12 kPa), dopamine modified PEG/Laponite (8 kPa), and catechol-functionalized poly(ester urea) (9-10 kPa, similar to fibrin glue). Ex vivo mucoadhesion time study performed by evaluating the adhesion in aqueous environment revealed that BC films remain adhered for 2-7 days to wet tissues with the aid of PDz bioadhesive, contrary to 6-8 hours. BC_PDz composites have sufficient strength for retention at the site, yet are easily removed without the danger of tissue damage (unlike for cyanoacrylates adhesives) (Cohesive failure as observed in FIG. 5D).

BC serves as the structural component within the composite, providing barrier and mechanical properties of the film. In contrast to plant cellulose, BC biosynthesized by aerobic bacterium *Gluconacetobacter* family members is pure and yields a form that prevents overhydration. Cellulose obtained from plant sources contains impurities, requires processing to produce nanofibrillated cellulose, has low surface area, and poor light transmission. Hydrophilic cellulose derivatives utilized as mucoadhesives undergo swelling in contact with water leading to dissolution/disintegration of the matrix. BC extends the opportunity to utilize pure cellulose in the form of nanofibrous thin films that exhibit selfregulated swelling in aqueous environment while not being dissolved/eroded off. The intrinsic nanofiber matrix provides a high surface area for interactions with drug/active molecules and light transmission properties. Two methods of film post-processing are demonstrated. Freeze-drying helps to maintain the 3D structure of BC film but is likely to be an energy-intensive manufacturing practice. On the other hand, press-drying allows in-line manufacturing through industry standard calendaring operations with the tradeoff of compacted 3D network and dense aggregation of nanofibers as visible in FIG. 9 (top right image). Press-drying yields thinner structures with high tensile stress at break and elastic modulus (FIG. 6) but at the cost of matter aggregation.

Hydrated BC films exhibit lower tensile strength, increased elongation, and higher modulus of elasticity (0.04-1 GPa, similar to reported traditional non-BC films) compared to the dry state as observed in FIG. 6. This suggests that moisture leads to film elasticity and reduced brittleness due to decreased intermolecular forces and hydrogen bonds among cellulose molecules (elongation% of 7-17% for rehydrated films compared to 2-4% for dry films). The nanofibrous matrix of BC also allows for higher storage modulus (20-60 kPa) and viscoelastic nature in the hydrated state as presented in FIG. 14 compared to other cellulose derivative systems (G' 0.01-30 kPa). The storage modulus and elasticity increase significantly upon PDz application, with and without photocuring. These material properties of BC in hydrated form supports it as a potential candidate for oral cavity applications.

Drying process also affects rehydration ability (59% for BC_FD, 4% for BC_PD) and transparency of the films (FIG. 9 bottom left plot). Thus, BC films can be chosen based on hydration and transparency requirements of the application. The transparency of BC is a requirement for the light activation of PDz bioadhesive, thereby tuning the adhesion strengths of BC films. Significantly higher adhesion strength for applied UV dose of 18 J (BC_FD, BC_PD) compared to 3 J and 6 J implies that increased UV dose leads to increased crosslinking. The results correlate with reports of UV dose dependence on adhesion strength for PDz composites.

PDz bioadhesives are activated using light/voltage to bond wet tissue surfaces (adhesion strength 6-27 kPa) by means of covalent bonds. Surface conforming PDz gel composites have been evaluated for adhesion strength with various macromolecules, including hydrophilic PEG400 (5-15 kPa), hydrophobic polycaprolactone alcohols (5-25 kPa), and alginate (8-10 kPa). PDz has also been evaluated on film substrates like PLGA (4.5 kPa) and polymer brush coated glass (15-40 kPa). Herein, the present example demonstrated similar adhesion strength ranging from 7-17 kPa exhibited by BC_PDz film systems that combine the advantages of gels (conforming, water absorption) and films (strength). The thin, flexible BC matrix allows conformal contact with the tissue surface, thereby overcoming limitations offered by stiffer, hydrophobic substrates like PLGA. Moreover, the BC matrix does not disintegrate in aqueous environments (FIG. 5C), providing an enhanced interfacial interaction with tissues.

The presence of PDz on BC surface leads to pore formation at the interface upon photocuring, as observed in FIG. 12A to 12I. The pores are formed due to nitrogen evolution upon the formation of carbene. Rehydrated films exhibit an increase in the pore length with reduced pore frequency and uniform crosslinking over the area (FIG. 12I). This can be attributed to dilution of PDz crosslinking due to reaction with water molecules, thus reducing foaming, increased pore length. It can be inferred that lower UV dose (BC_FD) leads to denser matrices whereas higher UV dose (BC_PD) exhibits higher porosity. Cohesive fracture occurs within the PDz adhesive matrix and does not propagate to the BC or tissue as BC comes off completely (FIG. 5D). This is further confirmed by SEM wherein fracture through the pore surface exhibited by both films in dry and rehydrated state is similar to that of neat PDz. No fibrils are observed in the fractured surfaces as opposed to uncured composites in FIG. 12A and 12D. The observed porous surface is similar to reported viscous PDz adhesive composites. This brittle fracture occurring within PDz matrix highlights that the stresses are not distributed across the BC matrix, suggesting poor integration of the viscous PDz into nanofibrous BC matrix. The removal of adhesive left on the tissues is expected to follow natural epithelial sloughing, where complete renewal of the oral epithelium surface occurs over 5 days. It is important to note that the epithelial tissues would not be harmed upon removal due to cohesive failure.

Hydrophilic water swelling macromolecules based on natural, synthetic polymers and their blends have been widely employed in mucoadhesive systems. Maximum adhesion strengths of mucoadhesive films have been reported to be 0.65 kPa for polyacrylates, 0.2-0.55 kPa (assuming 2 $cm^2$ film area) for chitosan and hydroxyethylcellulose (HEC) blend, 1-2.7 kPa for Eudragit®, carboxymethylcellulose (CMC) and hydroxymethylcellulose (HMC) blends, 3 kPa (assuming 2 $cm^2$ film area) for electrospun chitosan membranes with pectin coating and 0.5-2 kPa (assuming 2 $cm^2$ film area) for various natural gum (alginate, xanthan gum, CMC, HEC) based films evaluated using normal/tensile test. Injectable mucoadhesive based on chitosan grafted dihydrocaffeic acid and oxidized pullulan exhibited average adhesion strength of 5 kPa and polyvinylpyrrolidone (PVP), CMC blend films exhibited 20 kPa as evaluated by lap shear test. Electrospun nanofibrous meshes composed of poly(l-lactic acid) and mucoadhesive poly(ethylene oxide) exhibited adhesion strength ranging from 1.5-3 kPa (assuming 2 $cm^2$ film area). The lap shear tests exhibit about 2-5 times higher adhesive strength than normal/tensile test. Taking that into consideration, the average adhesion strength for these mucoadhesives relying on hydrogen bonds ranges from 0.5-5 kPa.

Desired properties of mucoadhesives in aqueous oral cavity environment include mucoadhesion time (>24 hours) and matrix stability. These properties are required to maintain extended contact with the diseased site. However, current systems exhibit mucoadhesion residence less than 24 hours. Carbopol 934, ethylcellulose blend (12 hours), blend of HPMC, Eudragits, Carbopol 940 (1.5-2 hours), chitosan/gelatin blend (6 hours), sulfhydryl anchored adhesive alginate (1.5 hours) and xanthan gum blends (1-5 hours) exhibit average mucoadhesion time of 1.5-6 hours. Other mucoadhesives like electrospun nanofiber membranes (polycaprolactone, dextran, polyethylene oxide) and catechol-chitosan based mucoadhesive hydrogels exhibited *in vitro* and *ex vivo* mucoadhesion time of 4-6 hours. BC_PDz patch exhibits *ex vivo* mucoadhesion time of 2-7 days under shear stress, but this does not take into account the dynamic environments within the oral cavity, e.g., tissue strains, ionic effects, friction. This relatively low adhesion strength (0.5-5 kPa) and adhesion time (<24 h) of existing mucoadhesives are limited in effectiveness due to weak adhesion forces (hydrogen bonds, van der Waals bonds), loss of structural integrity, or combination thereof. The present BC_PDz system addresses these limitations in two ways; first, the swelling of the composites plateau due to self-regulating water absorption property of BC, thus maintaining structural integrity in wet environments (FIG. 5C). Second, the higher mucoadhesion time and enhanced adhesion strength (FIG. 10A and 10B, FIG. 4B and 4C, and FIG. 11) exhibited by BC_PDz can be attributed to several mechanisms such as the formation of covalent bonds by carbene insertion (major adhesion mechanism), electrostatic interactions (cationic amines and anionic mucus layer), and mechanical interlocking of BC nanofibers with the mucosal proteins. Upon UV irradiation, generated carbene forms covalent bonds with the mucosal tissue proteins i.e., mucin glycoprotein, through amino acid insertion.

Several advantages of the present mucoadhesive are reiterated. The BC_PDz mucoadhesive system provides light tunable adhesion with higher adhesion strength (7-17 kPa) and extended *ex vivo* mucoadhesion time (2-7 days) compared to existing systems. Distinct from other types of reported films, hydration of BC films retain the adhesivity, i.e., the films exhibit comparable adhesion strength in both wet and dry states. The BC_PDz system exhibits cohesive failure upon removal thereby the assurance of no tissue damage upon adhesive patch removal. However, the system exhibits certain limitations. In the present example, PDz was applied each time to BC film before application, wherein clinical application need a shelf-stable dry composite. Cohesive failure within the PDz matrix highlights the stresses are not distributed across the BC matrix, suggesting poor integration of the globular PDz and nanofibrous BC.

Composites of bacterial cellulose and carbene-based bioadhesives are herein demonstrated to adhere directly to tissue substrates in the oral cavity. Bacterial cellulose films serve as the structural component capable of loading active pharmaceutical ingredients, and a surface layer of carbene-based bioadhesives provides light activated, on-demand adhesion. Tunable adhesion strength ranging from 7-17 kPa against porcine tissue with cohesive failure is obtained (compared to 0.5-5kPa of cellulose derivative-based systems). The composite adhesion strength correlates to the UV dose applied and the hydration state. The composite patch withstood 7 days in a submerged aqueous environment under constant shear stress when applied to ex vivo tissue substrates. The present composite demonstrates a new platform towards mucoadhesive delivery systems for the management of oral cavity diseases and lesions.

## Example 2T: Drug Release Profile of Adhesive Patches (BC PDz Composite)

The present example demonstrates that adhesive patches loaded with protein drug exhibits tunable drug release behaviours (24 hrs). The adhesive patches in this instance are BC_PDz composite. The adhesive patches were loaded with bovine serum albumin (BSA), a model protein drug (66.5 kDa) with the drug loading efficiency as mentioned in FIG.

38A. The release profile of BSA has been studied with the adhesive patch in detail (FIG. 38B). PCL coatings with varying coating thickness has been evaluated to modulate the release profile.

### Example 3A: Aqueous Adhesive Composites of Bacterial Cellulose and PAMAM-g-Diazirine as a Hydrogel for Oral Cavity Applications (General Summary)

Topical approaches to oral wounds and infections exhibit weak adhesion to wet surfaces, short retention duration (6-8 hours), frequent dosing requirement and patient incompatibility. To address these, film based adhesive platforms comprising of bacterial Cellulose (BC) and photoactive carbene-based bioadhesive (PDz) exhibiting adhesion strength of 7-17 kPa against wet tissues was demonstrated in examples 2A to 2S. However, films may be less suitable (albeit still applicable) for application to deep irregular shaped wounds, gap filling, and diseases widespread in the oral cavity. Thus to further extend the applicability of BC_PDz systems, photoactive aqueous adhesive hydrogels are formulated. Structure-activity relationships evaluate BC mole, bioadhesive moles, and total solute percentage on viscoelastic properties and wet tissue adhesion. The aqueous composites crosslink upon photocuring in 15-70 seconds and exhibit transition from viscous to elastic state gradually. The photoactive composites have shear moduli ranging from 1-10 kPa, thereby mimicking oral mucosa. The composites exhibit on-demand tunable adhesion strength of 7-22 kPa in wet environments, similar to previously reported BC film systems. The work highlights the potential of BC/PDz hydrogel systems for treatment of diseases in the wet environment of oral cavity.

In more details, examples 2A to 2S demonstrated the combination of Bacterial cellulose (BC) films with PAMAM-g-diazirine (PDz) adhesives exhibiting tunable adhesion strength of 7-17 kPa against wet tissues. The idea was to utilize stronger covalent bonds formed by carbenes with mucosal proteins to adhere BC films to oral mucosa in place of weak hydrogen and van der Waals bonds. The epithelial sloughing process responsible for epithelium renewal every 5-6 days can lead to release of the bonded system. PDz bioadhesive chemically constitutes of Poly(amido amine) (PAMAM) dendrimer grafted with 4-[3-(trifluoromethyl)-3H-diazirin-3-yl] benzyl bromide(diazirine) and has been evaluated for adhesion to wet tissue surfaces. Diazirine groups present in the PDz generate reactive crosslinking groups (carbenes) upon photocuring or electorcuring as demonstrated earlier. The photocured PDz composites have been demosntrated to exhibit tunable shear moduli of 1-250 kPa and maximum adhesion strength of 5-45 kPa against wet tissue substrates. However, the film-based BC systems are less suitable (albeit still applicable) for applications in cases such as widespread disease sites over oral cavity area, irregular shaped wounds/lesions, gap filling, etc. Thus, to further widen the applicability of BC based adhesive systems, an aqueous composite hydrogel of BC and PDz which can be applied/smeared to the irregular shaped diseased site was developed. The applied adhesive would turn into a viscoelastic solid upon photocuring thereby offering tunable shape property. BC is a naturally derived hygroscopic polymer from *Gluconacetobacter* species, which exhibits controlled hydration with retention of material properties. This inherent self-regulating water absorption ability of BC can be advantageous for moist environments like oral cavity. High storage modulus of nanofibrous BC hydrogel is proposed to be utilized to improve the material properties of BC_PDz composites upon photoactivation. Carbenes formed upon photocuring PDz has been demonstrated to react easily to -OH groups and form solid matrix. Similar reaction is expected in case of exposure of carbenes to -OH abundant BC hydrogel resulting in formation of viscoelastic solid. Moreover, the incorporation of BC may further lead to reduced concentration of cationic amine groups thereby increasing cytocompatibility. BC is sold commercially as a food product, *nata de coco,* attesting to low biotoxicity risks and ease of large scale industrial production of BC. Wound dressings based on BC for burns and other topical wounds have also been approved by FDA owing to its hydration properties.

Based on the abovementioned properties of PDz and BC, it has been demonstrated that composites of BC and PDz may exhibit higher shear moduli than BC or PDz alone. It is also demonstrated that these composites can further exhibit synergistic wet tissue adhesion (lap shear adhesion). Structure-activity relationships of BC_PDz aqueous composite is evaluated for dynamic viscosity, photocuring rheology, morphological analysis, crosslinking mechanism, and lap shear adhesion strength. The effect of total solute percentage, BC moles, and PDz moles in the composites upon mechanical properties and adhesion strength exhibited by BC_PDz composites is studied.

### Example 3B: Materials and Method for Example 3A

Materials: Polyamidoamine (PAMAM) dendrimer, Generation 5, Mw=28.8 kDa is bought from Dendritech, Inc, USA. 3-[4-(bromomethyl) phenyl]-3-(trifluoromethyl)-diazirine (bromo-diazirine) is purchased from TCI, Japan. All other chemicals were purchased from Sigma Aldrich, unless specified otherwise.

Strains and culture medium: *G.hansenii* 53582 ATCC is employed for the fermentation of BC in *Hestrin Schramm* (HS) media. HS is media composed of glucose (20 g L$^{-1}$, 0.11 M), citric acid monohydrate (1.15 g L$^{-1}$, 0.005 M) and Na$_2$HPO$_4$ (2.7 g L$^{-1}$, 0.02 M) enriched with yeast extract (5 g L$^{-1}$) and peptone (5 g L$^{-1}$) with final pH 4.5. Media is prepared and autoclaved at 120 °C for 15 mins.

Microbial fermentation: Bacterial strain *Gluconacetobacter hansenii* ATCC 53582 is inoculated in the culture media (HS Media) for 24 hours in shaking conditions at 26°C to produce the inoculum. 5% (v/v) of the *G.hansenii* inoculum is inoculated in HS basic medium and cultured in 24-well plates at 26 °C for 5 days. The BC pellicles are harvested and washed with distilled water followed by sodium hydroxide treatment (0.25 N) for 30 minutes at 60 °C for removal of bacterial cells (confirmed with protein determination as described in example 2C). The pellicles are then washed thoroughly with deionized water. BC produced and harvested after 5 days is further employed for formulating defibrillated dispersion.

Synthesis of PAMAM-g-diazirine: Grafting of diazirine to PAMAM backbone is performed as described in the above examples. PAMAM-g-diazirine with 20% conjugation is employed for further experiments.

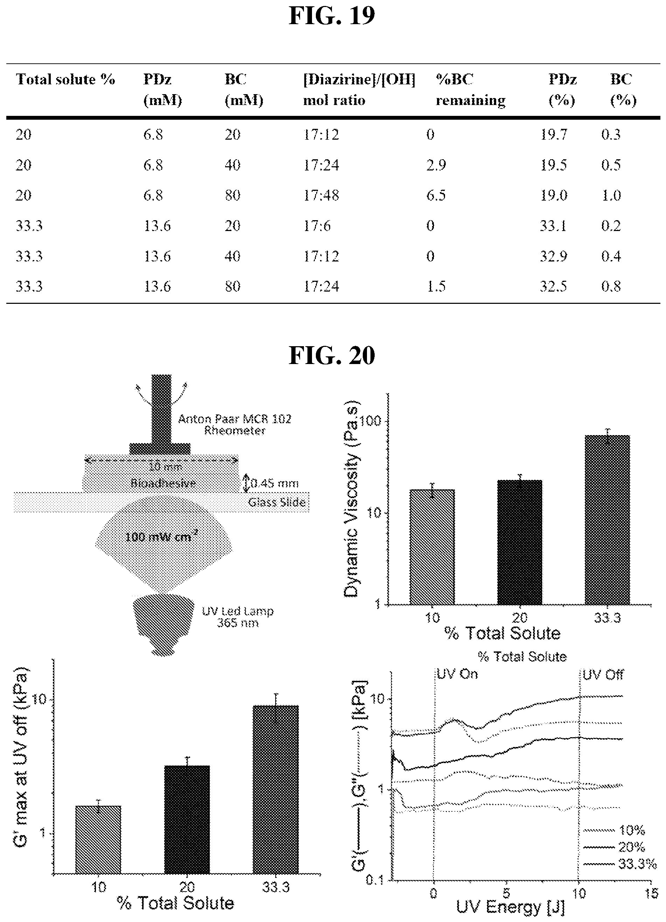

Formulation of BC_PDz hydrogels: BC_PDz composites are formulated as depicted in FIG. 17. Washed BC films are cut into pieces and suspended in deionized water. The obtained dispersion is sonicated using for 3 hours at 37% amplitude with 10 seconds on, 10 seconds off cycle (Vibra-Cell™, VCX 500, SONICS®). BC dispersion is then centrifuged (11180 g force, Thermo Scientific™ Sorvall™ Legend™ XTR Centrifuge), and excess water is removed to obtain BC hydrogel with a concentration of $1.3 \pm 0.2\%$. Different compositions of BC_PDz are prepared by mixing varying weights/moles of BC hydrogel and PDz, as mentioned in FIG. 18 and FIG. 19 and magnetically stirred to ensure uniform mixing. The obtained BC_PDz formulations are then employed for further experiments.

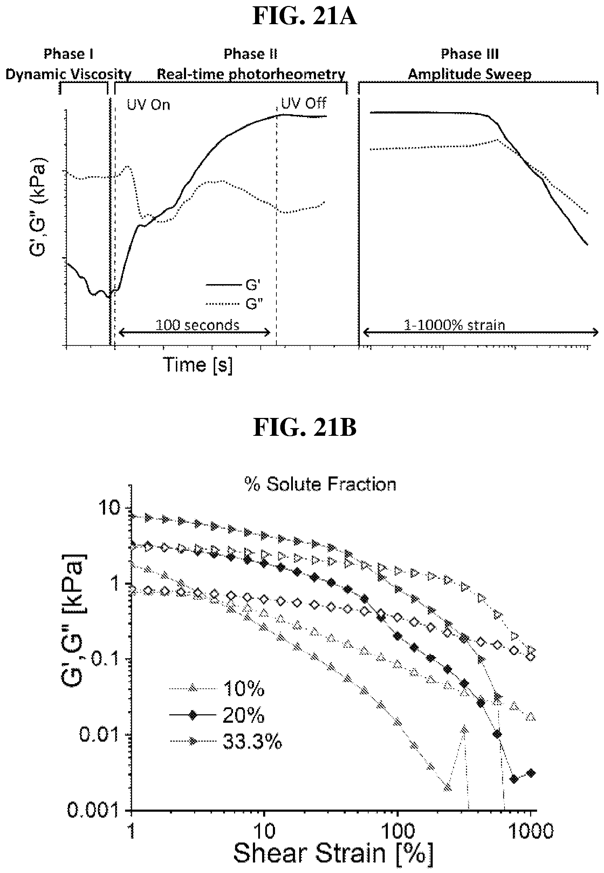

Real-time photorheometry evaluation of BC_PDz composites: Rheometry measurements are conducted with Anton Paar Physica MCR 102 rheometer fitted with parallel plate probe PP10 and UVA transparent glass slide, as presented in FIG. 20 (top left image). The applied UVA intensity (365 nm) is calibrated to $100 mWcW^{-2}$ with an IL 1400 Radiometer using the 365nm LED setup (SOLIS-365C, THORLABS) with current input of 1300 mA. The parameters of dynamic oscillatory strain are set as follows: 0.45 mm measuring gap, the amplitude of 1%, and a frequency of 10 Hz. The storage modulus (G') and loss modulus (G") are evaluated in the first 30 s to determine dynamic viscosity (phase I), followed by photocuring using UVA irradiation for 100 s (phase II), and an amplitude sweep of 1-1000% at 1 Hz (phase III) as presented in FIG. 21A.

SEM imaging: BC_PDz composites are placed between PET films and photocured with UVA dose of $10 J cm^{-2}$ (365 nm, $100 mWcm^{-2}$). After photocuring, the PET sheets are peeled apart to expose the cohesively fractured composite matrix to analyze the structural morphology. A field emission scanning electron microscope (JEOL, JSM-6700 F, Japan) (accelerating voltage of 5kV and 8 mm working distance) is employed to observe the structural morphology of the cured BC_PDz composites. The samples are sputter coated (JEOL JFC-1600 Auto Fine Coater) with platinum for 70 s, 20 mA current in vacuum (8-9 Pa) before imaging. Image J software is employed to determine the pore length and pore number per unit area. The scale bar is utilized as a measurement reference, and the longest length on the pore is recorded.

Lap shear adhesion assessment against PLGA and wet tissue mimicks: PLGA films ($2x1.5 cm^2$) and wet tissue mimic (hydrated collagen films, $2x1.5 cm^2$) are fixed on the glass slides using double sided adhesive tape. BC_PDz composites (10 mg, 100 $\mu$m thickness) are then smeared in between the films and activated by UVA (365 nm, 100 mW $cm^{-2}$) for 100 seconds (10J UV dose). The adhesion strength of the formulated BC_PDz composites is determined using modified tensile tester (Chatillon force measurement, Largo, FL, USA) with a controlled linear speed of 3 mm/min with 50N load cell (n = 5). PLGA films are cast using 30% (w/v) PLGA (50:50) solution in DCM (stirred overnight to aid dissolution). The viscous solution is then cast onto flat glass substrate using a knife applicator at 50 mm/sec at room temperature in fume hood. The cast PLGA films are dried at room temperature overnight in the fumehood to prevent foaming. Films are then transferred to vaccum oven and kept for one week for solvent evaporation at 37°C. Thickness of PLGA film was found to be $0.06 \pm 0.0039$ mm.

Statistical analysis: All the data are presented as mean $\pm$ SD (n=3, unless otherwise stated). OriginPro 2018 64-bit software is employed to determine significance employing Two-way ANOVA with Bonferroni as posthoc test. A $p < 0.05$ (*) is considered statistically significant. Rheology and lap shear adhesion curves are smoothed to remove transient noise by using percentile filter and Loess (span 0.1) method, respectively.

## Example 3C: Discussion of Results for Example 3A - Formulation of BC PDz hydrogels, Investigation of Structure-Activity Relationship with Photorheology

Aqueous adhesive composites are produced by mixing varying moles of Bacterial Cellulose (BC) and PAMAM-g-diazirine (PDz) as per the schematic in FIG. 17 and the formula mentioned in FIG. 18 and 19. These composites are evaluated for real-time mechanical properties through a customized photorheology setup, as illustrated in FIG. 20 top left image. Three-phase rheology protocol described in FIG. 21A is employed to demonstrate that BC_PDz composite has higher shear moduli than BC or PDz alone. The structure-activity relationships of the viscoelastic nature of composites with increasing total solute percentage, PDz moles, and BC moles are determined. SEM analysis is performed to evaluate the morphology of photocured adhesive composites. Lap shear adhesion test is performed to demonstrate that photocuring BC_PDz composite provides higher adhesion strength as compared to PDz alone. Rheological properties (dynamic viscosity, loss modulus (G")/storage modulus (G'), and yield stress) are related to lap shear adhesion strength to measure cohesive properties as a function of total solute percentage, BC moles and PDz moles.

**Example 3D: Discussion of Results for Example 3A - Adhesive Composites are Cured in 10 J or Less Energy and Exhibit BC, PDz Moles Dependent Tunable Shear Moduli of 1-10 kPa**

The total solute percentage of aqueous adhesives is varied from 10% to 33.3%. The percentage of PDz which is the major component is varied from 9-32.5%. In the BC film based platform of examples 2A to 2S, the concentration of PDz bioadhesive was 50%. However, reduced tissue exposure of cationic -$NH_2$ groups in PDz may help avoid mammalian cell cytotoxicity. To address this issue, lower concentrations of PDz (9-32.5%) are evaluated in the present compositions. Real-time photorheology is performed to examine the degree of crosslinking of BC_PDz composites under UVA exposure. The corresponding changes in the storage modulus (G') and loss (G") moduli are recorded in real-time using a customized photorheometry setup described in FIG. 20A top left image. During phase I, 1% strain is applied at 10 Hz frequency to disrupt any hydrogen bonds or other forces, and dynamic viscosity of the composites is determined. It is observed in FIG. 20 top right plot that increasing the total solute percentage in the composites leads to an increase in the viscosity from 18 to 70 Pa.s. A positive correlation (Pearson's r = 0.94) between total solute percentage and dynamic viscosity is observed. After that, UVA with intensity 100 mW cm$^{-2}$ is applied for 100 seconds, and the changes in the values of G' and G" are recorded, as displayed in phase II in FIG. 21A. Maximum G' value measured at 10 J UVA dose varying from 1.6 kPa to 8.9 kPa is positively correlated (Pearson's r = 0.97) to total solute percentage as presented in FIG. 20 bottom left plot. The representative photocuring profiles of BC_PDz composites with varying total solute percentages (10, 20, and 33.3%) are observed in FIG. 20 bottom right plot. It can be observed that composites with 10 and 20% solute percentage behave as viscoelastic solid before curing as G' values are higher than that of G" values. However, as the curing progresses, the G' values increase and G" values lower down, thereby increasing the gap after 30-40 seconds of UV application (UV dose 3-4 J). On the contrary, composite with 33.3% total solute behaves as a viscoelastic liquid in Phase I, which later changes to viscoelastic solid as curing progresses, observed by the crossover of G' and G" after 15 seconds of UV exposure (UV dose 1.5 J). G' and G" values for the composites before photocuring exhibit an increase with the increase in the total solute percentage, as can be seen in FIG. 20 bottom right plot. It is observed that all the composites with varying total solute percentage require less than 10 J of UV dose translating to less than 100 seconds to crosslink.

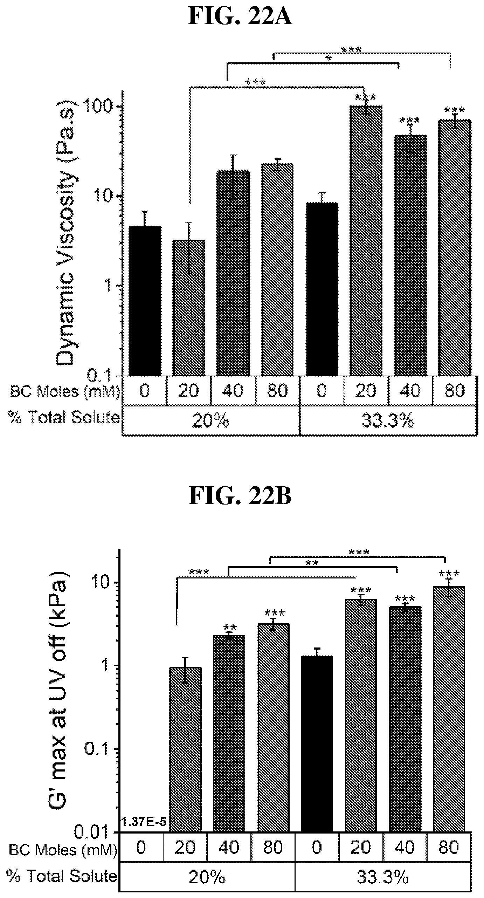

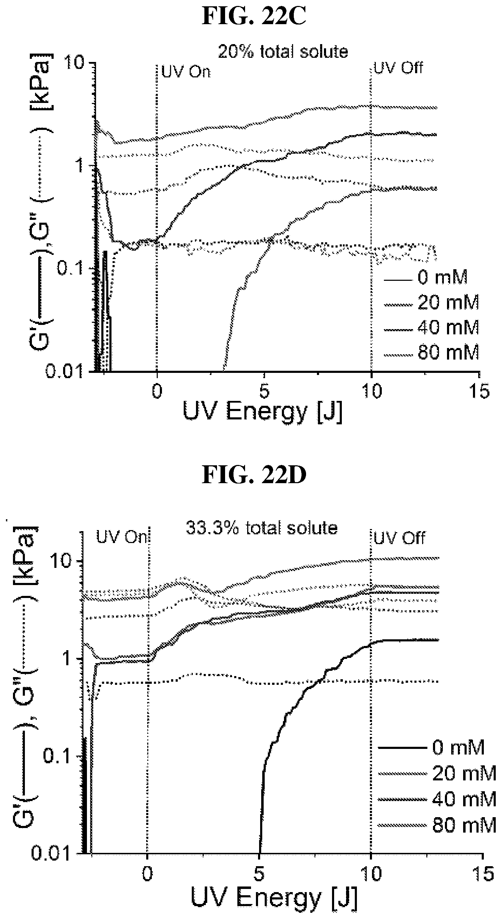

Thereafter, the variation in the photocuring behavior of composites with the variation of BC moles for 20 and 33.3% total solute is determined. BC moles are varied from 0-80 millimoles. FIG. 22A represents the dynamic viscosity of the formulations in phase I of photorheology. It can be observed that the dynamic viscosity ranges from 4-22 Pa.s for 20% total solute composites and 45-100 Pa.s for 33.3% total solute. An increase in total solute due to the increase in PDz moles lead to a significant rise in viscosity, as can be observed for all the composites with varying BC moles. The increase in BC moles is positively correlated (Pearson's r = 0.89) with the increase in dynamic viscosity for composites with 20% total solute. Contrary to this, no such correlation is observed for composites with 33.3% total solute. Maximum G' value measured at 10 J UVA dose is presented in FIG. 22B for the BC_PDz composites consisting of varying BC moles and total solute percent fixed at 20 and 33.3%. It is observed that the increase in BC moles leads to an increase in G' for both the groups (0.6-3.7 kPa for 20%, 1-11 kPa for 33%). Moreover, an increase in the total solute percentage leads to a significant increase in maximum G' for all the corresponding BC moles. Increase in G' maximum is positively correlated to increase in BC moles for both 20% (Pearson's r = 0.97) and 33.3% (Pearson's r = 0.89) total solute composites. The representative photocuring profiles of BC_PDz composites with varying BC moles (20, 40 and 80 mM) are presented in FIG. 22C and 22D for 20 and 33.3% total solute respectively. It can be observed from FIG. 22C that composites with 20 and 40 mM of BC behave as viscoelastic liquid before curing as G' values are lower than that of G" values. However, as the curing progresses, the G' values increase and G', G" crossover occurs after the UV application of 5-6 J. On the contrary, composite with 80 mM behave as viscoelastic solid before curing, and as the curing progresses, the gap between G' and G" increases. It is observed that all the composites with varying total solute percentage require less than 10 J of UV dose to crosslink. Moreover, it is observed that composite with 0 mM BC does not crosslink even after 10 J of UV dose.

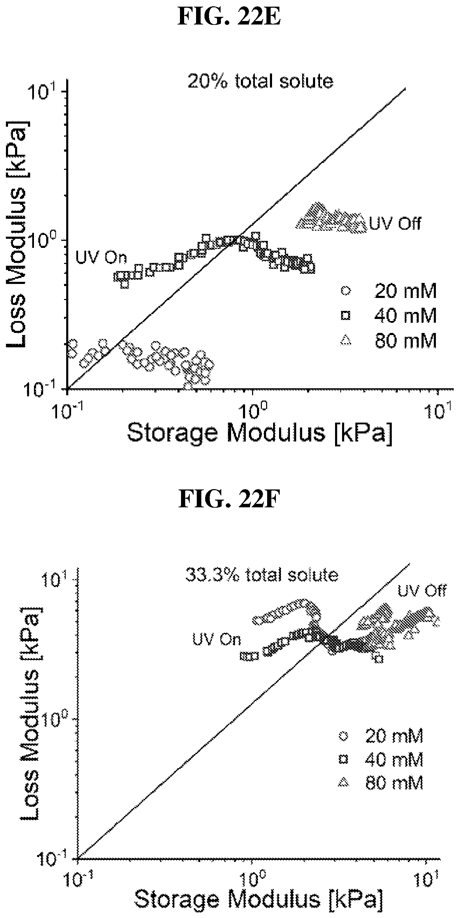

In case of 33.3% total solute photorheograms presented in FIG. 22D, all the composites start off as viscoelastic liquid in Phase I, which then change to viscoelastic solid upon crosslinking initiated due to UVA exposure after application of 2.5 to 7 J for different formulations. From both FIG. 22C and 22D, it can be observed that the UV joules required to crosslink the composite is reduced as the BC moles increase. The minimum UV dose needed to crosslink is 1.5 J for 80 mM BC moles composite with 33.3% total solute. The photocuring kinetics of BC_PDz composites from FIg. 22C and 22D are replotted as a vector plot to evaluate the changes in the viscoelastic properties of the composites during curing in FIG. 22E and 22F. A diagonal line marks the gelation point, defined as G" divided by G' or tan δ= 1. The line also divides the plot into two regions, the liquid region (above the line) and solid-like regions (below the line). It is observed that the BC_PDz composite progress from the liquid region and as the curing continues transition into the solid-like material properties with an elastic modulus near kPa values for 20- and 40mM BC in both the groups. Contrary to this, composites with 80 mM BC in both plots already lie in the solid-like region before curing and exhibit a rise in the elastic modulus upon photocuring.

Thus, the BC_PDz hydrogels exhibit storage modulus of 1-10 kPa with crosslinking time varying from 15-70 seconds and light dose of 1.5-7 J. The crosslinking time, light dose and light intensity required for photcuring of the composites fall within the general curing protocols employed in dentistry, which varies from 5-100 seconds with light intensity ranging from 300-2000 mW cm$^{-2}$, dose of 8-50 J/cm$^2$ for lights with 350-500 nm wavelengths.

**Example 3E: Discussion of Results for Example 3A - Increase in BC Content and Total Solute Percentage have Opposite Effects on Yield Stress and Strain Values for the Break**

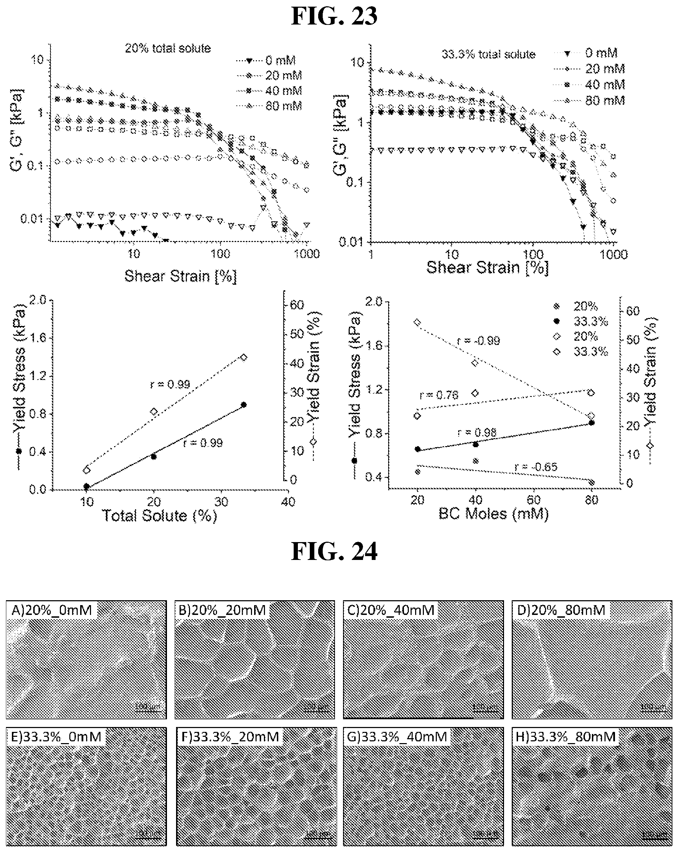

The third phase of photorheology consists of amplitude sweeps ranging from 1-1000% strain performed after photocuring as depicted in FIG. 21A. The amplitude sweep helps to determine the linear viscoelastic range (LVR), yield stress, and yield strain, as presented in FIG. 23. All examined BC_PDz composites exhibit an LVR up to 56% of strain, as observed in FIG. 23 (top left and top right plots). The composites with 80 mM BC in case of both 20 and 33.3% total solute exhibit plastic yield characterized by a slow decrease of G' and G", whereas composites with 20 and 40 mM exhibit elastic failure which is characterized by the sharp decline in G'. Yield stress and yield strain both exhibit a strong correlation with total solute percentage for BC_PDz composites, as presented in FIG. 23 bottom left plot. However, for 20% total solute formulations, it is found that increasing BC moles exhibit a negative correlation with yield stress and yield strain, as presented in FIG. 23 bottom right plot. Contrary to this, composites with 33.3% total solute exhibit a positive correlation of increase in BC moles with yield stress and yield strain, as is presented in FIG. 23 bottom right plot. Thus, the results suggest that the material properties (yield stress, yield strain, storage modulus) of the composites can be tuned based on the requirements by varying the concentrations of BC and PDz.

**Example 3F: Discussion of Results for Example 3A - Tunable Adhesion Ranging from 10-55 kPa against PLGA Substrate and 7-35 kPa Against the Wet Tissue Mimic is Obtained upon Photoactivation**

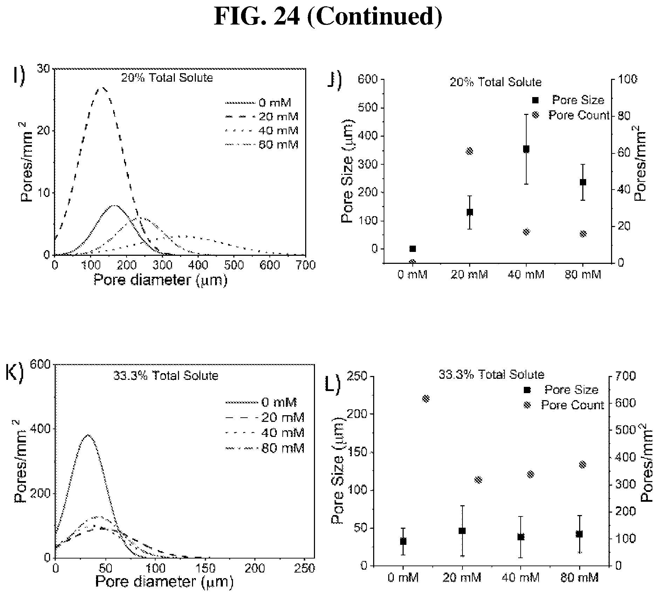

FIG. 24A and 24E represent the SEM image of photocured BC_PDz composites with 0mM of BC and 20% and 33.3% of PDz respectively. No pores are observed in the case of photocured 20% PDz, which indicates that formulation is not cured, which can be due to low concentration of PDz. This further supports the G' max at UV off presented in FIG. 22B, which is negligible as compared to other groups. However, uniform pore distribution over the area is observed in FIG. 24E for 33.3% PDz, suggesting that it is cured and forms a porous matrix. FIG. 24B to 24D represent the SEM images of the photocured cohesively fractured BC_PDz composites with 20% total solute fraction and increasing moles of BC. It can be observed that these composites exhibit pores with pore size ranging from 100-300 $\mu$m, as depicted in FIG. 24I. Pore count for 20% total solute composites exhibit a negative correlation (Pearson's r = -0.77) with increasing BC moles as observed in FIG. 24J. FIG. 24F to 24H represent the SEM images of the photocured cohesively fractured BC_PDz composites with 33.3% total solute fraction and increasing moles of BC. The composites exhibit pores with pore size ranging from 10-80 $\mu$m, as depicted in FIG. 24K. The pore size for all three composite ranges from 10-80 $\mu$m and does not exhibit any correlation with increasing BC moles. A positive correlation (Pearson's r = 0.99) of pore count with increasing BC moles is observed from FIG. 24L. All the composites exhibit lower pore count when compared to that 33.3% PDz with 0 mM BC. The adhesion failure occurs cohesively among the PDz bioadhesive matrix rather than the PET sheets. While comparing between 20% and 33.3% total solute composites, it is observed that there is a decrease in the pore size and increase in the pore count as the total solute increases from 20% to 33.3%. This can be attributed to the increase in the amount of PDz available to react which results in the formation of pores with higher pore count, observed from the FIG. 24A and 24E representing 20% and 33.3% PDz.

**Example 3G: Discussion of Results for Example 3A - Tunable Adhesion Ranging from 10-55 kPa against PLGA Substrate and 7-35 kPa against the Wet Tissue Mimic is Obtained upon Photoactivation**

The setup of lap shear adhesion test of photocured BC_PDz composite performed against PLGA and wet tissue mimic hydrated collagen is illustrated in FIG. 25 top left image. The composites placed in the sandwich structure are exposed to UVA (365 nm, 100 mW cm$^{-2}$, 10 J UV dose).

The representative stress-strain profile obtained by the lap shear test of BC_PDz composites placed between PLGA films is presented in FIG. 25 (top right and bottom left plots) for 20% and 33.3% total solute, respectively. FIG. 25 bottom right plot compares the maximum adhesion strength at failure for 20 and 33.3% total solute BC_PDz composites varying with the BC moles. It is found that BC_PDz composites with 20% total solute exhibit mean adhesion strength ranging from 8-40 kPa for varying BC moles. All three groups exhibit significantly higher adhesion strength as compared to the control wherein 0 mM of BC is added, which is 20% PDz. The 20% solute composites exhibit a negative correlation of adhesion strength with increase in the BC moles (Pearson's r = -0.87). On the other hand, composites with 33.3% total solute are found to exhibit mean adhesion strength ranging from 22-70 kPa. While comparing between 20 and 33.3% formulations, it is observed that 33.3% total solute composites with 40 and 80 mM exhibit higher adhesion strength as compared to corresponding 20% total solute composites (***, p <0.001 and * p <0.05 respectively).

FIG. 26 top left and top right plots depict the representative stress-strain curves for 20%, and 33.3% BC_PDz composites performed against wet tissue mimic. FIG. 26 bottom left plot compares the maximum adhesion strength at failure for 20 and 33.3% total solute BC_PDz composites varying with the BC moles. It is found that BC_PDz composites with 20% total solute exhibit mean adhesion strength ranging from 3-33kPa. All groups exhibit adhesion strength higher than that of the 20% PDz with 0 mM of BC. BC_PDz composite with 40 mM BC exhibit significantly higher adhesion strength compared to the control. Moreover, composites with 33.3% total solute are found to exhibit mean adhesion strength ranging from 8- 30kPa. All the composites exhibit higher adhesion strength than the control, 33.3% PDz with 0 mM of BC. However, composites with 20 and 80 mM of BC exhibit significantly higher adhesion strength. While comparing between 20 and 33.3% formulations, it is observed that 33.3% total solute composites with 20 and 80 mM exhibit higher adhesion strength as compared to corresponding 20% total solute composites (*, $p < 0.05$ and * $p < 0.05$ respectively) with similar strain values. However, no correlation of adhesion strength with BC moles is observed. The high standard deviation can be attributed to factors like variations in the hydration extent of the wet tissue mimic.

Overall, the composites exhibit adhesion strength ranging from 10-55 kPa against PLGA substrates and 7-35 kPa against wet tissue mimic. The adhesion strength is lower in the case of wet tissue mimics which is expected due to reaction with water molecules. FIG. 26 bottom right plot represents the comparison of maximum adhesion strength of BC_PDz hydrogel and film based adhesive systems with varying UV dose, BC moles and total solute percentage. It is observed that the aqueous hydrogel systems exhibit similar adhesion strength to that of the previously reported adhesive BC_PDz film systems (not significantly different $p > 0.5$). Moreover, the adhesion strength can be tuned by modulating the concentrations of BC and PDz in the composites.

## Example 3H: Overall Discussion of Results for Example 3A

From the examples above, it can be seen that the present aqueous BC_PDz adhesive hydrogels display higher storage modulus and adhesion strength compared to non-aqeuous PDz composites with higher PDz concentration.

PAMAM-g-diazirine was demonstrated as a liquid adhesive hydrogel for application in minimally invasive surgery. The shear moduli of the formulations and the adhesion strength were found to be varying with the percentage of diazirine conjugated to the PAMAM dendrimer, the concentration of the PDz in aqueous media and UV dose. For the PDz formulation with 20% conjugation, the lowest concentration evaluated was 25% which upon photocuring was found to exhibit storage modulus ranging from 1-3 kPa (measuring gap 0.1 mm, 6J UV dose). The adhesion strength for the same formulation against wet tissue was found to be around 5 kPa. Formulations with 20% diazirine conjugation and higher concentrations (50 and 75%) exhibited higher shear moduli ranging from 20-200 kPa and adhesion strength ranging from 25-35 kPa. However, there are several limitations associated with the presence of high concentrations of PDz. One of the limitations is higher foaming caused due to the nitrogen release that occurs due to higher PDz concentraton in the formulation, thereby causing a trade-off between adhesion strength and porous nature. Another concern may be the biocompatibility of the bioadhesive systems, due to the presence of cationic - $NH_2$ groups. Several attempts have been made to overcome these limitations by replacement of the aqueous solvent of PDz bioadhesive by hydrophilic PEG400 in one case and hydrophobic polycaprolactone alcohols in the other. The lowest concentration of PDz present in non-aqueous PEG/PDz composites was found to be 30% with an approximate storage modulus of 25 kPa after photocuring for applied UV doses of 10, 20 and 60 J cm$^{-2}$ (0.1 mm measuring gap). The examples also suggested that a negative correlation exists between effective storage modulus after photocuring and the initial measuring gap. The adhesion strength of 30% PDz composites against tissue was found to be ranging from 5-10 kPa. Similarly, the PDz/PCLT composites with concentration of PDz as low as 20 and 33.3% were formulated and found to exhibit G' maximum of approximately 0.1 kPa and 4 kPa respectively (measuring gap 0.1 mm, 20 J UV dose). The adhesion strength of these composites against wet tissue mimic was found to be ranging from 5-15 kPa for 20% concentration and 5-25 kPa for 33.3% concentration. For the dry substrate, adhesion strength for 20% concentration ranged from 1-15 kPa, and for 33.3%, it ranged from 1-10 kPa. Another composite of PDz where alginate was incorporated exhibited storage modulus of 30 kPa (measuring gap 0.1 mm, 20 J UV dose) and adhesion strength of 8-10 kPa against wet substrates.

In examples 3A to 3G, the formulation and evaluation of the aqueous composites of BC and PDz for its mechanical properties and adhesion to wet as well as dry substrates are demonstrated. Composites with low PDz concentrations, i.e. 20 and 33.3% with varying BC moles have been evaluated. The composite comprises (1) BC hydrogel, which improves the mechanical properties (2) and PDz, which is PAMAM grafted with diazirine responsible for adhesion to wet surfaces by carbene insertion mechanism. The composites exhibited an increase in the dynamic viscosity depending on the PDz as well as BC moles. Moreover, the composites exhibit shear moduli ranging from 1-10 kPa in positive correlation with BC moles and percentage of total solute (measuring gap 0.45 mm). The composites thereby mimic the shear moduli of oral mucosal tissues. Taking into consideration discussed studies about negative correlation of measuring gap with shear moduli, the BC_PDz aqueous composites exhibit comparable shear moduli to non-aqueous PDz/PCLT and PDz/PEG composites even when measured at four times higher measuring gap. Moreover, the aqueous BC_PDz composites with about 20% and 33.3% PDz exhibit adhesion strength of 8-40 kPa and 22-70 kPa respectively, which is comparatively 2-3 times higher than that exhibited by PDz/PCLT composites with dry substrates. Similarly, composites with about 20% and 33.3% PDz exhibited adhesion strength of 3-33 kPa and 8-30 kPa respectively with hydrated tissue mimic, which is again 2-3 times higher than PDz/PEG, PDz/PCLT and PDz/alginate composites.

Inclusion of nanofibrous BC into the composite allows for crosslinking at lower PDz concentrations and exhibit higher storage modulus mimicking oral cavity tissues.

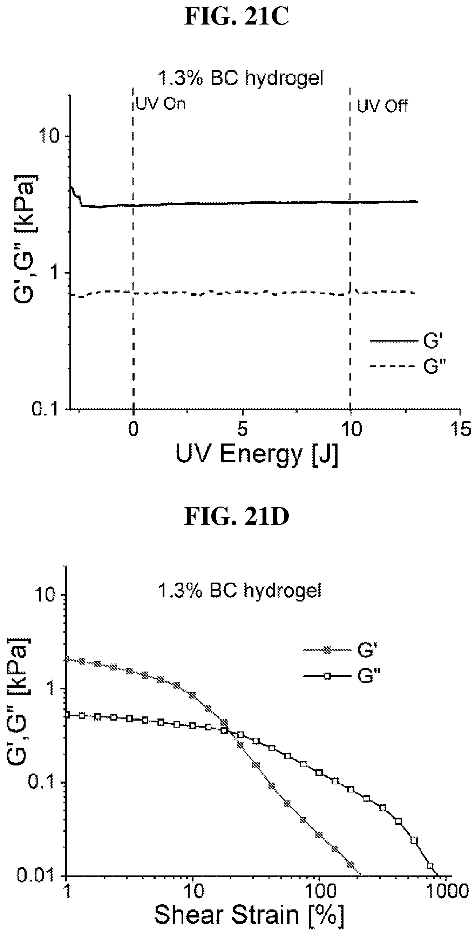

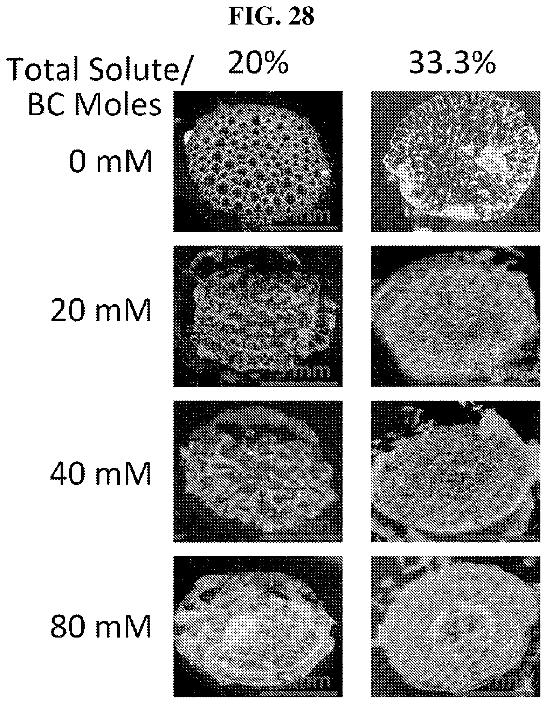

Bacterial Cellulose is one of the varieties of cellulose, most commonly synthesized by aerobic bacteria *Gluconacetobacter* species. BC includes a nanofibrous matrix with complex molecular structure wherein -OH groups of cellulose form hydrogen bond with inter and intra-molecular glucan chains. The nanofibrous matrix of BC dispersion is presented in FIG. 27. Moreover, hydrogen bonds are also formed between the -OH groups of cellulose and bound water. The presence of hydrogen bonds in the wet BC matrix provides it with a rigid structure on molecular level and mechanical flexibility. This property of BC is utilized to exploit the adhesive properties of BC_PDz composites occurring due to the interaction of BC and PDz upon UV irradiation. Moreover, the free -NH$_2$ groups of PAMAM are expected to form hydrogen bonds with -OH groups of cellulose in the BC_PDz composite hydrogel, thus providing stability to the hydrogel formulation. Inclusion of BC into low concentration PDz (20%) allows for crosslinking of the 20% PDz composite, which is not possible otherwise. It can be observed from FIG. 22B that 20% PDz with 0 mM BC exhibit G' maximum as low as zero. Contrary to which, similar composites with 20, 40, and 80 mM of BC crosslink and exhibit shear moduli ranging from 1-10 kPa in positive correlation with BC moles. This phenomenon is clearly visible in FIG. 28 wherein the images of composites after photocuring are presented. Composite with 20% total solute and 0 mM BC remains liquid after photocuring with bubbles whereas composites with BC form a solid sturdy matrix. Thus, it is observed that BC allows for crosslinking of lower percentages of PDz even though it does not have any crosslinking properties of its own upon UV irradiation (FIG. 21C).

BC has been found to exhibit linear viscoelastic region only up to 6-7% of strain as observed from FIG. 21D. A negative correlation of yield stress and yield strain with increasing BC moles for 20% total solute composites is observed. This behavior can be attributed to the increasing amount of unreacted BC as moles of BC increase for 20% total solute leading to lower yield stress and strain. On the contrary, positive correlation for the BC moles and yield stress, the strain is observed for 33.3% total solute composite, which can be attributed to decreasing percentage of unreacted BC moles (FIG. 19). A similar relation is observed as the total solute percentage increases (FIG. 18).

SEM micrographs exhibit trade-off between pore formation and adhesion strength upon photocuring dependent on PDz concentration.

SEM images presented in FIG. 24A to 24H depict a trade-off in adhesion strength and pore formation. The pores formed herein are due to the nitrogen molecules that are released upon the formation of carbenes similar to studied photocured and electrocured PDz bioadhesives. Uniform distribution of pores all over the matrix implies uniform presence of PDz and BC in the hydrogel. Higher overall pore count in case of 33.3% composites can be attributed to increased PDz moles, thus allowing for more diazirine molecules available to react. As there is increase in PDz molecules to react with available BC molecules in case of 33.3%, higher number of pores are observed. FIG. 28 also exhibits a similar trend wherein greater foaming is observed in case of higher solute percentage. Relatively higher pore size in case of 20% total solute composites can be attributed to the competitive reaction of PDz molecules with water owing to low solute concentration, leading to lower pore count and higher average pore size. Higher concentration of PDz leads to higher adhesion strength alongwith higher pore count. At the point of failure, the fracture occurs at the adhesive matrix, thereby not damaging the tissue.

BC_PDz adhesive composites display lower crosslinking duration and higher adhesion strength onto wet tissue surface mimic compared to other mucoadhesives.

The BC_PDz composites evaluated herein are demonstrated for adhesion to the oral cavity for applications like localized drug delivery, adhering drug loaded films to disease site and as protective coating onto diseased/wound surfaces. Currently employed mucoadhesives exploit hydrogen bond interactions formed by polymers as the adhesion mechanism, resulting in low adhesion strength (8-50 Pa). Mucoadhesive films based on chitosan, hydroxypropylmethylcellulose (HPMC), and xanthan gum loaded with lidocaine hydrochloride have been evaluated for periodontal systems and found to exhibit low adhesion strength of 8-16 Pa (assuming area of 3 cm$^2$). A reported emulsion-based mucoadhesive system for oral candidiasis was found to exhibit average adhesion strength of 33 Pa (assuming area of 3 cm$^2$). Another report evaluated a different mucoadhesive system composed of Eudragits (a mixture of methyl and ethyl acrylates), and HPMC loaded with Fluconazole with 50 Pa maximum adhesion strength (assuming area of 3 cm$^2$) for application in oral candidiasis. All these existing mucoadhesive polymer-based systems exhibit lower adhesion strength compared to that of BC_PDz composites, which is around 15-22 kPa. Higher strength displayed by BC_PDz can be attributed to the covalent bond mechanism by carbene insertion depicted in FIG. 17. An increase in ester group absorbance is observed which can be due to the reaction of carbene with -OH groups forming ester and increase in -C-C-bonds due to reaction of carbene with -CH$_2$ groups as hypothesized in FIG. 17 (also see FIG. 29 and 30). Another possible mechanism could be the electrostatic interactions of positively charged -NH$_2$ groups of PAMAM in PDz with negatively charged acid moieties present in the mucus layer of the oral cavity. Moreover, the theory of adhesion due to mechanical interlocking wherein nanofibers of BC entangle with the mucosal proteins could be one of the possible mechanisms.

Apart from drug-loaded mucoadhesive system, other mussel inspired bioadhesive systems have been evaluated. It was reported wet adhesion through tannic acid-based bioadhesives exhibited adhesion strength ranging from 30-50 kPa. Though these bioadhesive exhibit high adhesion strength, the shear moduli exhibited by the crosslinked adhesives is as low as 10 Pa, which indicates the weak mechanical properties of the system. The gelation requires the addition of silver nitrate solution, and the minimum gelation time required is around 240 seconds, thus making the application of bioadhesive a complicated process. Another citrate-based mussel inspired bioadhesive was reported, wherein adhesive strength ranging from 30-160 kPa is reported upon crosslinking. However, crosslinking requires the addition of silver nitrate and sodium periodate, which are oxidative along with the requirement of high gelation time as 60-500 seconds. In another report, prepolymer liquid-based system which transformed into in situ bioadhesive amphiphilic systems was studied. The gels were formed when appropriate amount of poly(2-dimethylaminoethyl)methacrylate (PDMA) was added to prepolymer mixture of PEG and PCL. The gels exhibited storage modulus ranging from 50-200 kPa and adhesion strength of 40-90 kPa. However, the minimum gelation time required for the formation of gels was as high as 8 minutes. None of the reported studies provided any evidence of cohesive failure upon removal despite of the high adhesion strength, thus exposing the tissues to damage upon removal, if required. The present BC_PDz composite hydrogel exhibits on demand curing in 15-60 seconds, cohesive failure when removed, and requires no additional chemicals.

Advantages of aqueous BC_PDz mucoadhesive hydrogels are further discussed below.

BC_PDz hydrogel adhesive composites of the present disclosure have several advantages as compared to previously reported systems, thus proving its suitability for application as mucoadhesive system. Some of the advantages of the BC_PDz composites are (i) on-demand adhesion with crosslinking time as low as 15-60 seconds, (ii) covalent bonding mechanism for adhesion, (iii) storage modulus ranging from 1-10kPa (measuring gap 0.45 mm) mimicking oral tissue, (iv) wet tissue adhesion strength of 15-22 kPa, (iv) cohesive failure in the composite matrix, (v) no harsh chemicals/oxidants required for crosslinking, and (vi) reduced concentration of PDz in the composite thus improving the biocompatibility. The present composites also exhibit adhesion strength similar to BC_PDz film (7-16 kPa) systems evaluated in examples 2A to 2S.

In conclusion, the aqueous adhesive BC_PDz hydrogel composites are formulated with the ability to adhere directly to tissue substrates in the oral cavity upon photocuring. BC hydrogel serves to improve the mechanical properties of the system employing nanofiber networks, which further form adhesive crosslinked matrix upon reaction with PDz on light exposure. Tunable adhesion strength ranging from 3-33 kPa against wet tissue mimic and 8-70 kPa against dry PLGA substrate is obtained. The adhesion strength is variable across the type of BC_PDz composite based on BC moles, PDz moles, and total solute percentage in the composite. The composites exhibited a change from viscous (liquid-like) state to elastic (solid-like) material properties as crosslinking progresses upon photocuring. The primary mechanism of adhesion is -OH insertion of carbenes generated upon photocuring followed by the formation of covalent bonds. Cohesive failure exhibited by the mucoadhesive patch prevents the damage of soft tissues in the oral mucosa upon removal. The aqueous BC_PDz composites forming covalent adhesion present a new tool towards mucoadhesive delivery systems targeting oral cavity diseases and lesions.

**Example 4A: Present Mucoadhesive in the Form of Integrated Adhesive Patch Exhibit Adhesion Properties Superior to Marketed Formulations**

Examples 4A to 4C are generally intended to demonstrate the present mucoadhesive configured in the form of an integrated adhesive patch instead of a film (examples 2A to 2S) or a hydrogel (examples 3A to 3H).

BC_PDz mucoadhesive patches are developed by coating BC films (freeze-dried (FD), press-dried (PD)) with varying moles (0.06-0.11 $\mu$moles) of PDz bioadhesive (PDz integrated to about 40-50 $\mu$m thickness of BC). This provides an integrated system wherein the patch can directly be applied to the application site and photocured, unlike the 2-component system in the form of a film. The adhesive patch can also be stored in dry form, thus prolonging the shelf stability. The adhesive patches are evaluated for lap shear adhesion test and compared for their performance with marketed denture and medicated adhesives. It is observed that the patches exhibit significantly higher adhesion strength than control BC films in dry (D) and rehydrated states (Rh) (FIG. 31 top left plot). The adhesion strength against porcine skin ranges from 7-22 kPa, which is not significantly different from other BC_PDz composites (2 component system and hydrogels) as presented in FIG. 31 top right plot. The adhesion performance was compared with marketed denture and medicated adhesives (composed of mucoadhesive polymers). BC_PDz patches exhibited significantly higher adhesion strength against all marketed adhesives studied and similar to the denture adhesive composed of polyvinyl alcohol (PVA) and carboxymethyl cellulose (CMC) (FIG. 31 bottom left plot). The patches were further evaluated for adhesion strength against mucosal tissues (pig cheek tissue, pig tongue) as presented in FIG. 31 bottom right plot. The patches exhibit significantly higher adhesion strength compared to their respective control BC films against both tissues. However, freeze-dried and press-dried films vary in their performance. Patches made of BC_FD exhibit significantly higher adhesion strength than BC_PD in case of porcine tongue, however opposite is observed in the case of pig cheek. Overall, the adhesion strength against mucosal tissues ranges from 10-40 kPa. The patches are also evaluated for peel strength which is the force/width required while peeling the patch away from the tissues. The patches exhibit peel strength ranging from 3-6 N/m (FIG. 32A). The patches exhibit peel strength higher than skin plaster (Band-aid) and significantly lower than denture adhesive composed of PVA-CMC, thus assuring no tissue damage upon peeling. FIG. 32C and 32D represent the ex vivo mucoadhesion study in aqueous environment performed for patches made of BC_PD and BC_FD respectively. The patches stay adhered for 7 days in the aqueous environment while maintaining the structural integrity of the BC films. In addition to these evaluations, BC_PDz patch is also evaluated for its ability of electrosurgical activation. BC with Caproglue is also evaluated. Collagen sheets are evaluated to owing to its widespread application in biomaterial composites (see example 4B below).

**Example 4B: Present Mucoadhesive in the Form of Integrated Adhesive Patch Exhibit Adhesion Properties Superior to Marketed Formulations - Light Free Method of Activation of BC PDz Adhesives by Commonly Used Clinical Surgical Approach (Electrosurgical Activation)**

Electrosurgery is used routinely in surgery to cut, coagulate, dissect, fulgurate, ablate and shrink tissue. High frequency (100 kilohertz to 5 megahertz), alternating electric current at various voltages (200-10,000 Volts) is passed through tissue to generate heat. In our recent investigation we evaluated BC_PDz adhesive patch and BC with other adhesives for electrosurgical activation. Other biomaterials like collagen were also evaluated. It is observed that collagen is malformed under electrosurgery and therefore cannot be used for electrosurgical activation (wherein collagen substrate was placed on the adhesive followed by electrosurgical activation). This behaviour can be attributed to protein denaturing that occurs in protein-based biomaterials and biomaterials with low heat tolerance. The activation of caproglue_BC combination through electrosurgery was also investigated. It is observed that BC does not get deformed upon electrosurgery and activates the adhesive (liquid caproglue is converted to solid, observed upon scratching with tweezer) while preventing any damage to the tissue underneath. Similarly, the activation of BC_PDz adhesive patch using electrosurgery was investigated. FIG. 33A and 33C represent the cured samples (top) before and after (bottom) lap shear test. FIG. 33B show that all BC_PDz patches are activated through electrosurgery thus exhibiting adhesion strength of 20-40 kPa against the tissues. BC_Caproglue samples are also activated and exhibit 10-40 kPa adhesion (FIG. 33C). In a study carried out, it was observed that a thin layer of adhesive is present on skin even after failure for BC_Caproglue and BC_PDz respectively. The skin underneath stays protected in both cases. This ability of BC to allow adhesive activation through electrosurgery can be attributed to the unique nanofibrous matrix that prevents deformation and allows adhesive activation through heat.

**Example 4C: Present Mucoadhesive in the Form of Integrated Adhesive Patch Exhibit Adhesion Properties Superior to Marketed Formulations - The Adhesive Patches Exhibit Tunable Drug Release Behaviours (24-48 hrs)**

The adhesive patches have been loaded with various drugs with the drug loading efficiency as mentioned in FIG. 34. The release profile of Valganciclovir has been studied with the adhesive patch has been studied in detail (FIG. 35). PCL and PLGA coatings with varying coating thickness has been evaluated to modulate the release profile.

Structure Property Relationships of BC delivery vehicles have been studied with Valganciclovir.

BC films give tunable drug elution rates of 0.5 to 50 ug per hour per $cm^2$.

BC films provide tunable drug release for 10-48 hours in sustained manner.

The burst release profile of BC films can be controlled to achieve 5-100% release in first 30 minutes.

Drug loaded films have also been evaluated for adhesion properties. FIG. 36A and 36B represent the maximum adhesion strength at failure for drug loaded patches. The patches exhibit adhesion strength varying from 10-40 kPa variable with the type of BC film used, coating polymer and coating thickness. FIG. 36C and 36D present the ex vivo mucoadhesion test for drug loaded patches. The patches stay adhered for 24 hours, as observed from the images. This suggests that drug loading and coating leads to variation in the adhesion strength, however no loss in adhesion is observed.

**Example 5: Summary of Various Configurations of the Present Mucoadhsive and Commercial and Potential Applications**

The preceding examples demonstrate that the present mucoadheisve can be configured to have at least 3 forms such as a film configuration, a hydrogel configuration and an integrative patch. The comparison of these forms are shown in FIG. 37.

The present mucoadhesive includes bacterial cellulose (BC), which is a natural product derived from *Gluconacetobacter* species. The extracellular polymer absorbs 80-100 times water of its own dry weight with the inherent ability to self-regulate water absorption. The cellulose matrix reaches a hydration plateau, after which no more water is absorbed, and the cohesive properties are retained, even in excess moisture. Another attribute of the material properties is the amorphous structure that results in translucent to transparent properties in both the dry and wetted states, which can be exploited for optical tissue sensors, photochemistry, photocuring, optical characterization, or combination thereof. BC and other similar natural polymers are edible food product, for example translucent *Nata de coco,* and is *generally regarded as*

*safe* (GRAS) by FDA. BC-based wound dressings have also been approved by FDA and marketed for burn wounds owing to its wound healing potential. In addition, the present mucoadhesive and method of manufacturing the composite mucoadhesive may include the following combination of characteristics/features that allows for extended fixation on wet substrates.

1. Composition of matter that includes composites formulations with one self-regulating hydrophilic polymer, one stimuli-sensitive bioadhesives capable of bonding substrates, including but not limited to wet substrates, and up to 50% w/w incipients, active pharmaceutical ingredients, microorganisms, minerals, particles, biologic, or combination thereof.
2. Self-regulating water absorption that maintains cohesive material properties, prevent brittle fracture of the mucoadhesive by physiological stress within a 2-7 day programmed period.
3. Transparent or translucent optical properties of light wavelengths from 350 to 1100 nm.
4. Film forming patches that withstand physiological stresses on epithelial and mucosa surfaces for 2-7 days.

The present mucoadhesive can be configured for other medications or other purposes, e.g. nicotine oral patch, tissue engineering scaffold for chronic oral ulcers. The present self-regulating mucoadhesive platform has numerous industrial applications, including but not limited to, controlled chemical delivery, substrate sealants, fixation of medical implants, film-forming barriers (microorganisms, chemical, physiological fluid), air/liquid interface exchange, tissue reconstruction, or combination thereof. For example, as a buccal wound dressing, it has the following advantages:

1) Provide wound dressing in wet oral environment through minimally invasive surgeries or robot-assisted surgeries.
2) Display non-inferiority to current buccal wound treatment options.
3) Display tissue fixation 10-100x times longer the oral gel or cream formulations.
4) Reduced dosing frequency and increase patient compliance.

The site of applications of the present mucoadhesive can include and are not limited to the gastrointestinal tract and mucosal tissues (e.g. eye, nasal cavity, reproductive systems). The present mucoadhesive can be used (i) to deliver active ingredients locally to the oral cavity for oral ulcers, lesions, and diseases, (ii) as a barrier patch for diseased tissues and wound protection, (iii) as an acid barrier in gastrointestinal acid reflux, (iv) prevent air/liquid exchange in pneumothorax, (v) for tissue reconstruction in transplanted or resected tissues, e.g. colon cancer, oesophagus transplant, and/or (vi) transmucosal drug delivery system, for example, antiinflammatory medications for chemotherapy patients experiencing inflamed mouth sores.

## Claims

1. A mucoadhesive comprising:

    a bacterial cellulose; and
    a bioadhesive incorporated to the bacterial cellulose,
    wherein the bioadhesive comprises a polymer or a dendrimer, wherein the polymer and the dendrimer are grafted with a moiety which forms covalent interactions with a tissue surface in the presence of a stimulus, and wherein the moiety comprises a diazirine.

2. The mucoadhesive of claim 1, wherein the polymer or the dendrimer comprises polycaprolactone or a poly(amidoamine) dendrimer, respectively.

3. The mucoadhesive of claim 1 or 2, wherein:

    the bacterial cellulose is a film comprising a network of cellulose nanofibers; and/or
    the bacterial cellulose is dried or rehydrated; and/or
    the bioadhesive is deposited on the bacterial cellulose.

4. The mucoadhesive of claim 1 or 2, wherein:

    the bacterial cellulose is a hydrogel; or
    the bacterial cellulose is a hydrogel and the bioadhesive is present in an amount of 10 wt% to 35 wt% of the mucoadhesive.

5. The mucoadhesive of claim 1 or 2, wherein:

> the bioadhesive is included in the bacterial cellulose; or
> the bioadhesive is included in the bacterial cellulose and the bioadhesive is present in an amount of 15 wt% to 60 wt% of the mucoadhesive.

6. The mucoadhesive of any one of claims 1 to 5, wherein:

> the bacterial cellulose is of a species derived from *Acetobacteraceae* family; and/or
> the bacterial cellulose is optically pervious to light having a wavelength ranging from 280 nm to 1100 nm.

7. The mucoadhesive of any one of claims 1 to 6, wherein the stimulus comprises:

> light having a wavelength ranging from 280 nm to 500 nm; and/or
> a voltage ranging from -10 kV to 10 kV.

8. The mucoadhesive of any one of claims 1 to 7, further comprising:

> a pharmaceutical drug incorporated to the mucoadhesive; and/or
> a polymeric coating on the mucoadhesive.

9. A method of forming the mucoadhesive of any one of claims 1 to 8, the method comprising:

> providing a bacterial cellulose;
> providing a bioadhesive comprising a polymer or a dendrimer, wherein the polymer and the dendrimer are grafted with a moiety which forms covalent interactions with a tissue surface in the presence of a stimulus, wherein the moiety comprises a diazirine; and
> incorporating the bioadhesive to the bacterial cellulose.

10. The method of claim 9, wherein providing the bacterial cellulose comprises:

> inoculating bacteria in one or more culture media;
> extracting raw bacterial cellulose from the one or more culture media; and/or
> treating the raw bacterial cellulose with an alkali to remove the bacteria from the bacterial cellulose.

11. The method of claim 9 or 10, wherein:

> providing the bacterial cellulose comprises freeze-drying or press-drying the bacterial cellulose; or
> providing the bacterial cellulose comprises freeze-drying or press-drying the bacterial cellulose and further comprising rehydrating the bacterial cellulose which is freeze-dried or press-dried prior to incorporating the bioadhesive.

12. The method of any one of claims 9 to 11, wherein providing the bioadhesive comprises:

> grafting the diazirine on a polycaprolactone or a poly(amidoamine) dendrimer; and
> mixing the polycaprolactone or the poly(amidoamine) dendrimer grafted with the diazirine in a buffer.

13. The method of any one of claims 9 to 12, wherein incorporating the bioadhesive to the bacterial cellulose comprises depositing the bioadhesive on the bacterial cellulose.

14. The method of claim 9, wherein incorporating the bioadhesive to the bacterial cellulose comprises:

> (i) contacting the bacterial cellulose with an organic solvent comprising the bioadhesive; and
> removing the organic solvent to have the bioadhesive included in the bacterial cellulose;
> or
> (ii) mixing the bacterial cellulose and the bioadhesive to form a hydrogel.

15. The method of any one of claims 9 to 14, further comprising coating a polymeric coating on the mucoadhesive.

**Patentansprüche**

1. Mukoadhäsivum, aufweisend:

   eine bakterielle Cellulose und
   ein in die bakterielle Cellulose eingebrachtes Bioadhäsivum,
   wobei das Bioadhäsivum ein Polymer oder ein Dendrimer aufweist, wobei das Polymer und das Dendrimer mit einem Teil gepfropft sind, der in Gegenwart eines Reizes kovalente Wechselwirkungen mit einer Gewebefläche eingeht, und wobei der Teil ein Diazirin aufweist.

2. Mukoadhäsivum gemäß Anspruch 1, wobei das Polymer oder das Dendrimer jeweils ein Polycaprolacton- oder ein Poly(amidoamin)-Dendrimer aufweist.

3. Mukoadhäsivum gemäß Anspruch 1 oder 2, wobei:

   die bakterielle Cellulose ein Film ist, der ein Netzwerk aus Cellulose-Nanofasern aufweist, und/oder
   die bakterielle Cellulose getrocknet oder rehydriert ist, und/oder
   das Bioadhäsivum auf die bakterielle Cellulose aufgebracht ist.

4. Mukoadhäsivum gemäß Anspruch 1 oder 2, wobei:

   die bakterielle Cellulose ein Hydrogel ist, oder
   die bakterielle Cellulose ein Hydrogel ist und das Bioadhäsivum in einer Menge von 10 Gew.-% bis 35 Gew.-% des Mukoadhäsivums vorliegt.

5. Mukoadhäsivum gemäß Anspruch 1 oder 2, wobei:

   das Bioadhäsivum in der bakteriellen Cellulose enthalten ist, oder
   das Bioadhäsivum in der bakteriellen Cellulose enthalten ist und das Bioadhäsivum in einer Menge von 15 Gew.-% bis 60 Gew.-% des Mukoadhäsivums vorliegt.

6. Mukoadhäsivum gemäß irgendeinem der Ansprüche 1 bis 5, wobei:

   die bakterielle Cellulose von einer Art ist, die aus der Familie der *Acetobacteraceae* stammt, und/oder
   die bakterielle Cellulose für Licht mit einer Wellenlänge im Bereich von 280 nm bis 1100 nm optisch durchlässig ist.

7. Mukoadhäsivum gemäß irgendeinem der Ansprüche 1 bis 6, wobei der Reiz aufweist:

   Licht mit einer Wellenlänge im Bereich von 280 nm bis 500 nm, und/oder
   eine Spannung im Bereich von -10 kV bis 10 kV.

8. Mukoadhäsivum gemäß irgendeinem der Ansprüche 1 bis 7, ferner aufweisend:

   ein in das Mukoadhäsivum eingebrachtes Arzneimittel, und/oder
   eine Polymerbeschichtung auf dem Mukoadhäsivum.

9. Verfahren zum Ausbilden des Mukoadhäsivums gemäß irgendeinem der Ansprüche 1 bis 8, wobei das Verfahren aufweist:

   Bereitstellen einer bakteriellen Cellulose,
   Bereitstellen eines Bioadhäsivums, das ein Polymer oder ein Dendrimer aufweist, wobei das Polymer und das Dendrimer mit einem Teil gepfropft sind, der in Gegenwart eines Reizes kovalente Wechselwirkungen mit einer Gewebefläche eingeht, wobei der Teil ein Diazirin aufweist, und
   Einbringen des Bioadhäsivums in die bakterielle Cellulose.

10. Verfahren gemäß Anspruch 9, wobei das Bereitstellen der bakteriellen Cellulose aufweist:

Beimpfen von Bakterien in einem oder mehreren Kulturmedien,
Extrahieren von roher bakterieller Cellulose aus dem einen oder den mehreren Kulturmedien, und/oder
Behandeln der rohen bakteriellen Cellulose mit einem Alkali, um die Bakterien aus der bakteriellen Cellulose zu entfernen.

11. Verfahren gemäß Anspruch 9 oder 10, wobei:

das Bereitstellen der bakteriellen Cellulose das Gefriertrocknen oder Presstrocknen der bakteriellen Cellulose aufweist, oder
das Bereitstellen der bakteriellen Cellulose das Gefriertrocknen oder Presstrocknen der bakteriellen Cellulose aufweist und ferner das Rehydrieren der bakteriellen Cellulose aufweist, die vor dem Einbringen des Bioadhäsivums gefriergetrocknet oder pressgetrocknet wurde.

12. Verfahren gemäß irgendeinem der Ansprüche 9 bis 11, wobei das Bereitstellen des Bioadhäsivums aufweist:

Pfropfen des Diazirins auf ein Polycaprolacton- oder ein Poly(amidoamin)-Dendrimer, und
Mischen des mit dem Diazirin gepfropften Polycaprolacton- oder Poly(amidoamin)-Dendrimers in einem Puffer.

13. Verfahren gemäß irgendeinem der Ansprüche 9 bis 12, wobei das Einbringen des Bioadhäsivums in die bakterielle Cellulose das Aufbringen des Bioadhäsivums auf die bakterielle Cellulose aufweist.

14. Verfahren gemäß Anspruch 9, wobei das Einbringen des Bioadhäsivums in die bakterielle Cellulose aufweist:

(i) In-Kontakt-Bringen der bakteriellen Cellulose mit einem organischen Lösungsmittel, das das Bioadhäsivum aufweist, und
Entfernen des organischen Lösungsmittels, damit das Bioadhäsivum in der bakteriellen Cellulose enthalten ist, oder
(ii) Mischen der bakteriellen Cellulose und des Bioadhäsivums, um ein Hydrogel zu bilden.

15. Verfahren gemäß irgendeinem der Ansprüche 9 bis 14, ferner aufweisend das Aufbringen einer Polymerbeschichtung auf das Mukoadhäsivum.

## Revendications

1. Mucoadhésif, comprenant :

une cellulose bactérienne ; et
un bioadhésif incorporé à la cellulose bactérienne,
dans lequel le bioadhésif comprend un polymère ou un dendrimère, le polymère et le dendrimère étant greffés d'un fragment qui forme des interactions covalentes avec une surface tissulaire en présence d'un stimulus, et le fragment comprenant une diazirine.

2. Mucoadhésif selon la revendication 1, dans lequel le polymère ou le dendrimère comprend respectivement un dendrimère de polycaprolactone ou de poly(amidoamine).

3. Mucoadhésif selon la revendication 1 ou 2, dans lequel :

la cellulose bactérienne est un film comprenant un réseau de nanofibres de cellulose ; et/ou
la cellulose bactérienne est séchée ou réhydratée ; et/ou
le bioadhésif est déposé sur la cellulose bactérienne.

4. Mucoadhésif selon la revendication 1 ou 2, dans lequel :

la cellulose bactérienne est un hydrogel ; ou
la cellulose bactérienne est un hydrogel et le bioadhésif est présent en une quantité allant de 10 % à 35 % en poids du mucoadhésif.

**5.** Mucoadhésif selon la revendication 1 ou 2, dans lequel :

le bioadhésif est inclus dans la cellulose bactérienne ; ou
le bioadhésif est inclus dans la cellulose bactérienne et le bioadhésif est présent en une quantité allant de 15 % à 60 % en poids du mucoadhésif.

**6.** Mucoadhésif selon l'une quelconque des revendications 1 à 5, dans lequel :

la cellulose bactérienne est d'une espèce dérivée de la famille des *Acetobacteraceae ;*
et/ou
la cellulose bactérienne est optiquement transparente à la lumière ayant une longueur d'onde allant de 280 nm à 1 100 nm.

**7.** Mucoadhésif selon l'une quelconque des revendications 1 à 6, dans lequel le stimulus comprend :

de la lumière ayant une longueur d'onde allant de 280 nm à 500 nm ; et/ou
une tension allant de -10 kV à 10 kV.

**8.** Mucoadhésif selon l'une quelconque des revendications 1 à 7, comprenant en outre :

un médicament pharmaceutique incorporé au mucoadhésif ; et/ou
un revêtement polymère sur le mucoadhésif.

**9.** Procédé de formation du mucoadhésif selon l'une quelconque des revendications 1 à 8, le procédé comprenant :

la fourniture d'une cellulose bactérienne ;
la fourniture d'un bioadhésif comprenant un polymère ou un dendrimère, le polymère et le dendrimère étant greffés avec un fragment qui forme des interactions covalentes avec une surface tissulaire en présence d'un stimulus, le fragment comprenant une diazirine ; et
l'incorporation du bioadhésif à la cellulose bactérienne.

**10.** Procédé selon la revendication 9, dans lequel la fourniture de la cellulose bactérienne comprend :

l'inoculation de bactéries dans un ou plusieurs milieux de culture ;
l'extraction de la cellulose bactérienne brute à partir dudit un ou desdits plusieurs milieux de culture ; et/ou
le traitement de la cellulose bactérienne brute avec un alcali pour éliminer les bactéries de la cellulose bactérienne.

**11.** Procédé selon la revendication 9 ou 10, dans lequel :

la fourniture de la cellulose bactérienne comprend la lyophilisation ou le séchage par compression de la cellulose bactérienne ; ou
la fourniture de la cellulose bactérienne comprend la lyophilisation ou le séchage par compression de la cellulose bactérienne et comprend en outre la réhydratation de la cellulose bactérienne qui a été lyophilisée ou séchée par compression avant l'incorporation du bioadhésif.

**12.** Procédé selon l'une quelconque des revendications 9 à 11, dans lequel la fourniture du bioadhésif comprend :

le greffage de la diazirine sur un dendrimère de polycaprolactone ou de poly(amidoamine) ; et
le mélange du dendrimère de polycaprolactone ou de poly(amidoamine) greffé avec la diazirine dans un tampon.

**13.** Procédé selon l'une quelconque des revendications 9 à 12, dans lequel l'incorporation du bioadhésif à la cellulose bactérienne comprend le dépôt du bioadhésif sur la cellulose bactérienne.

**14.** Procédé selon la revendication 9, dans lequel l'incorporation du bioadhésif à la cellulose bactérienne comprend :

(i) la mise en contact de la cellulose bactérienne avec un solvant organique comprenant le bioadhésif ; et
l'élimination du solvant organique pour que le bioadhésif soit inclus dans la cellulose bactérienne ;

ou

(ii) le mélange de la cellulose bactérienne et du bioadhésif pour former un hydrogel.

**15.** Procédé selon l'une quelconque des revendications 9 à 14, comprenant en outre l'application d'un revêtement polymère sur le mucoadhésif.

# FIG. 1

| Drug/Active ingredient | Ingredients | Clinical Conditions | Frequency |
|---|---|---|---|
| Menthol | Carbomer, HPMC | Canker Sores | 3 times a day |
| Dexamethasone acetate | Sodium CMC, HPC, Carbomer | Oral Ulcer | 4 times a day |
| Sesame oil | Chitosan | Oral Ulcer | NA |
| Lithospermum Root Extract Glycyrrhetinic Acid | Carbomer | Aphthous Stomatitis | 4 times a day |
| Lidoacaine | Karaya Gum | Anaesthetic | 30 minutes stay |
| Miconazole | Pregelatinized potato starch | Oropharyngeal Candidiasis | 4 times a day |
| Glycyrrhetinic acid | Poly-Vinyl-Pyrrolidone, Hyaluronic Acid | Oral mucositis, Ulcers, lesions | 4 times a day |
| Anesthetics/Anti-inflammatory/ Anti-viral drugs/biologics | Bacterial Cellulose, PAMAM-g-diazirine | Oral mucositis, Ulcers, lesions, Stomatitis | Once in 1-7 days |

## FIG. 2

| Properties | Existing Products | Present Mucoadhesive |
|---|---|---|
| **Adhesion time** | 6-8 hours on average | 1-7 days |
| **Adhesion strength** | Weak due to non-covalent interactions (hydrogen bonds, Van der Waals interactions)<br>• Hydrophilic macromolecules (Carbomers, HPMC, CMC, Alginate, Chitosan etc.): 2-3 kPa | Strong due to covalent interactions<br>• Tunable adhesion ranging from 6-22 kPa |
| **Mechanical stability in wet environment** | Disintegrates upon hydration within 6-8 hours<br>• Low storage modulus- 0.01-3 kPa | BC is water insoluble which would thus prevent dissolution and disintegration of the BC system by salivary fluids which is the key problem with existing marketed systems.<br>• High storage modulus- 10-200 kPa |
| **Regeneration matrix** | Need to be synthetically produced in form of nanofibers to form wound dressings. | BC has been shown to promote skin wound healing and regeneration owing to inherent nanofibrous nature, which can be utilized to promote oral wound management |
| **Ease of handling/ Structural Strength** | Low tensile strength and brittle nature might lead to tearing off of the films during handling. | High tensile strength (3-30 MPa) and storage modulus (30-200 kPa) allows it to withstand the stresses due to movements in the oral cavity and offers ease of handling. |

# FIG. 3

Two-component mucoadhesive system

Bacterial Cellulose         PAMAM-g-Diazirine
(BC) film                   (PDz)

Bacterial cellulose mucoadhesive patch

Bacterial Cellulose film
(BC)

PAMAM-g-Diazirine
(PDz)

PDz coated BC film

Aqueous mucoadhesive hydrogel

Bacterial Cellulose (BC) film

Sonication

PAMAM-g-Diazirine
(PDz)

BC_PDz hydrogel

# FIG. 4A

UV Led Lamp
365 nm@100 mW cm⁻²

Microscopic
glass slide

BC film

Bioadhesive
(PDz)

Porcine tissue
substrate

50 N Load

3 mm/minute

## FIG. 4B

| UV Dose Actual | 0J | 0.21J | 0.42J | 1.26J | 0J | 0.42J | 0.84J | 2.52J |
|---|---|---|---|---|---|---|---|---|
| UV Dose Applied | 0J | 3J | 6J | 18J | 0J | 3J | 6J | 18J |
| | Dry | | | | Rehydrated | | | |

## FIG. 4C

| UV Dose Actual | 0J | 0.57J | 1.14J | 3.42J | 0J | 0.75J | 1.5J | 4.5J |
|---|---|---|---|---|---|---|---|---|
| UV Dose Applied | 0J | 3J | 6J | 18J | 0J | 3J | 6J | 18J |
| | Dry | | | | Rehydrated | | | |

## FIG. 4D

| UV Dose | 10J | | | | | | 3J | 6J | 18J | 3J | 6J | 18J |
|---|---|---|---|---|---|---|---|---|---|---|---|---|
| BC (μmoles) | 5 | 10 | 20 | 5 | 10 | 20 | 40 | | | | | |
| % Total Solute | 20% | | | 33.3% | | | 25% | | | | | |
| | Hydrogel | | | | | | Film (BC_FD) | | | Film (BC_PD) | | |

## FIG. 5A

— Porcine Tissue

— BC film with PDz

— pH 6.8 Buffer

1.5 cm

## FIG. 5B

## FIG. 5C

Day 1                    Day 7

## FIG. 5D

PDz adhesive on both sides
Cohesive fracture

## FIG. 6

## FIG. 7

## FIG. 8

26°C, static
5 days

**5% inoculum of *G.Hansenii*
in Hestrin Schramm media**

BC pellicle at
liquid air interface

Wash and treatment
with 1% NaOH

**Weight: 6.3 ± 0.3 mg
Thickness: 160 ± 20 μm
%Rehydration: 58**

Freeze Dried

Press Dried
(Under Pressure)

**Weight: 7.1 ± 0.3 mg
Thickness: 30 ± 5 μm
%Rehydration: 3**

**Native BC Pellicle
Weight: 860 ± 40 mg
Thickness: 3 ± 0.2 mm**

## FIG. 9

| % Hydration | Absorption Coefficient (cm²/g) | | |
|---|---|---|---|
| | 365 nm | 385 nm | 405 nm |
| **BC_FD** 0% | 827 ± 196 | 755 ± 181 | 592 ± 172 |
| 100% | 707 ± 167 | 611 ± 152 | 479 ± 119 |
| 200% | 625 ± 151 | 565 ± 144 | 520 ± 128 |
| 300% | 648 ± 155 | 589 ± 151 | 509 ± 124 |
| **BC_PD** 0% | 476 ± 118 | 421 ± 102 | 420 ± 120 |
| 100% | 342 ± 83 | 274 ± 69 | 263 ± 76 |
| 200% | 388 ± 94 | 332 ± 80 | 310 ± 78 |
| 300% | 418 ± 101 | 329 ± 79 | 355 ± 91 |

## FIG. 10A

## FIG. 10B

# FIG. 11

# FIG. 12

# FIG. 12 (Continued)

G) 50% PDz

100 µm

H)

BC_FD Rh

BC_PD Rh

BC_FD

BC_PD

50% PDz

Pore length (µm)

Pores/mm²

I)

■ Pores/mm²
⬤ Pore length

Pores/mm²

Pore length (microns)

50% PDz | BC_PD | BC_FD | BC_PD Rh | BC_FD Rh

# FIG. 13

| FTIR Peak cm<sup>-1</sup> | Functional Group | Normalized Ratio (peak absorbance/1630 absorbance) | | |
|---|---|---|---|---|
| | | BC_PDz | BC_PDz cured | %change |
| 3345 | -OH stretch | 0.75 | 0.44 | -42 |
| 3291 | Amine/Hydroxyl | 0.78 | 0.50 | -35 |
| 2938 | -CH- strech (alkane) | 0.31 | 0.30 | -4 |
| 2895 | -CH stretch | 0.28 | 0.25 | -12 |
| 2090 | Diazo | 0.05 | 0.12 | 166 |
| 1630 | Amide I | 1.00 | 1.00 | 0 |
| 1460 | Methylene (-CH$_2$) | 0.37 | 0.43 | 17 |
| 1556 | Amide II | 0.81 | 0.91 | 12 |
| 1280 | -CH bending | 0.18 | 0.28 | 54 |
| 1056 | Bending of -C-O-H bonds | 0.59 | 0.15 | -75 |
| 1024 | C-O/ C-N | 0.60 | 0.16 | -73 |

## FIG. 14

# FIG. 15

| FTIR Peak cm⁻¹ | Functional Group | Absorbance (Abs) | | | | Normalized (peak/1630) | | Ratio |
|---|---|---|---|---|---|---|---|---|
| | | BC | PDz | BC_PDz | BC_PDz cured | BC_PDz | BC_PDz cured | % change |
| 3345 | -OH stretch | 0.18 | 0.24 | 0.21 | 0.14 | 0.75 | 0.44 | -42 |
| 3291 | Amine/Hydroxyl | 0.12 | 0.25 | 0.22 | 0.16 | 0.78 | 0.50 | -35 |
| 2938 | -CH- stretch (alkane) | 0.03 | 0.07 | 0.09 | 0.10 | 0.31 | 0.30 | -4 |
| 2895 | -CH stretch | 0.04 | 0.05 | 0.08 | 0.08 | 0.28 | 0.25 | -12 |
| 2090 | Diazo | 0.01 | 0.01 | 0.01 | 0.04 | 0.05 | 0.12 | 166 |
| 1630 | **Amide I** | **0.03** | **0.27** | **0.28** | **0.32** | **1.00** | **1.00** | **0** |
| 1460 | Methylene (-CH₂) | 0.03 | 0.06 | 0.08 | 0.13 | 0.37 | 0.43 | 17 |
| 1556 | Amide II | 0.02 | 0.19 | 0.23 | 0.29 | 0.81 | 0.91 | 12 |
| 1280 | -CH bending | 0.04 | 0.02 | 0.05 | 0.09 | 0.18 | 0.28 | 54 |
| 1056 | Bending of -C-O-H bonds | 0.50 | 0.01 | 0.16 | 0.05 | 0.59 | 0.15 | -75 |
| 1024 | C-O/ C-N | 0.32 | 0.02 | 0.17 | 0.05 | 0.60 | 0.16 | -73 |

## FIG. 16A

## FIG. 16B

| | %Hydration | Absorbance | | | Equation | R-square | Pearson's r |
|---|---|---|---|---|---|---|---|
| | | 365 nm | 385 nm | 405 nm | | | |
| BC_FD | 0% | 1.15 ± 0.03 | 1.05 ± 0.04 | 0.82 ± 0.14 | y= -0.0061x + 3.4 | 0.93 | -0.96 |
| | 100% | 0.98 ± 0.02 | 0.85 ± 0.06 | 0.66 ± 0.05 | y= -0.0078x + 3.8 | 0.99 | -0.99 |
| | 200% | 0.87 ± 0.05 | 0.78 ± 0.08 | 0.72 ± 0.06 | y= -0.0037x + 2.2 | 0.99 | -0.99 |
| | 300% | 0.90 ± 0.05 | 0.82 ± 0.08 | 0.71 ± 0.03 | y= -0.0049x + 2.7 | 0.99 | -0.99 |
| BC_PD | 0% | 0.73 ± 0.05 | 0.65 ± 0.04 | 0.65 ± 0.10 | y= -0.0030x + 1.8 | 0.8 | -0.89 |
| | 100% | 0.53 ± 0.03 | 0.42 ± 0.03 | 0.40 ± 0.06 | y= -0.0038x + 1.9 | 0.89 | -0.95 |
| | 200% | 0.60 ± 0.03 | 0.51 ± 0.03 | 0.48 ± 0.04 | y= -0.0030x + 1.7 | 0.92 | -0.96 |
| | 300% | 0.64 ± 0.03 | 0.51 ± 0.01 | 0.55 ± 0.05 | y= -0.0043x + 2.1 | 0.59 | -0.77 |

## FIG. 17

## FIG. 18

| Total solute % | PDz (mM) | BC (mM) | [Diazirine]/[OH] mol ratio | %BC remaining | PDz (%) | BC (%) |
|---|---|---|---|---|---|---|
| 10 | 2.4 | 73.8 | 15:110 | 21.4 | 9.0 | 1.0 |
| 20 | 6.5 | 76.6 | 17:48 | 6.7 | 19.0 | 1.0 |
| 33.3 | 13.5 | 78.9 | 17:24 | 1.5 | 32.5 | 0.8 |

# FIG. 19

| Total solute % | PDz (mM) | BC (mM) | [Diazirine]/[OH] mol ratio | %BC remaining | PDz (%) | BC (%) |
|---|---|---|---|---|---|---|
| 20 | 6.8 | 20 | 17:12 | 0 | 19.7 | 0.3 |
| 20 | 6.8 | 40 | 17:24 | 2.9 | 19.5 | 0.5 |
| 20 | 6.8 | 80 | 17:48 | 6.5 | 19.0 | 1.0 |
| 33.3 | 13.6 | 20 | 17:6 | 0 | 33.1 | 0.2 |
| 33.3 | 13.6 | 40 | 17:12 | 0 | 32.9 | 0.4 |
| 33.3 | 13.6 | 80 | 17:24 | 1.5 | 32.5 | 0.8 |

# FIG. 20

## FIG. 21A

## FIG. 21B

## FIG. 21C

1.3% BC hydrogel

## FIG. 21D

1.3% BC hydrogel

# FIG. 22A

# FIG. 22B

## FIG. 22C

## FIG. 22D

## FIG. 22E

## FIG. 22F

## FIG. 23

## FIG. 24

# FIG. 24 (Continued)

# FIG. 25

## FIG. 26

## FIG. 27

FIG. 28

Total Solute/ BC Moles — 20% — 33.3%

0 mM

20 mM

40 mM

80 mM

## FIG. 29

## FIG. 30

| FTIR Peak cm⁻¹ | Functional Group | Absorbance (Abs) | | | | Normalized Ratio (peak/1626) | | |
|---|---|---|---|---|---|---|---|---|
| | | BC | PDz | BC_PDz (33.3%_80) | BC_PDz cured | BC_PDz (33.3%_80) | BC_PDz cured | %change |
| 3345 | -OH stretch | 0.18 | 0.24 | 0.28 | 0.17 | 1.83 | 0.44 | -76 |
| 3291 | Amine/Hydroxyl | 0.12 | 0.25 | 0.28 | 0.20 | 1.80 | 0.50 | -72 |
| 2938 | -CH₂- | 0.03 | 0.07 | 0.05 | 0.11 | 0.31 | 0.29 | -9 |
| 2895 | -CH stretch | 0.04 | 0.05 | 0.04 | 0.09 | 0.24 | 0.23 | -5 |
| 2090 | Diazo | 0.01 | 0.01 | 0.01 | 0.05 | 0.10 | 0.12 | 27 |
| 1626 | Amide I | 0.03 | 0.27 | 0.15 | 0.39 | 1.00 | 1.00 | 0 |
| 1556 | Amide II | 0.02 | 0.19 | 0.08 | 0.37 | 0.50 | 0.94 | 88 |
| 1280 | -CH bending | 0.04 | 0.02 | 0.01 | 0.11 | 0.05 | 0.28 | 419 |
| 1161 | Ester | 0.14 | 0.04 | 0.03 | 0.11 | 0.19 | 0.29 | 56 |
| 1108 | -C-C bonds | 0.25 | 0.00 | 0.03 | 0.11 | 0.21 | 0.27 | 26 |
| 1056 | Bending of -C-O-H bonds | 0.50 | 0.01 | 0.08 | 0.08 | 0.52 | 0.20 | -62 |
| 1024 | C-O/ C-N | 0.32 | 0.02 | 0.10 | 0.07 | 0.64 | 0.18 | -72 |

# FIG. 31

## FIG. 32A

## FIG. 32B

**CA** - Cellulose Acetate
**PVA** - Polyvinyl Alcohol
**CMC** - Carboxymethyl Cellulose

## FIG. 32C

Day 0       Day 7

## FIG. 32D

Day 0       Day 7

## FIG. 33A

After failure

# FIG. 33B

# FIG. 33C

## FIG. 33D

## FIG. 34

| Sr.No. | Drug | Activity | Drug loading efficiency |
|--------|------|----------|-------------------------|
| 1. | Lidocaine | Local Anaesthetic | $96 \pm 4\,\%$ |
| 2. | Dexamethasone | Anti-inflammatory | $98 \pm 10\,\%$ |
| 3. | Valganciclovir | Anti-viral | $90 \pm 10\,\%$ |

# FIG. 35

# FIG. 36A

## FIG. 36B

BC_PD

## FIG. 36C

BC_FD

PCL coated

PLGA coated

# FIG. 36D

## FIG. 37

| Characteristics | 2-component film system | Integrated patch | Liquid hydrogel adhesive |
|---|---|---|---|
| Configuration | Film | Film | Liquid → Hydrogel |
| Description of components | Composed of two separate components: (1) BC film and (2) PDz layer.<br>• PDz needs to be applied onto BC films before use each time followed by photocuring (layered) | Composed of a single component where the PDz is integrated to the BC matrix.<br>• Predefined amount of PDz is coated (organic solvent) onto BC films followed solvent evaporation to yield dry patch (PDz integrated in BC matrix) | Varying % of BC (hydrogel form) and PDz are mixed to yield liquid/low yielding solid composites |
| PDz integration | PDz only on BC surface, not integrated (As observed by fracture through PDz matrix, pore size 70-250 µm) | PDz integrated up to 40-50 µm thickness of BC (pore size upon fracture ~ 5-30 µm) | Uniform mixing as observed by uniform pores upon fracture |
| Application | PDz is manually applied to the BC film and the combination is subsequently applied to the site | Apply directly to disease site and photocured | Apply directly to disease site and photocured<br>Can be applied to irregular sites |
| Storage temperature | Both components are stored separately<br>• BC @ room temperature (RT), PDz (50% w/w in PBS)@ -20°C)] | Patch can be stored at RT | Stored at 4°C |
| Adhesion strength | Adhesion strength 7-17 kPa against mucosal tissues | Adhesion strength 20-40 kPa against mucosal tissues (2-4 times higher than the 2-component system) | Adhesion strength 3-35 kPa against wet tissue mimic |

EP 4 251 216 B1

# FIG. 38A

| Drug | Activity | Drug loading efficiency |
|---|---|---|
| Bovine Serum Albumin | Protein | $80\% \pm 15\%$ |

# FIG. 38B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- SG 10202011922 U

**Non-patent literature cited in the description**

- **AHMAD NAVEED** ; **AMIN MOHD CAIRUL IQBAL MOHD** ; **MAHALI SHALELA MOHD** ; **ISMAIL ISMANIZAN** ; **CHUANG VICTOR TUAN GIAM**. Molecular Pharmaceutics. Americal Chemical Society, vol. 11, 4130-4142